(19) Europäisches Patentamt / European Patent Office / Office européen des brevets

(11) **EP 3 785 632 A1**

(12) **EUROPEAN PATENT APPLICATION**

(43) Date of publication:
03.03.2021 Bulletin 2021/09

(51) Int Cl.:
*A61B 5/145* (2006.01)  *A61B 5/1495* (2006.01)
*B08B 3/04* (2006.01)

(21) Application number: 20192627.6

(22) Date of filing: 25.08.2020

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 30.08.2019 JP 2019158640

(71) Applicant: SYSMEX CORPORATION
Kobe-shi
Hyogo 651-0073 (JP)

(72) Inventors:
• **Inutsuka, Takumi**
  **Hyogo, 651-0073 (JP)**
• **Fujii, Ryosuke**
  **Hyogo, 651-0073 (JP)**
• **Suganuma, Toshikuni**
  **Hyogo, 651-0073 (JP)**

(74) Representative: **Hoffmann Eitle**
**Patent- und Rechtsanwälte PartmbB**
**Arabellastraße 30**
**81925 München (DE)**

(54) **PRETREATMENT METHOD AND IN-VIVO COMPONENT MEASURING DEVICE**

(57)     A pretreatment method and an in-vivo component measuring device in which problems caused by bubbles is suppressed is provided.

The pretreatment method uses an electrode sensor 21 for measuring a measurement target component contained in a measurement sample collected from a subject, and a liquid used for pretreatment of measurement, the pretreatment includes step (A) for forming a droplet 8 of liquid, step (B) for pressing the droplet 8 of liquid onto the surface of the electrode type sensor 21 by relative movement of the droplet 8 of liquid and the electrode type sensor 21, and step (C) for removing the droplet 8 pressed against the surface of the expression sensor 21.

FIG. 1

**Description**

CROSS REFERENCE TO RELATED APPLICATIONS

This application claims priority from prior Japanese Patent Application(s) No. 2019-158640, filed on August 30, 2019, entitled "PRETREATMENT METHOD AND IN-VIVO COMPONENT MEASURING DEVICE", the entire contents of which are incorporated herein by reference.

FIELD OF THE INVENTION

The present invention relates to a pretreatment method and in-vivo component measuring device.

BACKGROUND

In order to measure in-vivo components contained in a biological sample, there are known measuring devices equipped with an electrode sensor, and this type of measuring device requires pretreatment for measurement. For example, it is necessary to perform a pretreatment of acquiring measurement values of a plurality of standard solutions having different concentrations and creating a calibration curve using the acquired measurement values. Pretreatment for cleaning the electrode type sensor with a cleaning liquid also is necessary in order to prevent carryover of biological components attached to the surface of the electrode type sensor.

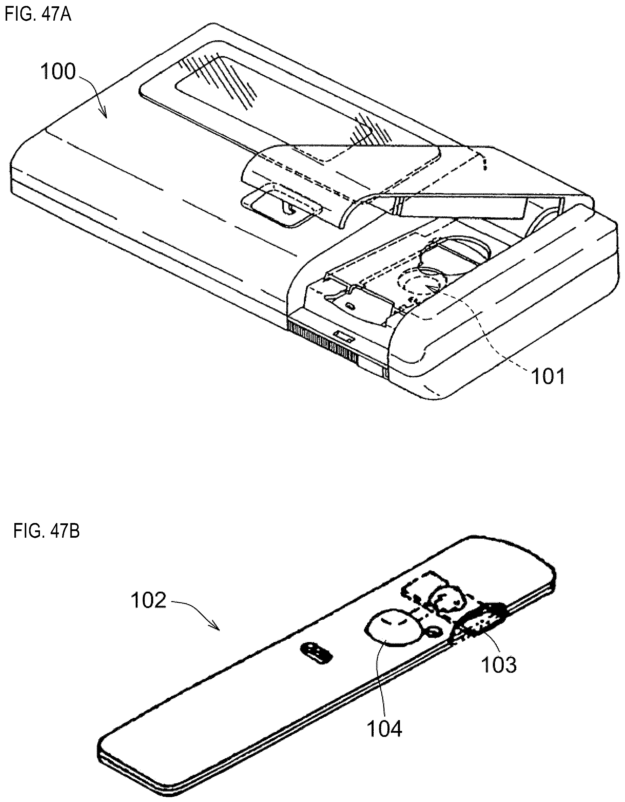

In Japanese Utility Model Publication No. 1989-207756, for example, a calibration standard solution is guided to an enzyme electrode 101 through a diffusion limiting film 103 to obtain a measurement value of the calibration standard solution, and the enzyme electrode 101 is initially calibrated by crushing a calibration standard solution container 104 after pressing the diffusion limiting film 103 and the enzyme electrode 101 against each other with a calibration standard solution holder 102 attached to a predetermined position of a casing 100 of the blood glucose level measuring device, as shown in FIG. 47.

On the other hand, in Japanese Patent Application Publication No. 2006-126046, an enzyme electrode sensor 107 is attached to a sponge-like porous substance 106 provided on the bottom surface of a storage solution storage tank 105 that stores a cleaning/humidifying storage solution, a measurement target substance remaining on the surface of the enzyme electrode sensor 107 is washed with a preservative solution that is brought into contact with the porous substance 106 and exudes into the porous substance 106.

PRIOR ART DOCUMENTS

Patent Documents

Patent Document 1: Japanese Utility Model Publication No. 1-207756
Patent Document 2: Japanese Patent Application Publication No. 2006-126046

SUMMARY OF THE INVENTION

PROBLEM TO BE SOLVED BY THE INVENTION

In the pretreatment for the measurement described in Japanese Utility Model Publication No. 1989-207756, air bubbles are mixed in the calibration standard solution container 104 since the calibration standard solution is guided to the enzyme electrode 101 by crushing the calibration standard solution container 104, and when air bubbles are mixed in the calibration standard solution container 104, the air bubbles may also be guided to the enzyme electrode 101 at the same time such that the air bubbles may be present on the surface of the enzyme electrode 101.

In the pretreatment for the measurement described in Japanese Patent Application Publication No. 2006-126046, air bubbles may exist between the storage solution and the enzyme electrode sensor 107 since the enzyme electrode sensor 107 is washed using the storage solution exuded in the sponge-like porous material 106.

The presence of such bubbles on the surfaces of the enzyme electrode 101 and the enzyme electrode sensor 107 may cause various problems in the pretreatment for the measurement.

In order to solve the above problems, the present invention provides a pretreatment method and an in-vivo component measuring device in which problems due to air bubbles are suppressed.

MEANS FOR SOLVING THE PROBLEM

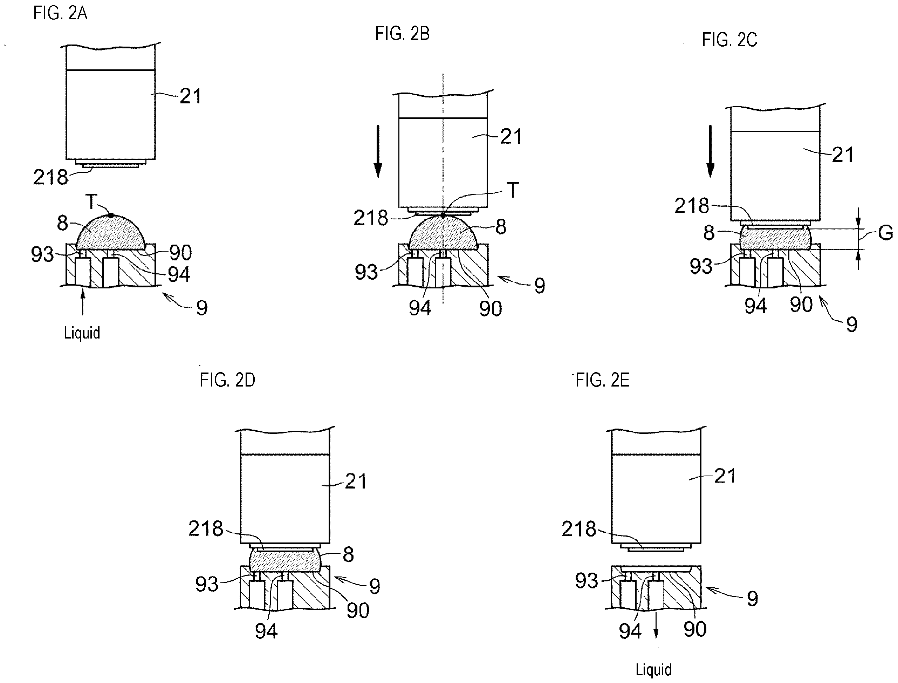

The present invention relates to a pretreatment method using an electrode-type sensor for measuring a measurement target component contained in a measurement sample collected from a subject, and a liquid used for the pretreatment of the measurement. As shown in FIGS. 2, 6, 30, 37, 39, 41, 43, and 45, the pretreatment method according to the present invention includes (A) forming a droplet 8, (B) pressing the droplet 8 against the surface of the electrode-type sensor 21 by the relative movement of the droplet 8 and the electrode -type sensor 21, and (C) removing the droplet 8.

According to the pretreatment method of the present invention, the droplet 8 to be used for the pretreatment is formed, and then the droplet 8 is pressed against the surface of the electrode-type sensor 21, so that the droplet 8 is in contact with the surface of the electrode-type sensor 21. In this way, even if bubbles are mixed in when the droplets 8 are formed, the bubbles float on the surface of the droplets during the formation of the droplet 8 and the droplet 8 is pressed against the surface of the electrode-type sensor 21, such that the bubbles escape or break from the surface of the droplet. Hence, when the droplet 8 used for the pretreatment is brought into contact with the surface of the electrode-type sensor 21, it is possible to suppress the presence of bubbles between the surface of the electrode-type sensor 21 and the electrode 218, thereby reducing the possibility of causing various problems in the pretreatment.

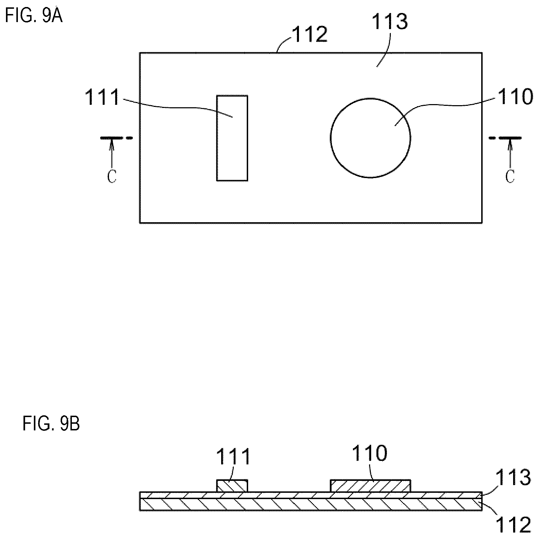

The present invention relates to an in-vivo component measuring device for measuring components contained in a measurement sample collected from a subject. As shown in FIGS. 9, 10, 13, 25, and 26, the in-vivo component measuring device 1 according to the present embodiment includes electrode-type sensors 21A and 21B for measuring the measurement target components contained in a measurement sample by contacting the collection bodies 110 and 111 which collect the measurement sample, counterpart objects 9A and 9B facing the surfaces of the electrode-type sensors 21A and 21B, a fluid circuit unit 4 configured to send a liquid onto the droplet forming surface 90 of the counterpart objects 9A and 9B and discharge the liquid from the droplet forming surface 90, a moving unit 231 for moving at least one of the electrode-type sensors 21A, 21B and the counterpart objects 9A, 9B, and a control unit 5 for controlling the fluid circuit unit 4 and the moving unit 231, wherein the control unit 5 controls the fluid circuit unit 4 to form the droplets 8 on the droplet forming surface 90, controls at least one of the fluid circuit unit 4 and the moving unit 231 so as to press the droplet 8 onto the surfaces of the electrode sensors 21A and 21B by the relative movement of the droplet 8 and the electrode-type sensors 21A and 21B, and controls the fluid circuit unit so as to remove the droplet 8 from the droplet forming surface 90.

According to the in-vivo component measuring device of the present invention, the droplet 8 to be used for pretreatment is formed on the droplet forming surface 90 of the counterpart objects 9A and 9B facing the electrode-type sensors 21A and 21B, and the droplet 8 is brought into contact with the surfaces of the electrode-type sensors 21A and 21B by pressing the droplet 8 against the surfaces of the electrode-type sensors 21A and 21B. In this way, even if bubbles are mixed in when the droplet 8 is formed, the bubbles float on the droplet surface during the formation of the droplet 8, and the bubbles escape or break from the surface of the droplet 8 when the droplet 8 is pressed against the surface of the electrode-type sensor 21A and 21B. Hence, when the droplet 8 of the liquid used for the pretreatment is brought into contact with the surface of the electrode-type sensor 21A and 21B, it is possible to suppress the presence of bubbles between the surface of the electrode-type sensor 21A and 21B, thereby reducing the possibility of causing various problems in the pretreatment.

EFFECT OF THE INVENTION

According to the present invention, it is possible to suppress the problems caused by bubbles during the pretreatment for measurement.

BRIEF DESCRIPTION OF THE DRAWINGS

FIG. 1 is a flowchart showing a procedure of a pretreatment method;
FIG. 2 is a diagram illustrating each step of the pretreatment method;
FIG. 3A is a plan view of a counterpart object, FIG. 3B is a cross-sectional view on the line AA of FIG. 3A, and FIG. 3C is a cross-sectional view showing an enlarged main part of FIG. 3B;
FIG. 4 is a diagram showing a state in which droplet is formed on the droplet forming surface of the counterpart object;
FIG. 5A and FIG. 5B are cross-sectional views showing an enlargement of a main part of a counterpart object of a modified example;
FIG. 6 is a diagram illustrating each step of the pretreatment method of a modified example;
FIG. 7 is a diagram illustrating a part of the steps of the pretreatment method of the modified example;
FIG. 8A is a plan view of a counterpart object of a modified example, FIG. 8B is a cross-sectional view on line BB of FIG. 8A, and FIG. 8C is an enlarged view of a main portion of the cross section of FIG. 8B;
FIG. 9A is a plan view of a holding sheet holding an interstitial fluid collector and a sweat collector, and FIG. 9B is a sectional view on line CC of FIG. 9A;
FIG. 10 is a diagram illustrating a procedure of collecting interstitial fluid with a collector;
FIG. 11A is a perspective view of the in-vivo component measuring device when the first cover is in a closed state, and FIG. 11B is a perspective view of the in-vivo component measuring device when the first cover is in an open state;
FIG. 12A is a side view showing a schematic internal structure of the in-vivo component measuring device, and FIG. 12B is a front view showing a schematic internal structure of the in-vivo component measuring device;
FIG. 13 is a perspective view of a detection unit;
FIG. 14A is a plan view of a sample plate, FIG. 14B is a side view of the sample plate, and FIG. 14C is a DD cross-sectional view;
FIG. 15A is a plan view showing a state in which an interstitial fluid collector and a sweat collector are placed on a sample plate, and FIG. 15B is a sectional view taken along line EE of FIG. 15A;
FIG. 16A is a plan view of a sample stage, FIG. 16B is a sectional view on line FF of FIG. 16A, and FIG. 16C is a sectional view on line GG of FIG. 16A;

FIG. 17A is a perspective view of a glucose sensor and a sodium ion sensor, and FIG. 17B is a side view of the glucose sensor and the sodium ion sensor;
FIG. 18A is a plan view of a fixture, and FIG. 18B is a rear view of the fixture;
FIG. 19 is a front view showing a summary of the structure of an installation unit moving unit;
FIG. 20 is a diagram showing a summary of the structure of an installation unit moving unit in a plan view;
FIG. 21 is a diagram illustrating a position where the installation unit moves;
FIG. 22A is a diagram showing a summary of the structure of the sensor moving unit in a plan view, and FIG. 22B is a diagram showing a summary of the structure of the sensor moving unit in a rear view;
FIG. 23 is a diagram illustrating a moving range of a glucose sensor and a sodium ion sensor;
FIG. 24 is a diagram illustrating positions where a glucose sensor and a sodium ion sensor move;
FIG. 25 is a diagram showing a summary of the structure of a sample storage unit and a fluid circuit unit connected to a counterpart object;
FIG. 26 is a block diagram of an in-vivo component measuring device;
FIG. 27 is a flowchart showing a procedure for measuring an in-vivo component by the control unit;
FIG. 28 is a flowchart showing a procedure for creating a calibration curve using the glucose sensor of FIG. 27;
FIG. 29 is a flow chart showing a procedure for creating a calibration curve using the sodium ion sensor of FIG. 27;
FIG. 30 is a diagram describing each step of the pretreatment method;
FIG. 31 is a flowchart showing a procedure of cleaning the glucose sensor and the sodium ion sensor of FIG. 27;
FIG. 32 is a flowchart showing the measurement procedure of FIG. 27;
FIG. 33 is a flow chart showing the procedure of measurement by the sodium ion sensor of FIG. 32;
FIG. 34 is a flow chart showing a procedure of measurement by the glucose sensor of FIG. 32;

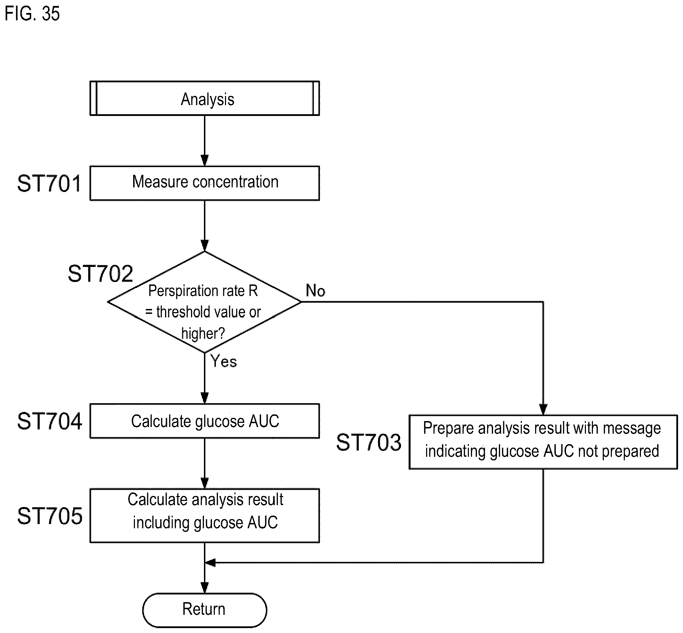

FIG. 35 is a flow chart showing the analysis procedure in FIG. 27;
FIG. 36 is a flowchart showing a cleaning procedure of the glucose sensor and the sodium ion sensor of a modification;
FIG. 37 is a diagram illustrating each step of the pretreatment method of the modification;
FIG. 38 is a flowchart showing a cleaning procedure of the glucose sensor and the sodium ion sensor of a modification;
FIG. 39 is a diagram illustrating each step of the pretreatment method of a modification;
FIG. 40 is a flowchart showing a cleaning procedure of the glucose sensor and the sodium ion sensor of a modification;

FIG. 41 is a diagram describing each step of the pretreatment method of a modification;
FIG. 42 is a flowchart showing a cleaning procedure of the glucose sensor and the sodium ion sensor of a modification;
FIG. 43 is a diagram describing each step of the pretreatment method of a modification;
FIG. 44 is a flowchart showing a cleaning procedure of the glucose sensor and the sodium ion sensor of a modification;
FIG. 45 is a diagram describing each step of the pretreatment method of a modification;
FIG. 46 is a diagram showing a summary of the structure of a sample storage unit and a fluid circuit unit connected to a counterpart object of a modification;
FIG. 47A is a perspective view of a conventional blood glucose level measuring device, and FIG. 47B is a perspective view of a calibration standard solution holder; and

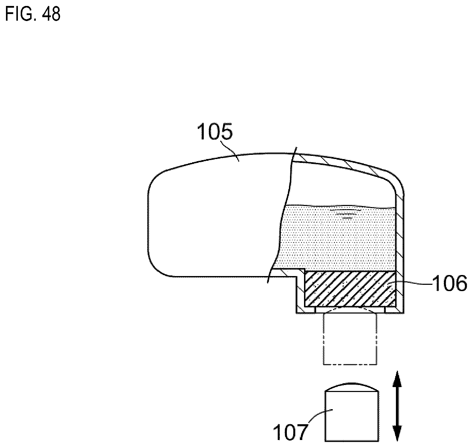

FIG. 48 is a diagram showing a summary of the structure of a conventional electrode type sensor cleaning process method.

DESCRIPTION OF THE EMBODIMENTS OF THE INVENTION

Hereinafter, an embodiment of a pretreatment method and an in-vivo component measuring device including an electrode sensor of the present invention will be described in detail with reference to the accompanying drawings.

Pretreatment Method

First, the pretreatment method of this embodiment will be described. The pretreatment method of the present embodiment uses an electrode-type sensor that measures a measurement target component contained in a measurement sample collected from a subject, and a liquid that is used for pretreatment of measurement, wherein a droplet of the liquid is brought into contact with the surface of the electrode type sensor, and a pretreatment such as cleaning and moisturizing of the electrode type sensor with a washing liquid, and preparation of a calibration curve using a plurality of standard solutions having different concentrations are performed.
Note that the surface of the electrode-type sensor is a surface that includes electrodes, and refers to at least a region where the electrodes are present. The electrodes also generally include a working electrode, a counter electrode, a reference electrode, and the like, but in the following description, these are collectively referred to as an electrode, and are schematically represented by one electrode in the drawings.
The electrode-type sensor is used for measuring the amount of a measurement target component contained in a measurement sample collected from a subject, for example, an electrode type sensor used for measuring the concentration of a measurement target component by acquiring a signal reflecting the concentration. Examples of the measurement target component include glucose and electrolytes contained in interstitial fluid collected as a measurement sample from the subject; however, the measurement sample and the measurement target component are not limited to these examples.

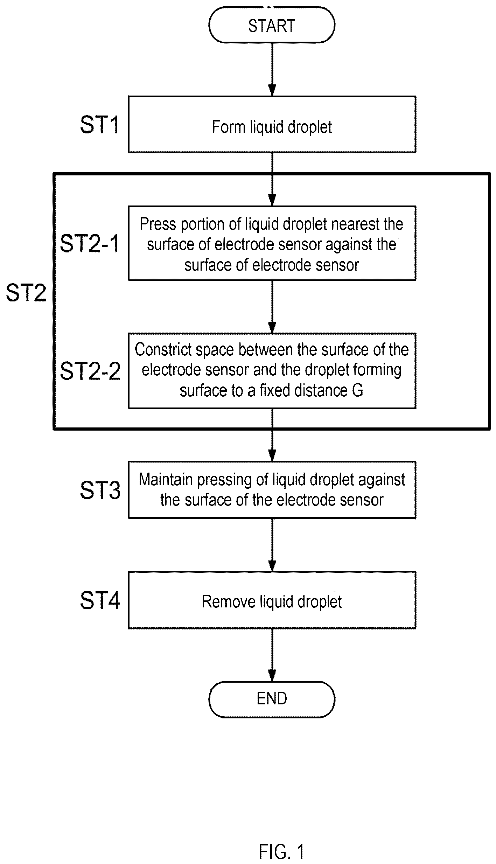

As shown in FIGS. 1 and 2, the pretreatment method of the present embodiment includes the following steps.

(A) Forming a droplet 8 of liquid used for pretreatment for measurement (ST1 in FIG. 1, FIG. 2A).

(B) Pressing the droplet 8 against the surface of the electrode sensor 21 by the relative movement of the droplet 8 and the electrode sensor 21 (ST2 of FIG. 1, FIGS. 2B and 2C).

(C) Removing the droplet 8 pressed against the surface of the electrode sensor 21 (ST4 in FIG. 1, FIG. 2E).

In the pretreatment method of the present embodiment, a step of pressing the droplet 8 on the surface of the electrode sensor 21 and then maintaining the state of pressing the droplet 8 on the surface of the electrode sensor 21 for a certain period of time. (ST3 of FIG. 1, FIG. 2D) is further included.

In the step of forming the droplet 8, the droplet 8 is formed on the droplet forming surface 90 of the counterpart object 9 facing the surface of the electrode type sensor 21, and the droplet 8 is removed from the droplet forming surface 90 of the counterpart object 9 in the step of removing the droplet 8.

The counterpart object 9 is located in a direction facing the electrode 218 of the electrode sensor 21. In the present embodiment, the counterpart object 9 is arranged below the electrode 218 so that the droplet forming surface 90 faces the electrode 218 with a space therebetween. Examples of the material of the counterpart object 9 include metal and synthetic resin, among which polyacetal resin is preferable from the perspective of cost and workability.

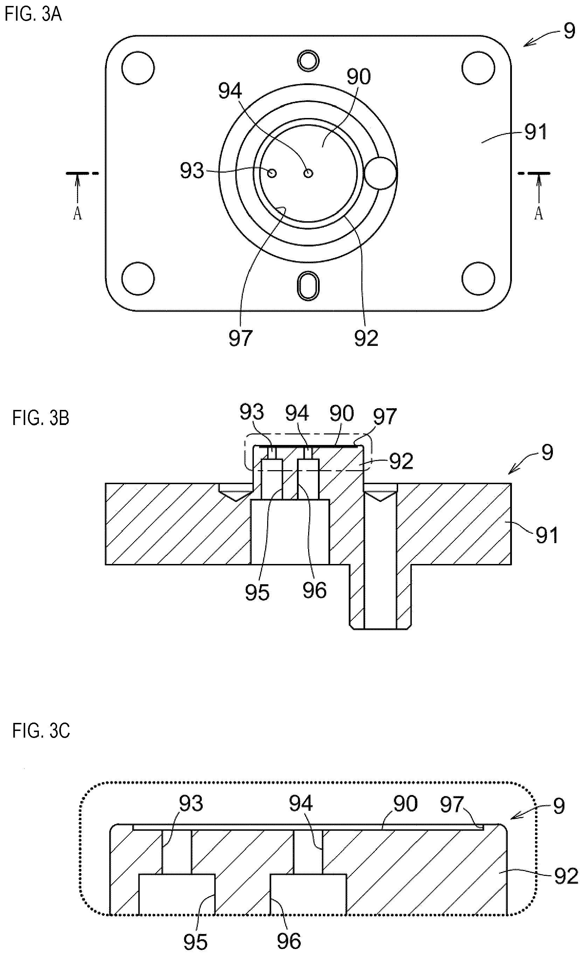

As shown in FIG. 3, the counterpart object 9 includes a base 91 having a substantially rectangular shape and a constant thickness, and a circular projection 92 that protrudes from the upper surface of the base 91 at a constant height in the center of the base 91, and a circular droplet forming surface 90 is provided on the upper surface of the circular projection 92. In the present embodiment, the upper surface of the projection 92 is recessed except for the outer peripheral edge portion, and the upper surface of this recessed portion serves as the droplet forming surface 90. A wall surface 97 rises around the droplet forming surface 90, which is circumscribed by the wall surface 97. The wall surface 97 may be perpendicular to the droplet forming surface 90 or may be inclined. The droplet forming surface 90 and the protrusion 92 do not necessarily have to be circular, and may be rectangular or polygonal.

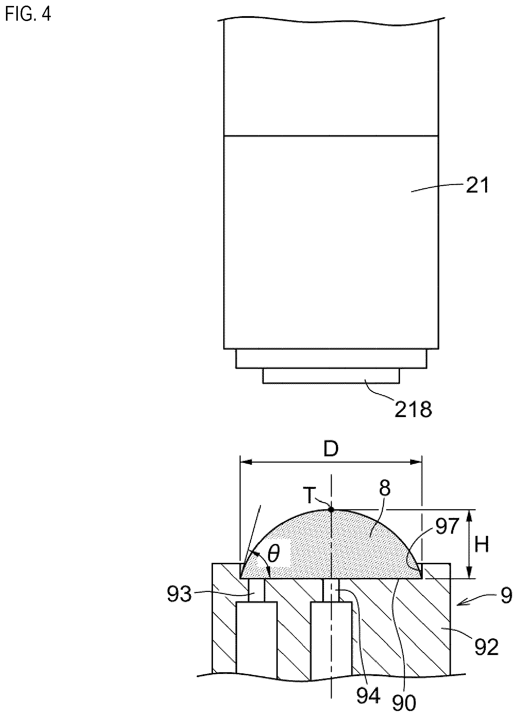

The droplet forming surface 90 is a surface on which a droplet 8 is formed by feeding a liquid such as a washing liquid or a standard liquid. Although the droplet 8 is not particularly limited, and is formed to have a convex shape toward the electrode type sensor 21 side (upper side in the present embodiment) so as to have one apex T, as shown in FIG. 4. It is preferable that the droplet forming surface 90 has a size such that, in a plan view, at least an area of the surface of the electrode-type sensor 21 in which the electrode 218 exists is located inside the outer peripheral edge of the droplet forming surface 90.

The droplet forming surface 90 is provided with a liquid supply hole 93 for supplying the liquid onto the droplet forming surface 90, and a liquid discharge hole 94 for discharging the liquid from the droplet forming surface 90. Inside of the projection 92, as shown in FIGS. 3B and 3C, is provided with a first pipe attachment part 95 to which the liquid supply pipe can be mounted in communication with the liquid supply hole 93, and a second pipe attachment part 96 to which a liquid discharge pipe can be attached in communication with liquid discharge hole 94. Note that the first pipe attachment part 95 and the second pipe attachment part 96 do not have to be provided inside the projection 92.

The liquid discharge hole 94 is arranged at the center of the droplet forming surface 90. In this way, when the droplet 8 is removed from the droplet forming surface 90 via the liquid discharge hole 94, the liquid can be uniformly discharged and the droplet 8 can be effectively removed while suppressing liquid residue. Note that the liquid discharge hole 94 does not necessarily have to be arranged at the center of the droplet forming surface 90, but can be arranged at an appropriate position on the droplet forming surface 90.

The liquid supply hole 93 is arranged near the outer peripheral edge of the droplet forming surface 90. In the present embodiment, a wall surface 97 stands on the outer peripheral edge of the droplet forming surface 90. Therefore, the top T of the droplet 8 can be located on the center of the drop formation surface 90 by forming the droplet 8 on the droplet forming surface 90 to a size reaching the outer peripheral edge of the droplet forming surface 90, even if the liquid supply hole 93 is arranged in the vicinity of the outer peripheral edge of the droplet forming surface 90. Note that the liquid supply hole 93 also may be arranged at the center of the droplet forming surface 90, in which case the top of the droplet 8 may be positioned above the center of the droplet forming surface 90 without providing the wall surface 97 at the outer peripheral edge of the droplet forming surface 90. It is possible to prevent the droplet 8 from spilling from the droplet forming surface 90 by providing the wall surface 97 on the outer peripheral edge of the droplet forming surface 90.

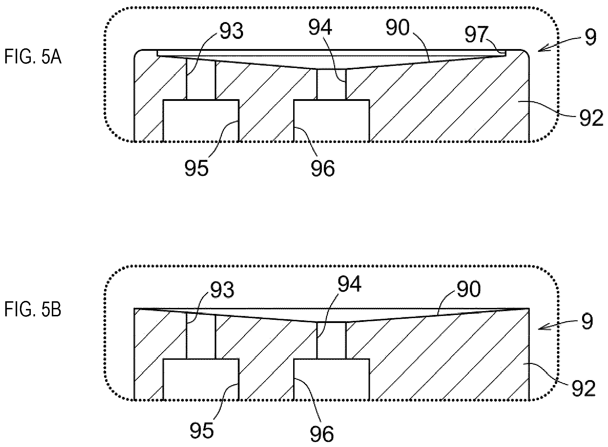

The droplet forming surface 90 is not particularly limited, but is preferably a smooth surface having no unevenness. The droplet forming surface 90 also may be a flat surface as shown in FIG. 3C insofar as there is no unevenness, or may be an inclined surface that inclines higher progressively from the center to the outer peripheral edge, shown in FIGS. 5A and 5B. The liquid existing on the outer peripheral edge (boundary with the wall surface 97) of the droplet forming surface 90 is easily discharged from the liquid discharge hole 94 when removing the droplet 8 of liquid from the droplet forming surface 90 via the liquid discharge hole 94 by making the droplet forming surface 90 an inclined surface. Hence, the droplet 8 can be effectively removed from the droplet forming surface 90, and residual liquid can be suppressed.

Spilling of the droplet 8 from the droplet forming surface 90 may be suppressed, as shown in FIG. 5B, by forming the droplet forming surface 90 as an inclined surface that progressively inclines higher from the center toward the outer peripheral edge, such that the wall surface 97 does not necessarily have to be provided on the outer peripheral edge of the droplet forming surface 90.

Although the size (diameter D) of the droplet 8 shown in FIG. 4 is not particularly limited, the diameter is preferably such that at least the region of the surface of the electrode-type sensor 21 in which the electrode 218 is present fits inside the outer peripheral edge in plan view. Although the height H of the droplet 8 shown in FIG. 4 is not particularly limited, the height H preferably exceeds the wall surface 97 of the outer peripheral edge of the droplet forming surface 90. Note that the height is preferably higher than the outer peripheral edge of the droplet forming surface 90 when the wall surface 97 is not provided at the outer peripheral edge of the droplet forming surface 90.

Next, the procedure of the pretreatment method of this embodiment will be described. First, in ST1 of FIG. 1 shown in FIG. 2A, the liquid is supplied from the liquid supply hole 93 onto the droplet forming surface 90 of the counterpart object 9 using a liquid supply means such as a pump to form the droplet 8. In the present embodiment, the droplet 8 is formed so as to be convex toward the electrode sensor 21 such that the top portion T is located on the center of the droplet forming surface 90. The droplet 8 also is formed to a size reaching the outer peripheral edge of the droplet forming surface 90, and is formed at a height exceeding the wall surface 97 around the droplet forming surface 90. The droplet 8 also may be formed by sending a fixed amount of liquid onto the droplet forming surface 90 at one time, or may be formed step-wise by sending the liquid multiple times.

When the droplet 8 is formed on the droplet forming surface 90 of the counterpart object 9, the droplet 8 is formed on the surface of the electrode type sensor 21 by the relative movement between the droplet 8 and the electrode type sensor 21 press the droplet 8 as shown in ST2 of FIG. 1. Relative movement between the droplet 8 and the electrode sensor 21 means movement of at least one of the droplet 8 and the electrode sensor 21 so that the droplet 8 and the surface of the electrode sensor 21 approach each other. In the present embodiment, as shown in FIGS. 2A and 2B, the distance between the surface of the electrode-type sensor 21 and the droplet forming surface 90 is narrowed such that the surface of the droplet forming surface 90 is brought close to the droplet 8 by moving the electrode type sensor 21 in the direction facing the counterpart object 9 (downward in the present embodiment). The droplet 8 also may be brought close to the surface of the electrode type sensor 21 by moving the counterpart object 9 in the direction facing the electrode type sensor 21 (upward in this embodiment). The surface of the electrode type sensor 21 and the droplet 8 also may be brought close to each other by mutually moving both the electrode type sensor 21 and the counterpart object 9 in the directions facing each other.

ST2 of FIG. 1 includes a step (ST2-1) of pressing a portion of the droplet 8 closest to the surface of the electrode type sensor 21 onto the surface of the electrode type sensor 21 by moving at least one of the electrode type sensor 21 and the counterpart object 9 in a direction facing each other as shown in FIG. 2B, and a step of (ST2-2) of narrowing the distance between the surface of the electrode type sensor 21 and the droplet forming surface 90 of the counterpart object 9 to a constant distance G by then moving at least one of the electrode type sensor 21 and the counterpart object 9 from the state of FIG. 2B in a direction facing each other, as shown in FIG. 2C. The portion of the droplet 8 closest to the surface of the electrode-type sensor 21 is the top portion T in this embodiment.

The droplet 8 contacts the surface of the electrode type sensor 21 over a broad area by being pressed by the surface of the electrode type sensor 21 and changing the droplet shape to a flat shape as shown in FIG. 2C after the top portion T of the droplet 8 abuts the surface of the electrode type sensor 21 as shown in FIG. 2B. The constant distance G in ST2-2 is preferably a distance capable of covering at least the region in which the electrode 218 is present on the surface of the electrode type sensor 21 is covered by the droplet 8 by changing the droplet 8 into a flat shape. In this way the droplet 8 can be brought into contact with the entire surface of the electrode 218.

When the droplet 8 is pressed against the surface of the electrode type sensor 21, in step ST3 of FIG. 1 the state in which the droplet 8 is pressed against the surface of the electrode type sensor 21 is maintained for a fixed time, as shown in FIG. 2D. In this way the droplet 8 can be sufficiently brought into contact with the electrode 218 on the surface of the electrode type sensor 21.

When the droplet 8 is pressed against the surface of the electrode type sensor 21 for a certain period of time, in ST4 of FIG. 1 the droplet 8 is removed by discharging the liquid through the liquid discharge holes 94 from above the droplet forming surface 90 using a pump, as shown in FIG. 2E.

Through the series of steps ST1 to ST4 (FIGS. 2A to 2E) in FIG. 1, the operation of bringing the droplet 8 of the liquid used for the pretreatment into contact with the surface of the electrode sensor 21 is completed once.

For example, when the electrode sensor 21 is washed as a pretreatment before measuring the measurement target component contained in the measurement sample by the electrode sensor 21, it is preferable that the series of steps ST1 to ST4 is performed once using a washing liquid as the liquid, and thereafter the series of steps ST1 to ST4 can be performed again using a washing liquid. The electrode 218 of the electrode sensor 21 can be effectively cleaned by repeating the series of steps ST1 to ST4 a plurality of times using the washing liquid.

For example, when preparing a calibration curve as a pretreatment before measuring the measurement target component contained in the measurement sample by the electrode type sensor 21, a series of steps ST1 to ST4 are performed using a standard solution having a known concentration of the measurement target component as the liquid. At that time, in step ST2, the measurement value of the standard solution is acquired in a state in which the droplet 8 of the standard solution is pressed against the surface of the electrode sensor 21. It is preferable that the series of steps ST1 to ST4 be performed on a plurality of standard solutions having different concentrations, and a calibration curve be created using the measurement values of the respective standard solutions obtained in step ST2. The calibration curve indicates the relationship between the measurement value acquired by the electrode sensor 21 and the concentration of the measurement target component. It is possible to accurately create a calibration curve by repeating the series of steps ST1 to ST4 a plurality of times using a plurality of standard solutions having different concentrations.

According to the pretreatment method of the present embodiment described above, for example, the droplet 8 used for the pretreatment is formed on the droplet forming surface 90 of the counterpart object 9, and the droplet 8 is brought into contact with the surface of the electrode type sensor 21 by pressing the droplet 8 on the surface of the electrode type sensor 21. In this way, even if bubbles are mixed in when the droplets 8 are formed, the bubbles float on the surface of the droplets during the formation of the droplet 8 and the droplet 8 is pressed against the surface of the electrode type sensor 22, such that the bubbles escape or break from the surface of the droplet. Hence, since the presence of bubbles between the surface of the electrode type sensor 21 and the electrode 218 can be suppressed when the droplet 8 used for the pretreatment is brought into contact with the surface of the electrode type sensor 21, the possibility of causing various problems in pretreatment is reduced, and pretreatment performance such as cleaning efficiency and calibration accuracy can be improved. Since the amount of bubbles mixed in the droplet 8 is small, it also is possible to prevent the amount of the liquid used for the pretreatment from increasing, so that the pretreatment can be performed with a lesser amount of the liquid.

In addition, according to the pretreatment method of the present embodiment, the pretreatment performance can be improved since the fresh droplet 8 is always in contact with the surface of the electrode type sensor 21.

According to the pretreatment method of the present embodiment, for example, the pretreatment can be performed with a simple structure in which the droplet 8 used for pretreatment is formed on the droplet forming surface 90 of the counterpart object 9 and the droplet 8 is pressed against the surface of the electrode type sensor 21. Hence, the in-vivo component measuring device including the electrode type sensor 21 can be made compact.

In addition, according to the pretreatment method of the present embodiment, the surface of the electrode type sensor 21 is covered by the droplet 8 when the droplet 8 comes into contact with the surface of the electrode type sensor 21 and the shape of the droplet 8 is changed to a flat shape. Hence, the liquid used for the pretreatment can be brought into contact with the surface of the electrode sensor 21 over a wide range.

According to the pretreatment method of the present embodiment, after the portion T of the droplet 8 nearest the surface of the electrode type sensor 21 makes contact therewith such that the droplet 8 is pressed on the surface of the electrode sensor 21, the droplet 8 is widely spread over the surface of the electrode-type sensor 21 in a plane via the applied pressure and comes into contact with the surface of the electrode-type sensor 21. For this reason, it is possible to favorably prevent bubbles from remaining between the surface of the electrode sensor 21 and the droplet 8 since the droplet 8 comes into contact from the center of the electrode sensor 21.

According to the pretreatment method of the present embodiment, the droplet 8 also is formed in such a size that the surface of the electrode type sensor 21 can be accommodated inside the outer peripheral edge thereof in plan view. Therefore, when the surface of the electrode type sensor 21 hits the droplet 8 and the shape of the droplet 8 is deformed into a flat shape, the surface of the electrode type sensor 21 is satisfactorily covered with the droplet 8. Furthermore, since the droplet 8 is formed at a height exceeding the wall surface 97 of the counterpart object 9, the surface of the electrode type sensor 21 can be satisfactorily pressed against the droplet 8.

Modification of Pretreatment Method

Although one embodiment of the pretreatment method has been described above, the pretreatment method of the present invention is not limited to the above embodiment, and various modifications may be made without departing from the spirit of the present invention. For example, the following changes are possible.

Regarding the relative movement of the droplet 8 and the electrode type sensor 21 when the droplet 8 is pressed against the surface of the electrode type sensor 21 in ST2 of FIG. 1, the above embodiment shows an example in which the electrode type sensor 21 is moved and/or the droplet 8 is moved in accordance with the movement of the counterpart object 9. However, the movement of the droplet 8 is not limited to the movement of the counterpart object 9, and includes displacing the surface of the droplet in the direction facing the electrode sensor 21 by increasing the liquid amount of the droplet 8 formed on the droplet forming surface 90 of the counterpart object 9 and gradually expanding the droplet 8 toward the electrode type sensor 21 side.

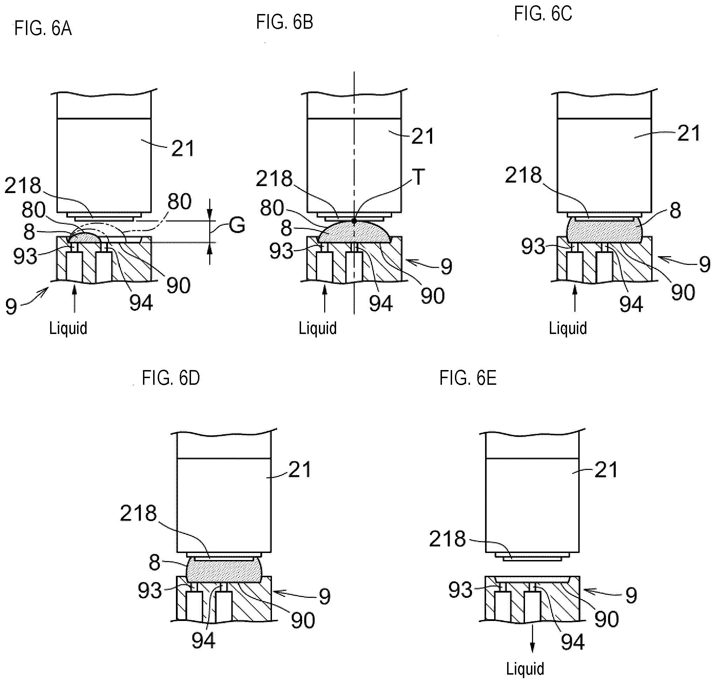

Specifically, as shown in FIG. 6A, a droplet is formed on the droplet forming surface 90 of the counterpart object 9 by feeding the liquid from the liquid supply holes 93 using a liquid feeding means such as a pump to form the droplet 8, and the droplet 8 gradually swells and grows large as the amount of liquid increases. In this way the droplet 8 is displaced in the direction in which the droplet surface 80 faces the electrode type sensor 21 (upward in this embodiment), and when the liquid amount of the droplet 8 reaches a certain amount, the portion of the droplet 8 closest to the surface of the electrode type sensor 21, that is, the top portion T in the present embodiment, abuts on the surface of the electrode type sensor 21 (ST1 and ST2-1 in FIG. 1), as shown in FIG. 6B. The droplet 8 also may be formed by sending a predetermined amount of liquid onto the droplet forming surface 90 at once, or may be formed step-wise by sending it a plurality of times.

The distance between the surface of the electrode-type sensor 21 and the droplet forming surface 90 of the counterpart object 9 is not particularly limited, but is preferably a constant distance G in the above embodiment.

As shown in FIG. 6C, the shape of the droplet 8 is changed to a flat shape by pressing against the surface of the electrode type sensor 21 such that the surface of the electrode type sensor 21 is covered by the droplet 8 (FIG. ST2-2 of 1) by further increasing the amount of liquid a certain amount after the top portion T of the droplet 8 abuts the surface of the electrode type sensor 21. In this way the droplet 8 comes into contact with the surface of the electrode sensor 21 over a wide range. It is preferable that the liquid amount of the droplet 8 to be increased in ST2-2 is set such that the droplet 8 covers at least the region containing the electrode 218 of the surface of the electrode type sensor 21 by changing the shape droplet 8 to a flat shape. In this way the droplet 8 can be brought into contact with the entire surface of the electrode 218.

Also in the present embodiment, even if bubbles are mixed in when the droplet 8 is formed, the bubbles float on the droplet surface 80 during the formation of the droplet 8 and the droplet on the surface of the electrode type sensor 21 such that the bubbles escape or break out of the droplet surface 80 by the droplet 8 being pressed against the surface of the electrode type sensor 21. Hence, when the liquid used for pretreatment is brought into contact with the surface of the electrode type sensor 21, it is possible to suppress the presence of bubbles between the electrode 218 on the surface of the electrode type sensor 21, thereby reducing the risk of causing various problems in pretreatment and improving pretreatment performance such as cleaning efficiency and calibration accuracy. Since the amount of bubbles mixed in the droplet 8 is small, it also is possible to avoid increasing the amount of the liquid used for the pretreatment, so that the pretreatment can be performed with a lesser amount of the pretreatment liquid.

When the droplet 8 is pressed against the surface of the electrode type sensor 21, the state in which the droplet 8 is pressed against the surface of the electrode type sensor 21 is maintained for a certain period of time (ST3 of FIG. 1), as shown in FIG. 6D. In this way the liquid can be sufficiently brought into contact with the surface of the electrode sensor 21.

When the state in which the droplet 8 is pressed against the surface of the electrode type sensor 21 is maintained for a certain time, the droplet 8 is removed by discharging the liquid from the droplet forming surface 90 through the liquid discharge hole 94 using a pump (ST4 in FIG. 1), as shown in FIG. 6E.

In the embodiment shown in FIG. 6, the operation which brings the droplet 8 used for the pretreatment into contact with the surface of the electrode sensor 21 by the series of steps ST1 to ST4 of FIG. 1 (FIGS. 6A to 6E) is completed once. For example, when the electrode sensor 21 is washed as a pretreatment before measuring the measurement target component contained in the measurement sample by the electrode sensor 21, it is preferable that the series of steps ST1 to ST4 is performed once using a washing liquid as the liquid, and thereafter the series of steps ST1 to ST4 can be performed again using a washing liquid. Further, when, for example, preparing a calibration curve as a pretreatment before measuring the measurement target component contained in the measurement sample by the electrode type sensor 21, it is preferable to perform the series of steps ST1 to ST4 for a plurality of standard solutions having different concentrations, and create a calibration curve using the measured values of each standard solution obtained in step ST2.

When the series of steps ST1 to ST4 in FIG. 1 are repeated a plurality of times as pretreatment, the first operation is performed by the procedure of FIGS. 2A to 2E of the above-described embodiment, and the second and subsequent operations are performed by the procedure of FIGS. 6A to 6E of the modified example.

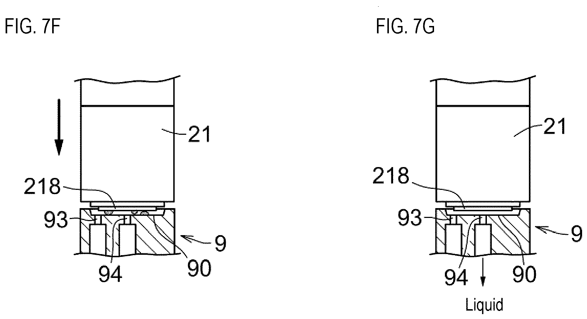

In the above-described embodiment, when there is concern the liquid may remain on the surface of the electrode type sensor 21 or the droplet forming surface 90 of the counterpart object 9 after the droplet 8 is removed in ST4 of FIG. 1, a step of removing the remaining liquid may be performed, as shown in FIG. 7.

Specifically, as shown in FIG. 7F, at least one of the electrode type sensor 21 and the counterpart object 9 moves in a direction in which they face each other, so that the surface of the electrode type sensor 21 and the droplet forming surface 90 approach each other. Although the electrode type sensor 21 moves in the direction facing the counterpart object 9 (downward in the present embodiment) to bring the surface of the electrode type sensor 21 close to the droplet forming surface 90 in the present embodiment, the counterpart object 9 also may move in a direction facing the electrode type sensor 21 (upward in this embodiment), or both the electrode type sensor 21 and the counterpart object 9 may move in a direction facing each other.

In this state, as shown in FIG. 7G, the remaining liquid is removed by discharging the liquid adhering to the surface of the electrode type sensor 21 and the droplet forming surface 90 from the liquid discharge hole 94 using a pump.

According to the embodiment of FIG. 7, in the measurement by the electrode type sensor 21 after the pretreatment, it is possible to suppress the liquid used for the pretreatment from being mixed in the measurement sample since it is possible to reliably remove the liquid after using it for pretreatment.

When the series of steps ST1 to ST4 of FIG. 1 are repeated a plurality of times as a pretreatment, the steps of FIGS. 7F and 7G may be performed each time the series of steps ST1 to ST4 are completed, or may be performed only once after the series of steps ST1 to ST4 is completed a plurality of times.

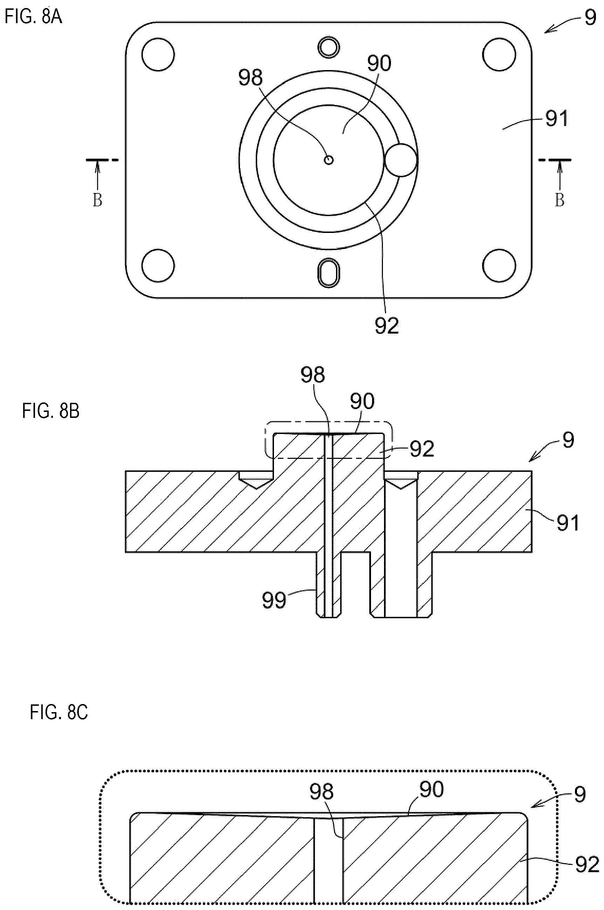

In the embodiment described above, the counterpart object 9 is provided with two holes, a liquid supply hole 93 and a liquid discharge hole 94, on the droplet forming surface 90, as shown in FIG. 3. The counterpart object 9 is not limited to this configuration, however, inasmuch as only a single liquid supply/discharge hole 98 also may be provided on the droplet forming surface 90, such that the liquid may be supplied to and discharged from the droplet forming surface 90 via the liquid supply/discharge hole 98, as shown in FIG. 8. Although the liquid supply/discharge hole 98 can be arranged at an appropriate position on the droplet forming surface 90, it is preferably arranged at the center of the droplet forming surface 90 as shown in FIG. 8A. In the embodiment of FIG. 8, although the droplet forming surface 90 is an inclined surface that inclines higher from the center of the droplet forming surface 90 toward the outer peripheral edge and no wall surface is provided on the outer peripheral edge, as shown in FIG. 8C, the droplet forming surface 90 also may be provided with a wall surface on the outer peripheral edge, or may be a flat surface instead of an inclined surface. In the embodiment shown in FIG. 8, the liquid supply/discharge hole 98 penetrates the projection 92 and the base 91 of the counterpart object 9 as shown in FIG. 8B, and on the lower surface of the base 91, there is provided a pipe mounting portion 99 which is in communication with the liquid supply/discharge hole 98 and to which a liquid supply/discharge pipe for supplying/discharging liquid to/from the droplet forming surface 90 can be attached. Note that the pipe mounting portion 99 also may be provided inside the protrusion 92 or the base 91.

In-vivo Component Measuring Device

Next, an in-vivo component measuring device employing the above-described pretreatment method will be described. In the present embodiment, an in-vivo component measuring device for measuring an area under the blood glucose-time curve (hereinafter referred to as "blood glucose AUC") will be described as an example. The blood glucose AUC is an area (unit: mg·h/dl) surrounded by a curve and a horizontal axis drawn in a graph showing blood glucose level over time. The blood glucose AUC is an index used for determining the effects of insulin and oral agents in the treatment of diabetes. For example, the total amount of glucose circulated in the body of the subject after glucose load can be estimated by measuring a value that reflects the total amount of glucose (blood glucose) circulating in blood within a predetermined period after glucose load (postprandial) using the blood glucose AUC. The total amount of glucose circulated in the body of a subject after glucose load is extremely useful information for knowing how long the hyperglycemic state due to glucose load lasted. For example, it becomes a clue to know the secretory response speed of insulin after glucose load, or becomes a clue to know the effect when an oral diabetes drug or insulin is administered.

In order to measure blood glucose AUC with the in-vivo component measuring apparatus of the present embodiment, first, in order to collect interstitial fluid from a subject as a measurement sample, a plurality of micropores are formed using a puncture device as a process for promoting the exudation of the interstitial fluid into the skin of the subject, whereupon the interstitial fluid is exuded through the plurality of micropores. A conventionally known puncture device can be used. Note that, in addition to the method of forming a plurality of micropores in the skin of a subject using a puncture device to promote the exudation of interstitial fluid and the like also may be used.

Next, the exuded interstitial fluid is collected using a collector 110 (hereinafter referred to as "interstitial fluid collector 110") shown in FIG. 9, glucose together with electrolyte (sodium ions) contained in the interstitial fluid are accumulated in the interstitial fluid collector 110.

Next, although details will be described later, glucose and sodium ions accumulated in the interstitial fluid collector 110 are measured using an in-vivo component measuring device, and the measurement values reflecting the glucose concentration and the sodium ion concentration are acquired as the glucose concentration and the sodium ion concentration measurement. Then, the blood glucose AUC of the subject is calculated based on the measured glucose concentration and sodium ion concentration, and an analysis result including the blood glucose AUC is generated and displayed.

Note that when the subject perspires, the sweat-derived sodium ions are accumulated in the collector from the skin of the subject so that the sodium ions are superposed on the sodium ions derived from the interstitial fluid, and the sodium ion concentration is increased. Since the blood glucose AUC of the subject is measured based on the sodium ions accumulated together with glucose in the in-vivo component measuring device of the present embodiment, the reliability of the measured blood glucose AUC may be reduced when excessive sodium ions derived from sweat are collected. Therefore, in the in-vivo component measuring device of the present embodiment, the interstitial fluid collector 110 for main measurement is fixed for a predetermined time in the region where the micropores are formed on the skin of the subject using the puncture tool, and a collector 111 for sweat check (hereinafter referred to as "sweat collector 111") shown in FIG. 9 is fixed in an area without the formed micropores for a predetermined time, and the interstitial fluid exuded from the skin is collected using the interstitial fluid collector 110 and at the same time the sweat collector 111 is used to collect sweat from the skin, whereby the sodium ions contained in the sweat are accumulated in the sweat collector 111. Then, by using the in-vivo component measuring device 1, the sodium ions value derived from sweat accumulated in the sweat collector 111 is measured, and the sodium ion concentration derived from sweat is taken into consideration when the blood glucose AUC is calculated, such that the blood glucose AUC is calculated with high reliability.

The interstitial fluid collector 110 and the sweat collector 111 described above are made of, for example, a gel having a water retention property capable of retaining the interstitial fluid and sweat. The gel is not particularly limited insofar as it can collect interstitial fluid and sweat, but a gel formed from at least one hydrophilic polymer selected from a group including of polyvinyl alcohol and polyvinylpyrrolidone is preferable. Although the hydrophilic polymer forming the gel also may be polyvinyl alcohol alone or polyvinylpyrrolidone alone, or may be a mixture of both, polyvinyl alcohol alone or a mixture of polyvinyl alcohol and polyvinylpyrrolidone is preferable.

The gel can be formed by a method of crosslinking a hydrophilic polymer in an aqueous solution. The gel can be formed by a method in which an aqueous solution of a hydrophilic polymer is applied on a substrate to form a film coating, and the hydrophilic polymer contained in the film coating is crosslinked. Although chemical crosslinking methods and radiation cross-linking methods and the like are examples of cross-linking methods for the hydrophilic polymer, it is preferable to adopt the radiation cross-linking method because it is difficult for various chemical substances to be mixed in the gel as impurities.

In the present embodiment, the interstitial fluid collector 110 and the sweat collector 111 are held by a single holding sheet 112 having a rectangular shape in plan view. The holding sheet 112 is flexible and transparent, and is made of a resin material such as polyethylene terephthalate. A transparent adhesive layer 113 is formed on one surface of the holding sheet 112, and the interstitial fluid collector 110 and the sweat collector 111 are attached to the adhesive layer 113 at intervals in the longitudinal direction.

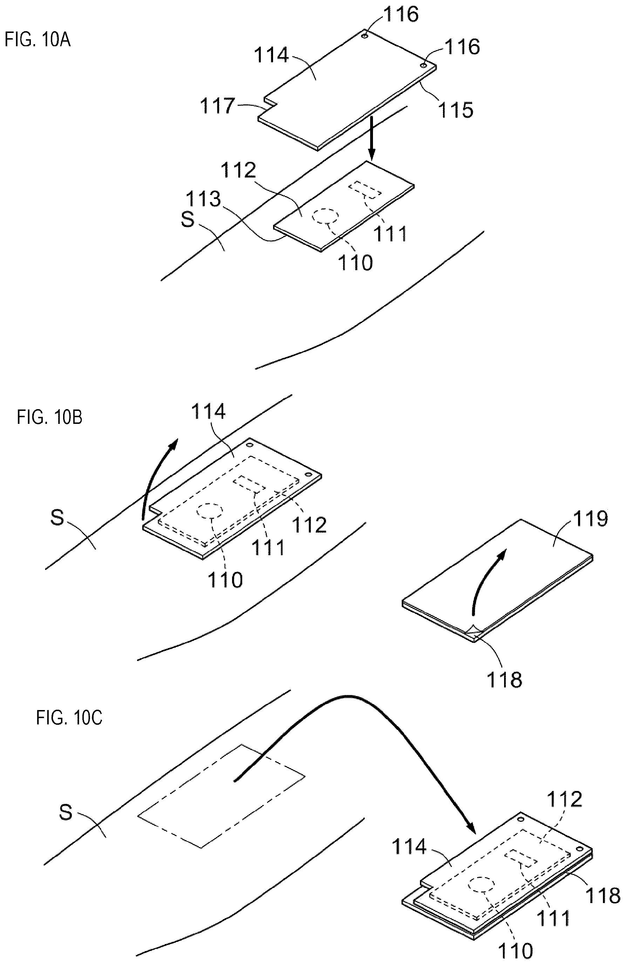

As shown in FIG. 10A, the interstitial fluid collector 110 and the sweat collector 111 are arranged so that the interstitial fluid collector 110 covers the region of the subject's skin S in which a plurality of micropores are formed,and the adhesive layer 113 of the sheet 112 is fixed to the skin S by being attached to the skin S of the subject. At this time, the sweat collector 111 is arranged in a region of the subject's skin S where a plurality of micropores are not formed.

When removing the interstitial fluid collector 110 and the sweat collector 111 from the skin S of the subject, a support sheet 114 shown in FIG. 10A can be used. The support sheet 114 has a rectangular shape in plan view and has a contour slightly larger than the holding sheet 112. The holding sheet 114 is flexible and transparent, and is made of a resin material such as polyethylene terephthalate. A transparent pressure-sensitive adhesive layer 115 is formed on one surface of the support sheet 114, and the support sheet 114 is adhered on the other surface of the support sheet 114 (on the side where the interstitial fluid collector 110 and the sweat collector 111 are not attached) by the pressure-sensitive adhesive layer 115). The adhesive force of the adhesive layer 115 of the support sheet 114 is stronger than the adhesive force of the adhesive layer 113 of the holding sheet 112, so that the adhesive layer 115 of the support sheet 114 is supported in the state of being adhered to the holding sheet 112 such that the holding sheet 112 can be smoothly removed from the skin S together with the interstitial fluid collector 110 and the sweat collector 111 by peeling the support sheet 114 from the skin S.

In the present embodiment, the interstitial fluid collector 110 and the sweat collector 111 are provided for measurement while being supported by the support sheet 114. In the support sheet 114, small-diameter through holes 116 are formed at two corners on one end side in the longitudinal direction in order to accurately perform measurement of the interstitial fluid collector 110 and the sweat collector 111. A notch 117 having a rectangular shape in plan view also is formed at one corner on the opposite end side in the longitudinal direction of the support sheet 114.

The interstitial fluid collector 110 and the sweat collector 111 removed from the skin S of the subject can be protected by a protective sheet 118 shown in FIG. 10B. The protection sheet 118 has a rectangular shape in a plan view and has a contour having substantially the same size as the support sheet 114. The holding sheet 118 is flexible and transparent, and is made of a resin material such as polyethylene terephthalate. In the state before use, the holding sheet 118 has a release film 119 attached to one surface. The release film 119 is for protecting one surface of the protective sheet 118 from contamination. The attaching surface of the release film 119 is a weakly viscous adhesive surface such that the release film 119 can be easily peeled off from the protective sheet 118. Note that one surface of the protective sheet 118 may be subjected to a release treatment such as silicon coating so that the release film 119 can be easily peeled off.

As shown in FIG. 10C, the release film 119 is peeled from the protective sheet 118, and one surface of the protective sheet 118 is superposed on the surface of the support sheet 114 on which the interstitial fluid collector 110 and the sweat collector 111 are attached, such that the protective sheet 118 is attached by the adhesive layer 115 of the support sheet 114. In this way the interstitial fluid collector 110 and the sweat collector 111 supported by the support sheet 114 are sealed by the protective sheet 118, so that the interstitial fluid collector 110 and the sweat collector 111 can be stored without being contaminated. Furthermore, when the collection place of the interstitial fluid or sweat is separated from the measurement place, the interstitial fluid collector 110 and the sweat collector 111 can be transported to the measurement place without being contaminated.

Note that the support sheet 114 and the protective sheet 118 do not necessarily have to be separate sheets. The support sheet 114 and the protection sheet 118 also may be integrated so that the support sheet 114 can be folded over the protection sheet 118.

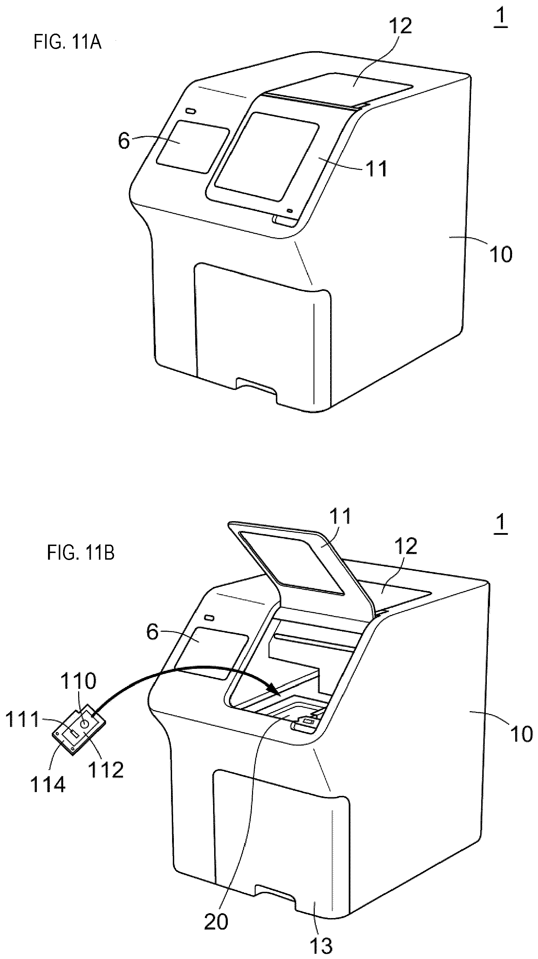

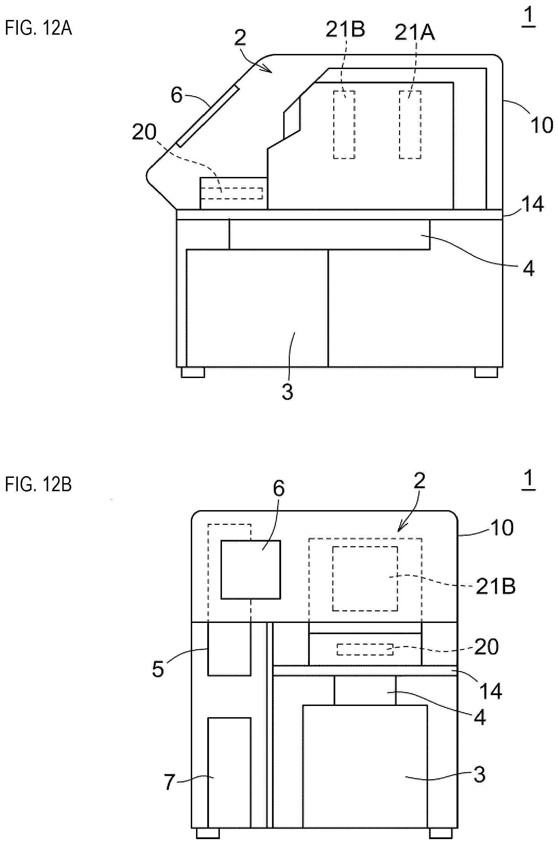

Next, the in-vivo component measuring device 1 of the present embodiment will be described. As shown in FIGS. 11 and 12, the in-vivo component measuring device 1 is configured by at least a detection unit 2, a reagent storage unit 3, a fluid circuit unit 4, a control unit 5, an operation display unit 6, and a power supply 7, and these parts are provided within a housing 10. Note that the reagent storage unit 3 does need not be provided in the housing 10. In this case, various tanks 30 to 34 described later are installed outside the housing 10, and the various tanks 30 to 34 are connected to the fluid circuit unit 4.

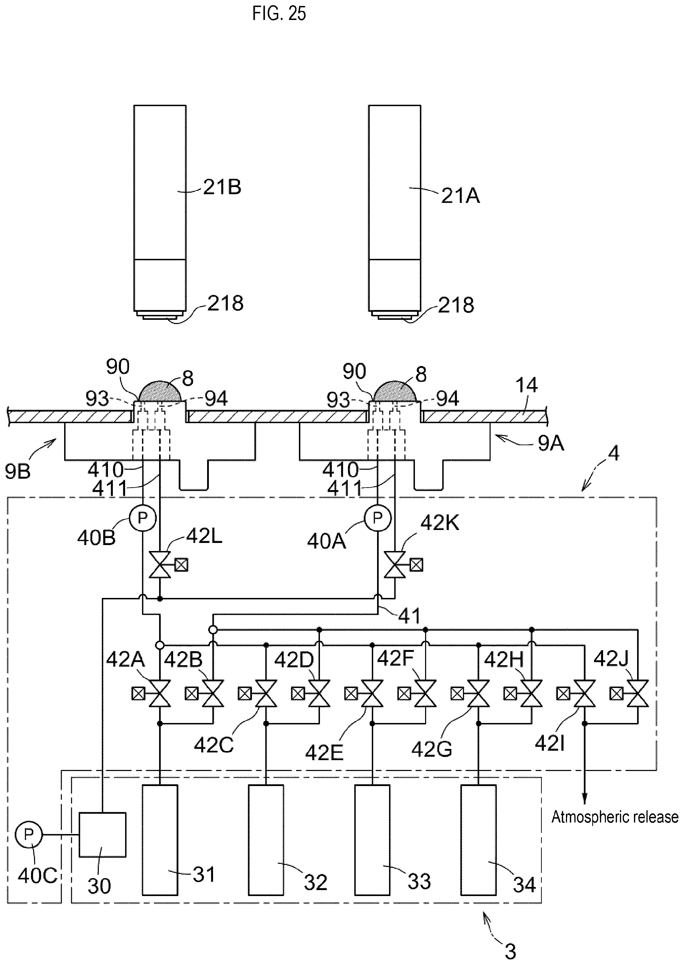

A first cover 11 is provided on the front upper portion of the housing 10 at a position adjacent to the operation display unit 6. The first cover 11 is a push-open type cover; when the first cover 11 is pushed, the first cover 11 stands up and changes from the closed state shown in FIG. 11A to the open state shown in FIG. 11B to expose the installation unit 20 for installing the 110 and the sweat collector 111. A second cover 12 is provided on the upper surface of the housing 10. The second cover 12 is also a push-open type cover; when pushed, the second cover 12 stands up and changes from the closed state to the open state to expose the glucose sensor 21A and the sodium ion sensor 21B of the detection unit 2 described later. A third cover 13 is provided on the lower front surface of the housing 10. When the third cover 13 is opened, the various tanks 30 to 34 of the reagent storage section 3 shown in FIG. 25 are exposed.

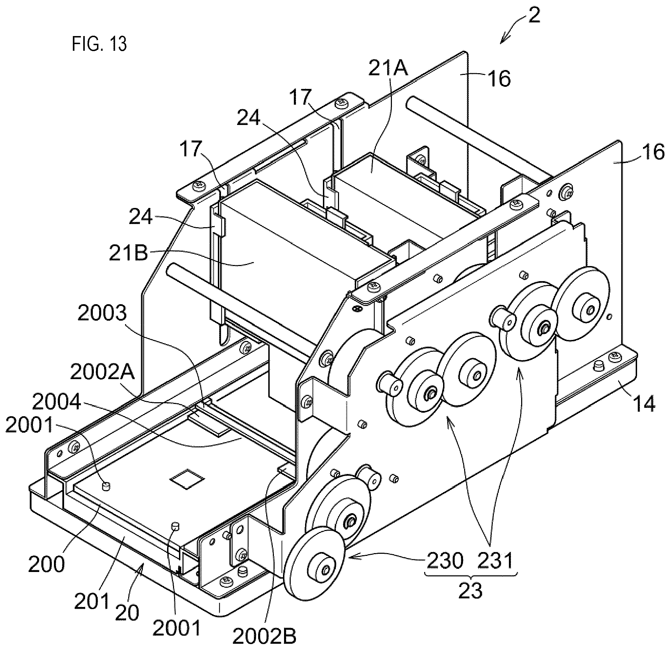

The detection unit 2 measures the components (glucose and sodium ions) contained in the interstitial fluid collected in the interstitial fluid collector 110 and the components (sodium ions) contained in the sweat collected in the sweat collector 111. As shown in FIG. 13, the detection unit 2 includes the installation unit 20 on which the interstitial fluid collector 110 and the sweat collector 111 are installed, a glucose sensor 21A that measures glucose contained in the interstitial fluid in a state of contact with the interstitial fluid collector 110 installed in the installation unit 20, a sodium ion sensor 21B that measures sodium ions contained in the interstitial fluid or sweat in a state of contact with the interstitial fluid collector 110 or the sweat collector 111, a drive unit 23 for bringing the respective collectors 110 and 111 installed in the installation unit 20 into contact with the respective sensors 21A and, 21B.

The interstitial fluid collector 110 and the sweat collector 111 are installed in the installation unit 20. The installation unit 20 includes a sample plate 200 on which the support sheet 114 supporting the interstitial fluid collector 110 and the sweat collector 111 is placed, and a sample stage 201 on which the sample plate 200 is installed.

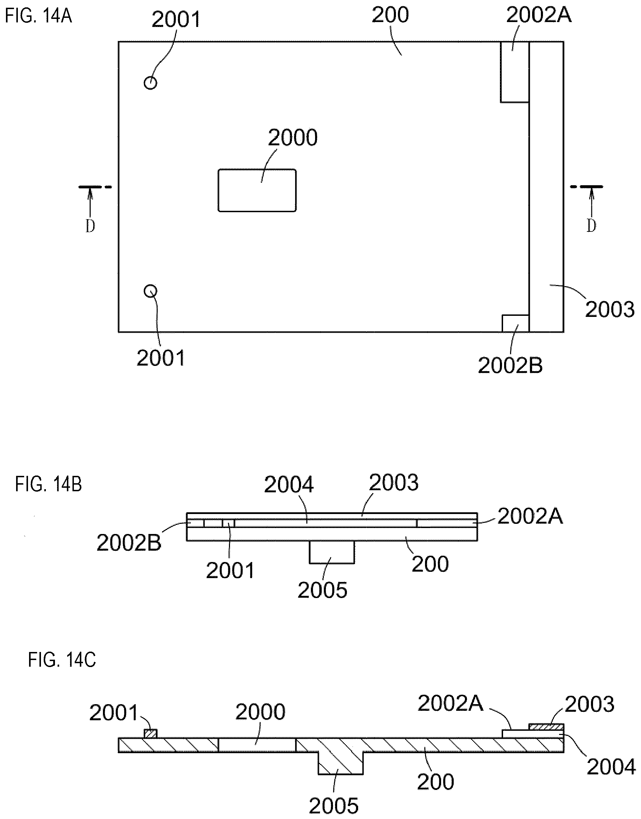

As shown in FIG. 14, the sample plate 200 has a rectangular shape in a plan view and has a contour slightly larger than the support sheet 114. The upper surface of the sample plate 200 is a flat surface, and the support sheet 114 can be stably placed on the sample plate 200. In this way, the sensors 21A and 21B can be brought into good contact with the interstitial fluid collector 110 and the sweat collector 111, respectively, when the interstitial fluid collector 110 and the sweat collector 111 are measured by the sensors 21A and 21B.

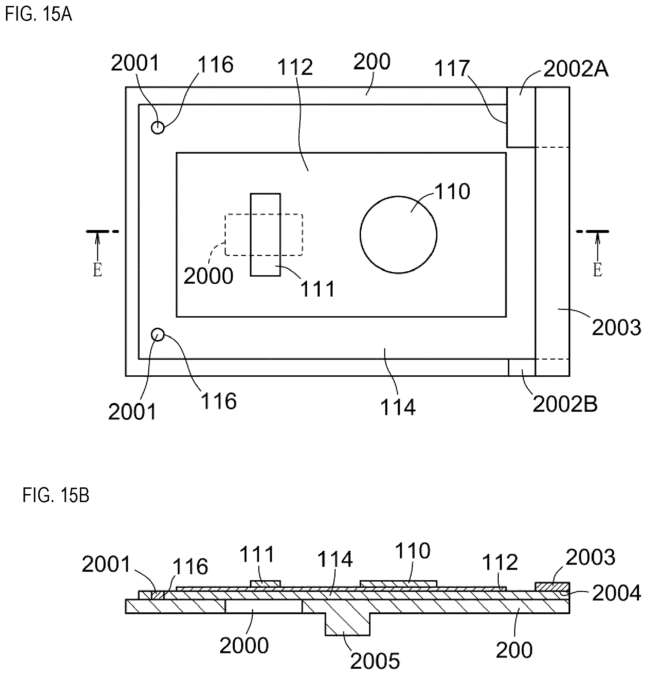

Small protrusions 2001 are provided at two corners of the sample plate 200 on one end side in the longitudinal direction. The two small protrusions 2001 function as a positioning part that positions the interstitial fluid collector 110, and when the support sheet 114 is placed on the sample plate 200, the support sheet 114 is fitted into the two through holes 116 formed in the support sheet 114, as shown in FIG. 15. In this way the support sheet 114 is placed on the sample plate 200 without displacement, so that the interstitial fluid collector 110 and the sweat collector 111 can be positioned at appropriate positions relative to the sample plate 200. As shown in FIG. 14, the thickness of the support sheet 114 and the standing walls 2002A and 2002B having the same or slightly higher height are provided at the two corners on the other end side in the longitudinal direction of the upper surface of the sample plate 200. As shown in FIG. 15, when the support sheet 114 is placed on the sample plate 200, one standing wall 2002A abuts the notch 117 of the supporting sheet 114 and the other standing wall 2002B is along the side edge of the support sheet 114 on the side opposite to the notch 117. In this way the support sheet 114 can be effectively positioned at the proper position on the sample plate 200. In this way, the sensors 21A and 21B can be brought into good contact with the interstitial fluid collector 110 and the sweat collector 111 during measurement of the interstitial fluid collector 110 and the sweat collector 111 by holding the support sheet 114 on the sample plate 200 without displacement.

On the other end side in the longitudinal direction of the sample plate 200, a horizontal bar 2003 spans between the two standing walls 2002A and 2002B, and an insertion hole 2004 is formed between the upper surface of the sample plate 200 and the horizontal bar 2003. As shown in FIG. 15, when the support sheet 114 is placed on the sample plate 200, part of the other end side in the longitudinal direction of the support sheet 114 is inserted into the insertion hole 2004. In this way the support sheet 114 is prevented from floating on the sample plate 200 by the horizontal bar 2003, and can be stably placed on the sample plate 200. In this way, the sensors 21A and 21B can be brought into good contact with the interstitial fluid collector 110 and the sweat collector 111, respectively, when the interstitial fluid collector 110 and the sweat collector 111 are measured.

In addition, as shown in FIG. 14, the sample plate 200 has a first detection hole 2000 formed at a position at which the interstitial fluid collector 110 or the sweat collector 111 is placed. An engaging convex part 2005 also is provided at the center of the lower surface of the sample plate 200.

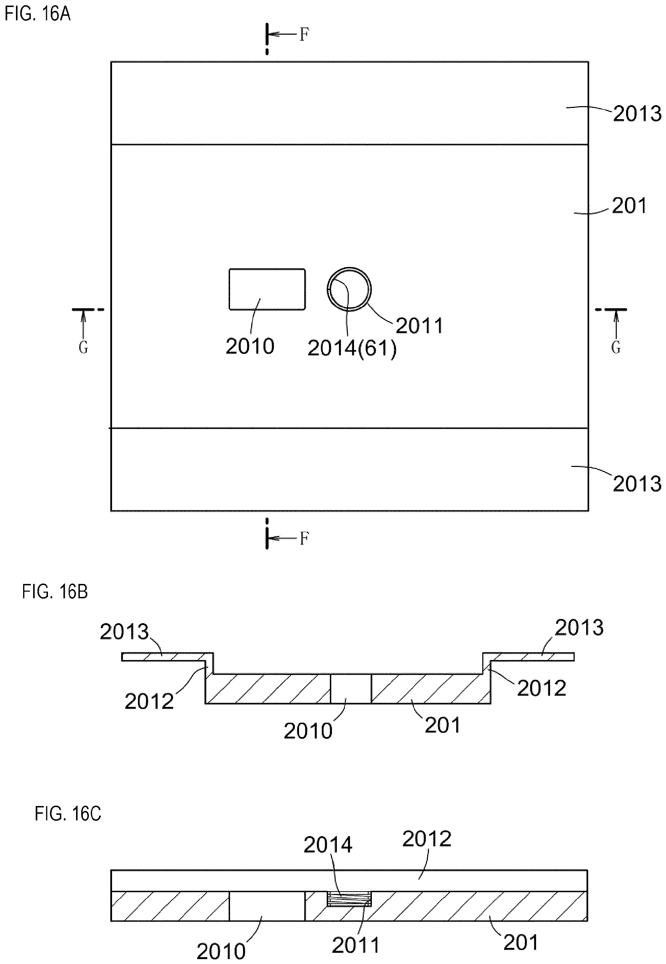

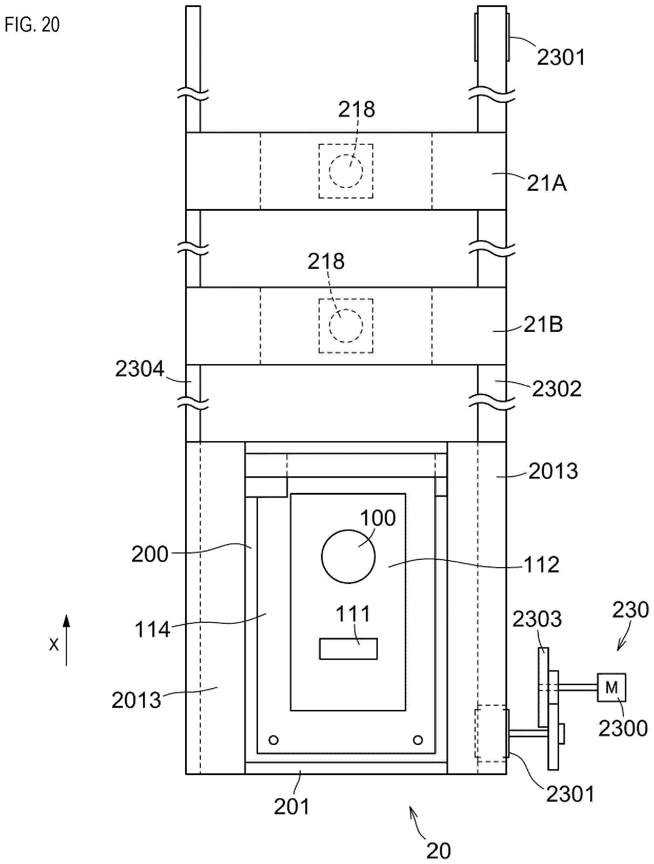

As shown in FIG. 16, the sample stage 201 has a rectangular shape in plan view and has a contour slightly larger than that of the sample plate 200. The sample plate 200 is installed on the upper surface of the sample stage 201. Side walls 2012 extending in the longitudinal direction are erected on both side edges of the upper surface of the sample stage 201, and projecting portions 2013 project horizontally outward from the upper ends of the side walls 2012. The sample stage 201 moves reciprocatingly in the X direction along the horizontal plane shown in FIG. 20 via the installation unit moving unit 230 of a drive unit 23 described later. In this way the interstitial fluid collector 110 and the sweat collector 111 are transported to positions below the sensors 21A and 21B.

An engagement recess 2011 is formed in the center of the upper surface of the sample stage 201, as shown in FIG. 16. The engagement recess 2011 is fitted with an engagement convex portion 2005 of the sample plate 200 when the sample plate 200 is installed on the sample stage 201. A pressure absorbing member 2014 is accommodated in the engagement recess 2011, and the engagement convex portion 2005 is supported by the pressure absorbing member 2014 inside the engaging concave portion 2011. A spring member such as a coil spring can be used as the pressure absorbing member 2014. The pressure absorbing member 2014 adjusts the contact pressure t a constant pressure when the sensors 21A and 21B come into contact with the interstitial fluid collector 110. When each sensor 21A and 21B come into contact with the interstitial fluid collector 110 and the sweat collector 111, the pressure absorbing member 2014 expands and contracts and the sample plate 200 is vertically displaced on the sample stage 201 such that the contact pressure of the electrode unit 212 contacting the body 111 can be adjusted to be constant pressure. In this embodiment, specifically, the contact pressure can be adjusted to 1N (tolerance ±2%~3%). Hence, even if there are variations in the shapes of the interstitial fluid collector 110 and the sweat collector 111, the electrode unit 212 can be brought into contact with the interstitial fluid collector 110 and the sweat collector 111 at a constant contact pressure. Note that the number of pressure absorbing members 2014 interposed between the sample plate 200 and the sample stage 201 is not one disposed at the center of the sample plate 200 and the sample stage 201, rather one at each of the four corners of the sample plate 200 and the sample stage 201, for example, and the installation position is not particularly limited, such as one at all four corners (four in total).

A second detection hole 2010 is formed in the sample stage 201. The second detection hole 2010 is formed at a position corresponding to the first detection hole 2000 of the sample plate 200 when the sample plate 200 is installed on the sample stage 201. The first detection hole 2000 and the second detection hole 2010 configure a detection means for confirming whether the interstitial fluid collector 110 and the like is installed on the sample stage 201.

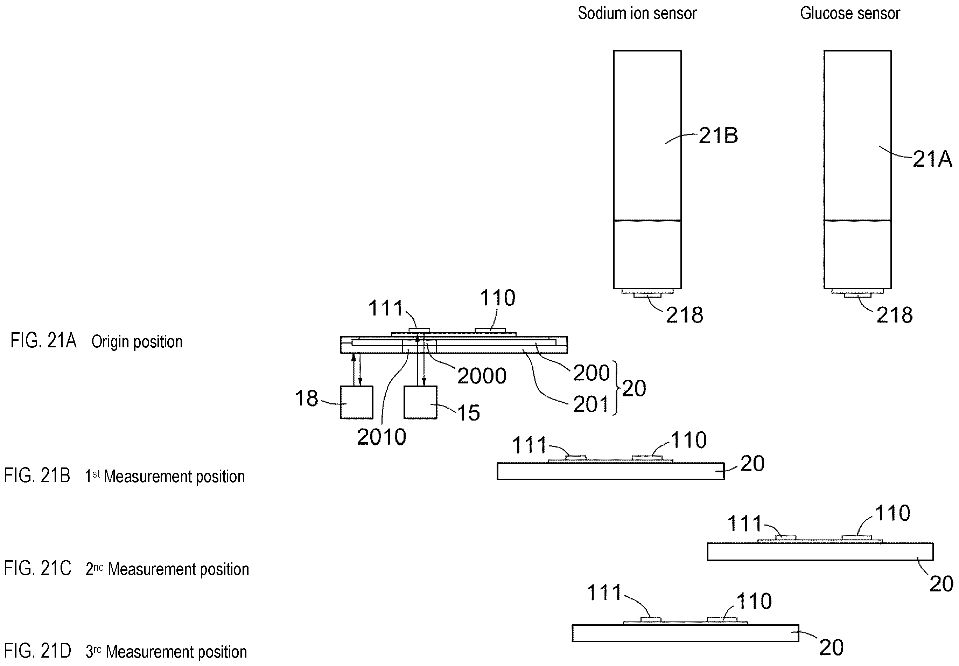

The in-vivo component measuring device 1 is provided, within the housing 10, with the collector detection sensor 15 shown in FIG. 21A. The collector detection sensor 15 configures a detecting means, and can be configured by, for example, a photo sensor or the like. The collector detection sensor 15 is arranged below the sample stage 201 so as to emit light upward at the origin position at which the sample plate 200 with the interstitial fluid collector 110 and the like are installed on the sample stage 201.

When the interstitial fluid collector 110 or the like is not installed on the sample stage 201, the light emitted from the light emitting element of the collector detection sensor 15 does not enter the light receiving element. On the other hand, when the interstitial fluid collector 110 or the like is installed on the sample stage 201, the light emitted from the light emitting element of the collector detection sensor 15 is reflected by the interstitial fluid collector 110 or the sweat collector 111 and received light receiving element. The collector detection sensor 15 is connected to the control unit 5 and outputs an electric signal to the control unit 5 when the light receiving element receives light. The control unit 5 detects that the interstitial fluid collector 110 or the like is installed on the sample stage 201 based on the electric signal from the collector detection sensor 15.

Although the collector detection sensor 15 is a reflection type photo sensor (photo reflector) in the present embodiment, it also may be a transmission type photo sensor (photo interrupter). The collector detection sensor 15 is not limited to the photo sensor, and may be any object detection sensor capable of non-contact detection that the interstitial fluid collector 110 or the like is installed on the sample stage 201.

Next, each of the sensors 21A and 21B is an electrode sensor that measures a measurement target component contained in the interstitial fluid or sweat by contacting the interstitial fluid collector 110 or the sweat collector 111. The glucose sensor 21A is an electrode sensor that measures glucose contained in interstitial fluid as a measurement target component. The sodium ion sensor 21B is an electrode sensor that measures sodium ions contained in interstitial fluid or sweat as a measurement target component.

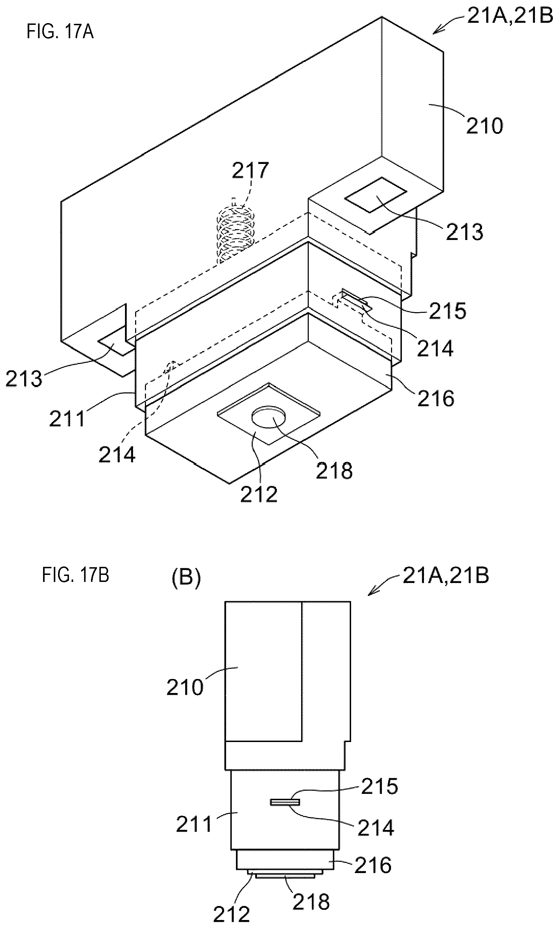

As shown in FIG. 17, each of the sensors 21A and 21B has, for example, a main body 210 made of plastic, a slide unit 211 made of plastic slidably attached to the main body 210, a cartridge unit 216 made of, for example, plastic which is removably mounted on the slide unit 211, and an electrode unit 212 attached to the bottom surface of the cartridge unit 216.

The main body 210 has a shape having an upper portion and a lower portion and a step between the upper portion and the lower portion, and a terminal 213 connected to the control unit 5 provided on the bottom surface of the upper portion. An opening is formed in the lower portion of the main body 210, and the slide unit 211 projects from this opening. A pressure absorbing member 217 is provided in the main body 210. A spring member such as a coil spring can be used as the pressure absorbing member 217. The pressure absorbing member 217 adjusts the contact pressure to a constant pressure when the sensors 21A and 21B come into contact with the interstitial fluid collector 110 and the sweat collector 111. The slide unit 211 is connected to the pressure absorbing member 217, and slides up and down relative to the main body 210 as the pressure absorbing member 217 expands and contracts. When each sensor 21A and 21B come into contact with the interstitial fluid collector 110 and the sweat collector 111, the pressure absorbing member 217 expands and contracts and the contact pressure of the electrode unit 212 which contacts the interstitial fluid collector 110 and the sweat collector 111 can be constantly adjusted by displacing the electrode unit 212 up and down. Hence, even if there are variations in the shapes of the interstitial fluid collector 110 and the sweat collector 111, the electrode unit 212 can be brought into contact with the interstitial fluid collector 110 and the sweat collector 111 at a constant contact pressure. Note that the pressure absorbing member 217 connected to the slide unit 211 in the main body 210 also may be provided in plurality rather than singly.

An opening is formed in the lower part of the slide unit 211, and the cartridge unit 216 can be attached to the lower part of the slide unit 211 through this opening. Engagement holes 215 are formed on both lower side surfaces of the slide unit 211.

The cartridge unit 216 is a consumable item that is disposable after being used for measurement of the interstitial fluid collector 110 and the like a predetermined number of times. The cartridge unit 216 is provided with a pair of locking claws 214 so as to correspond to the respective engagement holes 215 of the slide unit 211. The cartridge unit 216 is held by the slide unit 211 by the engagement claws 214 engaging with the corresponding engagement holes 215. At this time, it is preferable that the cartridge unit 216 is held so as to be oscillatable, for example, with a slight shaking without being fixedly positioned relative to the slide unit 211. The cartridge unit 216 adjusts the angle at which the sensors 21A and 21B come into contact with the interstitial fluid collector 110 and the sweat collector 111. In this way, when the sensors 21A and 21B come into contact with the interstitial fluid collector 110 and the sweat collector 111, the cartridge unit 216 oscillates relative to the slide unit 211 along the surface of the interstitial fluid collector 110 and the sweat collector 111. Hence, it becomes possible to adjust the angle of the surface of the electrode unit 212 that comes into contact with the interstitial fluid collector 110 or the sweat collector 111, and the electrode unit 212 can be brought into good contact with the interstitial fluid collector 110 and the sweat collector 111 even if there are variations in the shape of the interstitial fluid collector 110 or the sweat collector 111..

The electrode unit 212 is a rectangular plate made of an insulating material, and has an electrode 218 including a pair of working and counter electrodes and a reference electrode on its surface. The glucose measuring electrode 218 of the glucose sensor 21A has, for example, a platinum electrode as a working electrode with a glucose oxidase enzyme film formed thereon, and a counter electrode as a platinum electrode. On the other hand, in the sodium ion measuring electrode 218 of the sodium ion sensor 21B, for example, the working electrode is an ion selective electrode having a sodium ion selective membrane, and the counter electrode is a reference electrode. Note that the shape of the electrode unit 212 is not limited to a rectangular shape, and may be a circular shape, a polygonal shape, or the like. Although the shape of the electrode 218 is schematically depicted as a circular shape, the specific shape of the electrode 218 can be various shapes.

The glucose sensor 21A also includes a glucose measuring circuit as an electric circuit connected to the electrode 218, and a constant voltage is applied to the interstitial fluid collected in the interstitial fluid collector 110 via the contact of the electrode 218 with the interstitial fluid collector 110, and the electric current at that time is acquired as a detection value. This electric current value is dependent on the glucose concentration in the interstitial fluid. On the other hand, the sodium ion sensor 21B includes a sodium ion measuring circuit as an electric circuit connected to the electrode 218, and the voltages of the interstitial fluid collected by the interstitial fluid collector 110 and the sweat collected by the sweat collector 111 via the contact of the electrode 218 with the interstitial fluid collector 110 and the sweat collector 111 are acquired as detection values. The voltage value is dependent on the sodium ion concentration in the interstitial fluid and sweat. Each of the sensors 21A and 21B is connected to the control unit 5 and outputs the obtained current value or voltage value to the control unit 5 as a detection signal. The control unit 5 measures the glucose concentration and the sodium ion concentration based on the current value and voltage value included in the detection signal and a calibration curve stored in the storage unit.

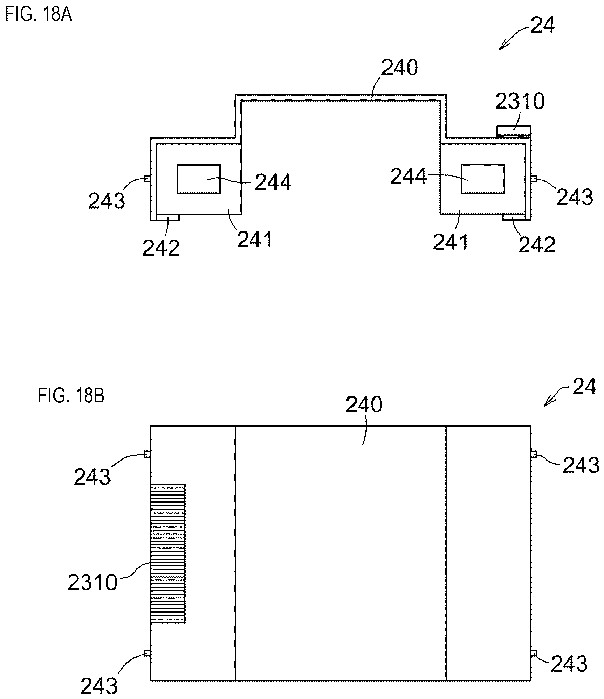

Each of the sensors 21A and 21B is set on the detection unit 2 by being mounted to the fixture 24. As shown in FIG. 18, the fixture 24 includes a frame 240 that can accommodate the upper portions of the sensors 21A and 21B, a pair of left and right support units 241 that support the lower portions of the sensors 21A and 21B from below, and a retainer 242 for holding the front and rear of the top portion of each sensor 21A and 21B via the frame 240. An opening 244 for exposing the terminal 213 of each sensor 21A and 21B is formed in each support unit 241. As shown in FIG. 13, the fixture 24 is attached to the pair of left and right side plates 16 provided on the base plate 14 of the detection unit 2 so as to be vertically movable. As shown in FIG. 18, a plurality of protrusions 243 are provided in the vertical direction on both side surfaces of the frame 240; the fixture 24 is movable straight up and down via the protrusions 243 sliding vertically in elongated guide holes 17 (shown in FIG. 13) formed on each side plate 16.

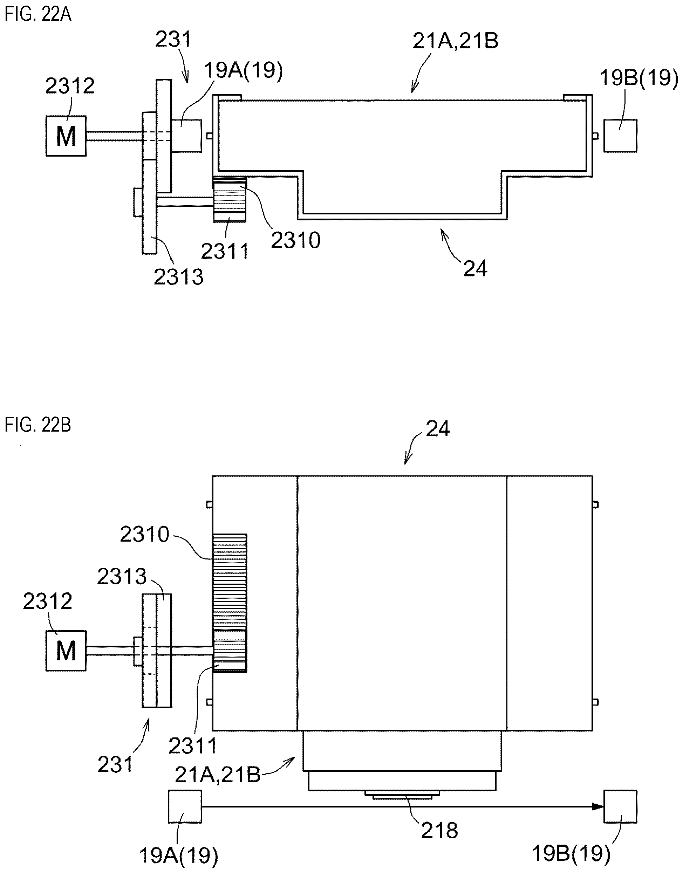

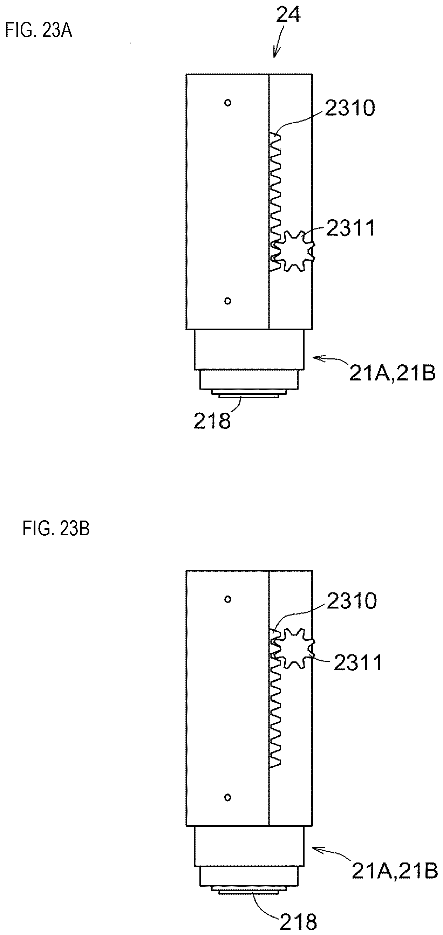

As shown in FIG. 18, a rack 2310 is provided along the vertical direction on the left and right side portions of the back surface of the frame 240. The rack 2310 configures a sensor moving unit 231 of the drive unit 23, which will be described later; a pinion gear 2311 shown in FIGS. 22 and 23 meshes with the toothed surface on the surface of the rack 2310.

Next, as shown in FIG. 13, the drive unit 23 brings the interstitial fluid collector 110 and the sweat collector 111 installed in the installation unit 20 into contact with the sensors 21A and 21B, and the installation unit 20 and the sensors 21A and 21B are moved in the present embodiment. The drive unit 23 includes an installation unit moving unit 230 that moves the installation unit 20 in the horizontal direction, and a sensor moving unit 231 that moves the sensors 21A and 21B in the vertical direction. The two sensor moving units 231 are provided in the detection unit 2 corresponding to the glucose sensor 21A and the sodium ion sensor 21B, respectively.

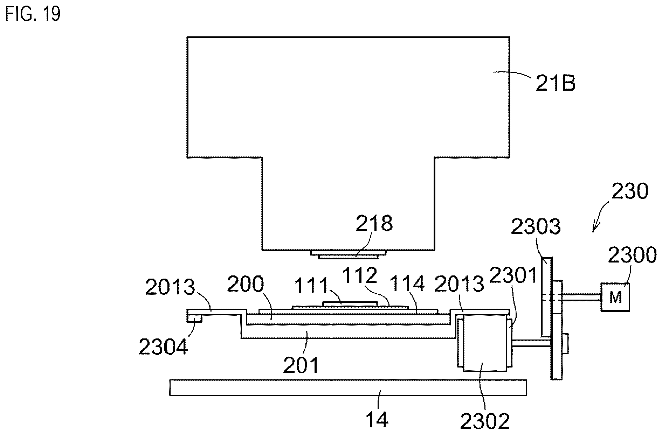

As shown in FIGS. 19 and 20, the installation unit moving unit 230 includes a motor 2300 serving as a drive source, a pair of pulleys 2301, a transmission belt 2302 spanning the pair of pulleys 2301, and a power transmission mechanism 2303 that transmits the rotational drive force of the motor 2300 to one of the pulleys 2301. shifts the force and transmits the force to one pulley 2301. In the installation unit 20, one protrusion 2013 of the sample stage 201 is fixed to the transmission belt 2302. The motor 2300 is, for example, a stepper motor that can rotate in the forward and reverse directions; the installation unit moving unit 230 runs the transmission belt 2302 only a distance according to the rotation angle (rotation speed) of the motor 2300 to transmit the transmission belt 2302, and the installation unit 20 fixed on the top of the transmission belt 2302 is reciprocatingly moved in the X direction along the horizontal plane. Note that the other protrusion 2013 of the sample stage 201 of the installation unit 20 slides on a rail 2304 provided on one side plate 16 (shown in FIG. 13) of the detection unit 2. The motor 2300 is connected to the control unit 5, and its operation is controlled by the control unit 5. Note that the stepper motor rotates only an angle corresponding to the number of drive pulses supplied thereto.

The in-vivo component measuring device 1 is provided, within the housing 10, with an origin detection sensor 18 shown in FIG. 21A. The origin detection sensor 18 can be configured by, for example, a photo sensor or the like, and is a reflective photo sensor (photo reflector) in the present embodiment. The origin detection sensor 18 is arranged below the sample stage 201 to emit light upward at the origin position where the interstitial fluid collector 110 and the sweat collector 111 can be installed. When the installation unit 20 is located at the origin position, the light emitted from the light emitting element of the origin detection sensor 18 is reflected by the sample stage 201 and enters the light receiving element. On the other hand, when the installation unit 20 is not located at the origin position, the light emitted from the light emitting element of the origin detecting sensor 18 does not enter the light receiving element. The origin detection sensor 18 is connected to the control unit 5 and outputs an electric signal to the control unit 5 when the light receiving element receives light. The control unit 5 detects that the installation unit 20 is located at the origin position based on the electric signal from the origin detection sensor 18. The control unit 5 also performs a process of setting the origin position where the installation unit 20 is detected by the origin detection sensor 18 as the horizontal origin.

Although the origin detection sensor 18 is a reflection type photo sensor (photo reflector) in the present embodiment, it also may be a transmission type photo sensor (photo interrupter). Further, the origin detection sensor 18 is not limited to a photo sensor, and may be any object detection sensor capable of contactlessly detecting that the installation unit 20 is located at the origin position.

The control unit 5 controls the installation unit moving unit 230 to move the installation unit 20 to the origin position at which the interstitial fluid collector 110 and the sweat collector 111 can be installed (FIG. 21A) at each predetermined position, and as shown in FIG.

21. Specifically, the installation unit 20 is moved from the origin position to each position, that is, a first measurement position at which the interstitial fluid collector 110 is located below the sodium ion sensor 21B (FIG. 21B), a second measurement position at which the interstitial fluid collector 110 is located below the glucose sensor 21A (FIG. 21C), and a third measurement position (FIG. 21(D) at which the sweat collector 111 is located below the sodium ion sensor 21B, by supplying a drive signal with a set number of pulses to the motor 2300. In this way the sensors 21A and 21B can contact the interstitial fluid collector 110 and the sweat collector 111, and the measurement of the interstitial fluid collector 110 and the sweat collector 111 is performed.

In this way, the installation unit 20 is transported between the origin position, the first measurement position, the second measurement position, and the third measurement position by the installation unit moving unit 230. Note that although the installation unit moving unit 230, the forward/reverse rotation of the motor 2300 is converted into a reciprocating linear movement by the transmission belt 2302 and is transmitted to the installation unit 20 to move the installation unit 20 in the horizontal direction in the present embodiment, as shown in FIG. 19, a power transmission mechanism other than the transmission belt 2302, such as a configuration in which the sample stage 201 is pushed from the rear, also may be used..

Each measurement position to which the installation unit 20 is conveyed from the origin position also may be reached by the control unit 5 giving the motor 2300 a number of drive pulses according to the distance from the origin position, and a rotary encoder may be used to further enhance the positioning accuracy.

As a method of positioning the installation unit 20 at each measurement position, an object detection sensor such as a photo sensor also may be provided at each measurement position such that the object detection sensor at each measurement position detects the installation unit 20, and thereby detect that the installation unit 20 has reached each measurement position.

As shown in FIGS. 22 and 23, a sensor moving unit 231 is provided with a rack 2310 provided on the fixture 24, a pinion gear 2311 that engages with the rack 2310, a motor 2312 serving as a drive source, and a power transmission mechanism 2313 that transmits the rotational drive force of the motor 2312 to the pinion gear 2311. The motor 2312 is, for example, a stepper motor that can rotate in forward and reverse directions, and the sensor moving unit 231 moves the fixture 24 up and down by a distance according to the rotation angle (rotation speed) of the motor 2312. The motor 2312 is connected to the control unit 5, and its operation is controlled by the control unit 5. Note that the stepper motor rotates only an angle corresponding to the number of drive pulses supplied thereto.

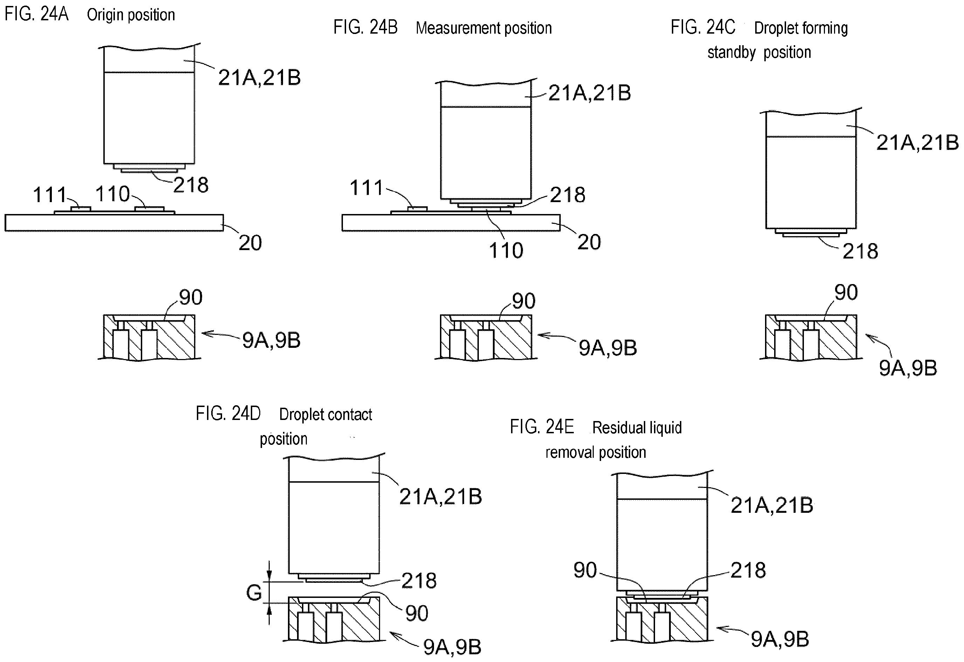

The in-vivo component measuring device 1 is provided with the origin detection sensor 19 shown in FIG. 22 on one side plate 16 (shown in FIG. 13) of the detection unit 2. The origin detection sensor 19 is configured by, for example, a photo sensor, and is a transmissive photo sensor (photo interrupter) in the present embodiment. The origin detection sensor 19 includes a pair of light emitting units 19A and light receiving units 19B, and the light receiving unit 19B is arranged to be interposed between the sensors 21A and 21B. When each of the sensors 21A and 21B is located at the origin position, the light emitted from the light emitting element of the light emitting section 19A of the origin detecting sensor 19 enters the light receiving element of the light receiving section 19B. This origin position is a standby position until there is an instruction for measurement or pretreatment unrelated to measurement of the interstitial fluid collector 110 and sweat collector 111 by the sensors 21A and 21B, washing of the sensors 21A and 21B, and pretreatment for preparation of a calibration curve used by the sensor 21A and 21B, as shown in FIG. 24A. On the other hand, when the sensors 21A and 21B are located below the origin position, the light emitted from the light emitting element of the light emitting unit 19A of the origin detection sensor 19 is blocked by the sensors 21A and 21B and the light receiving element of the light receiving unit 19B does not receive the light. The origin detection sensor 19 is connected to the control unit 5 and outputs an electric signal to the control unit 5 when the light receiving unit 19B receives light. The control unit 5 detects that the sensors 21A and 21B are located at the origin position based on the electric signal from the origin detection sensor 19. The control unit 5 also performs a process of setting the origin position detected by the origin detection sensor 19 by each of the sensors 21A and 21B as the origin in the vertical direction.

Although the origin detection sensor 19 is a transmissive photo sensor (photo interrupter) in the present embodiment, the origin detection sensor 19 also may be a reflective photo sensor (photo reflector). The origin detection sensor 19 is not limited to a photo sensor, and may be any object detection sensor capable of non-contact detection of each of the sensors 21A and 21B located at the standby position.

The control unit 5 controls the sensor moving unit 231 to move each of the sensors 21A and 21B from the origin position (FIG. 24A) to each predetermined position, as shown in FIG. 24. Specifically, by supplying the drive signals of a set number of pulses to the motor 2312, the sensors 21A and 21b are moved from the origin position to each position separated from the origin position by the set number of pulses, that is, a measurement position (FIG. 24B) at which the sensors 21A and 21B come into contact with the interstitial fluid collector 110 and the sweat collector 111 for measurement, a droplet formation standby position (FIG. 24C) for forming a droplet of a liquid used for the pretreatment which is a position for performing the pretreatment for measurement, a droplet contact position (FIG. 24D) at which a droplet of a liquid used for pretreatment is brought into contact with the surface of each sensor 21A, 21B, and a residual liquid removal position (FIG. 24E) for removing the residual liquid adhering to each sensor 21A, 21B and the like after removing the droplets used in the pretreatment. Note that the droplet contact position is a position at which the interval between the electrode 218 and the droplet forming surface 90 is narrowed to the above-mentioned constant distance G.

In this way the sensors 21A and 21B are transported between the origin position, the measurement position, the droplet formation standby position, the droplet contact position, and the residual liquid removal position by the sensor moving unit 231 as shown in FIG. 22. Although the sensor moving unit 231 converts the forward/reverse rotation of the motor 2312 into a reciprocating linear movement by the rack 2310 and the pinion gear 2311 and transmits the reciprocating linear movement to the sensors 21A, 21B in the present embodiment, a power transmission mechanism other than the rack 2310 and the pinion gear 2311 also may be used..

Each measurement position to which the sensors 21A and 21B are conveyed from the origin position also may be reached by the control unit 5 giving the motor 2312 a number of drive pulses according to the distance from the origin position, and a rotary encoder may be used to further enhance the positioning accuracy.

Next, as shown in FIG. 25, the reagent storage unit 3 includes a waste liquid tank 30, a first tank 31 that stores a cleaning liquid used for cleaning the sensors 21A and 21B as a pretreatment, a second tank 32, a third tank 33, and a fourth tank 34 for storing a standard solution used for preparing a calibration curve as a pretreatment. A PB-K solution is an example of the cleaning liquid. A PB-K solution containing glucose is an example of the standard solution. A low concentration standard liquid is stored in the second tank 32, a medium concentration standard liquid is stored in the third tank 32, and a high concentration standard liquid is stored in the fourth tank 34. Note that although the standard solution for sodium ions is the same as the standard solution for glucose in the present embodiment, different solutions such as saline solution and Tris solution also may be used. The glucose concentration and sodium ion concentration of the standard solution in each of the tanks 32 to 34 are stored in the memory storage unit of the control unit 5.

Next, as shown in FIG. 25, the fluid circuit unit 4 sends the liquid stored in each of the tanks 31 to 34 onto the droplet forming surface 90 of the counterpart objects 9A and 9B fixed to the base plate 14, the droplets 8 used for processing are formed, and the droplets 8 are then removed by discharging the liquid from the droplet forming surface 90 to the waste liquid tank 30. Note that detailed description thereof will be omitted here since the counterpart objects 9A and 9B have the same configuration as the counterpart object 9 shown in FIG. 3 described in the above-described pretreatment method. The fluid circuit unit 4 includes pumps 40A to 40C, a tube 41 such as a pipe, and a plurality of solenoid valves 42A to 42L.

The pump 40A has the function of sending liquid of each tank 31 to 34 onto the droplet forming surface 90 of the counterpart object 9A (hereinafter, referred to as a first counterpart object 9A) facing the surface of the glucose sensor 21A, and forming the droplet 8 of the liquid. The pump 40B has the function of sensing liquid of each tank 31 to 34 onto the droplet forming surface 90 of the counterpart object 9B (hereinafter, referred to as the second counterpart object 9B) facing the surface of the sodium ion sensor 21B, and forming the droplet 8 of the liquid. The pump 40C has a function of discharging the liquid from the droplet forming surface 90 of each of the counterpart objects 9A and 9B, and removing the droplet 8 of the liquid. The pipe 41 includes a liquid supply passage 410 and a liquid discharge passage 411 corresponding to each of the counterpart objects 9A and 9B; the liquid supply passage 410 is connected to the liquid supply holes 93 of the counterpart objects 9A and 9B, and a liquid discharge path 411 is connected to the liquid discharge holes 94 of the counterpart objects 9A and 9B. Each of the solenoid valves 42A to 42L has a function of switching between opening and closing of the flow path in which the pipe 41 is arranged. Each of the pumps 40A to 40C and each of the solenoid valves 42A to 42L is connected to the control unit 5, and the operations thereof are controlled by the control unit 5.

The first counterpart object 9A is located in a direction facing the electrode 218 of the glucose sensor 21A. In the present embodiment, the first counterpart object 9A is arranged below the electrode 218 of the glucose sensor 21A so that the droplet forming surface 90 faces the electrode 218 with a space therebetween. The second counterpart object 9B is located in a direction facing the electrode 218 of the sodium ion sensor 21B. That is, the second counterpart object 9B is arranged below the electrode 218 of the sodium ion sensor 21B so that the droplet forming surface 90 faces the electrode 218 with a space therebetween.

In the present embodiment, the respective sensors 21A and 21B can be moved by the sensor moving unit 231 in the direction facing the corresponding counterpart objects 9A and 9B (downward in the present embodiment). The movement of each sensor 21A, 21B causes the surface of each sensor 21A and 21B to approach the droplet 8 of the liquid on the droplet forming surface 90 of the corresponding counterpart object 9A and 9B, and droplets 8 can be pressed against the surface of the sensor 21A and 21B and brought into contact thereby.

The first tank 31 is connected to the liquid supply hole 93 of the first counterpart object 9A via the electromagnetic valve 42B, and connected to the liquid supply hole 93 of the second counterpart object 9B via the electromagnetic valve 42A, such that the cleaning liquid is supplied onto the droplet forming surface 90 via the respective liquid supply hole 93. The second tank 32 is connected to the liquid supply hole 93 of the first counterpart object 9A via the electromagnetic valve 42D, and connected to the liquid supply hole 93 of the second counterpart object 9B via the electromagnetic valve 42C, such that the low concentration calibration liquid is supplied onto the droplet forming surface 90 via the respective liquid supply hole 93. The third tank 33 is connected to the liquid supply hole 93 of the first counterpart object 9A via the electromagnetic valve 42F, and connected to the liquid supply hole 93 of the second counterpart object 9B via the electromagnetic valve 42E, such that the medium concentration calibration liquid is supplied onto the droplet forming surface 90 via the respective liquid supply hole 93. The fourth tank 34 is connected to the liquid supply hole 93 of the first counterpart object 9A via the electromagnetic valve 42H, and connected to the liquid supply hole 93 of the second counterpart object 9B via the electromagnetic valve 42G, such that the high concentration calibration liquid is supplied onto the droplet forming surface 90 via the respective liquid supply hole 93. The waste liquid tank 30 is connected to the liquid discharge hole 94 of the first counterpart object 9A via the electromagnetic valve 42K and to the liquid discharge hole 94 of the second counterpart object 9B via the electromagnetic valve 42L, respectively, such that the liquid discharged from the droplet forming surfaces 90 of 9A and 9B is collected.

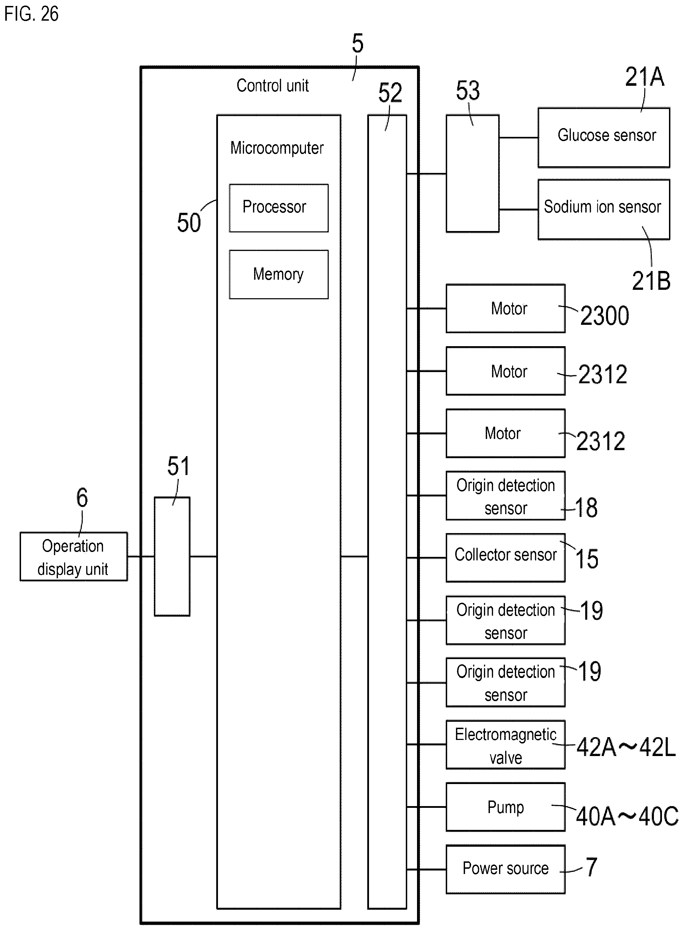

Next, as shown in FIG. 26, the control unit 5 includes a microcomputer 50 having a processor (for example, a CPU) and a memory (for example, a ROM and RAM), and circuit board for processing various signals such as a user interface control board 51, an I/O board 52, and analog board 53. The RAM is used as a program development area when the program stored in the ROM is executed. The control unit 5 causes the CPU to read out and execute the program stored in the ROM, and controls the operation of the motors 2300 and 2312 of the detection unit 2, the solenoid valves 42A to 42L and the pumps 40A to 40C of the fluid circuit unit 4, and the operation of the operation display unit 6 to bring the interstitial fluid collector 110 and the sweat collector 111 installed in the installation unit 20 into contact with the respective sensors 21A and 21B, perform measurement by the respective sensors 21A and 21B, as well as perform pretreatment such as cleaning the sensors 21A and 21B before measurement and creating a calibration curve using the sensors 21A and 21B. The control unit 5 calculates the blood glucose AUC based on the measurement value that reflects the concentration of glucose and the measurement value that reflects the concentration of sodium ion received from the sensors 21A and 21B of the detection unit 2, and generates and displays an analysis result on the operation display unit 6.

Next, the operation display unit 6 is provided to display an instruction to start measurement and display an analysis result and the like. The operation display unit 6 can be configured by a touch panel display. Note that the operation display unit 6 may be divided into an operation unit and a display unit, in which case the operation unit can be configured by buttons, switches, a keyboard, and a mouse.

Next, the power supply 7 converts an AC power supply voltage input from a power supply plug (not shown) into a DC voltage and supplies the DC voltage to the control unit 5. The power supply 7 also is connected to each of the other parts and supplies electric power to each part.

Next, a procedure for measuring the interstitial fluid collector 110 and the sweat collector 111 by using the in-vivo component measuring device 1 of the present embodiment will be described.

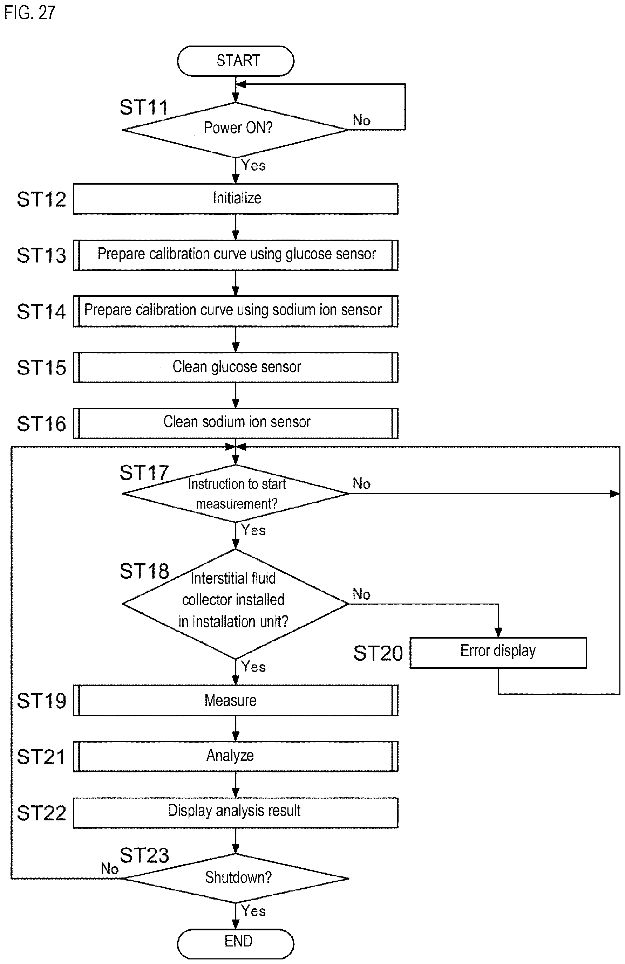

As shown in FIG. 27, when the power source of the device is first turned ON in ST11, the control unit 5 performs an initialization process in ST12. For example, when the installation unit 20 is positioned at the origin position shown in FIG. 21A, this position is set as the horizontal origin position based on the detection result of the origin detection sensor 18 of the installation unit 20, and when the sensors 21A and 21B are positioned at the origin position shown in FIG. 24A, this position is set as the vertical origin position.

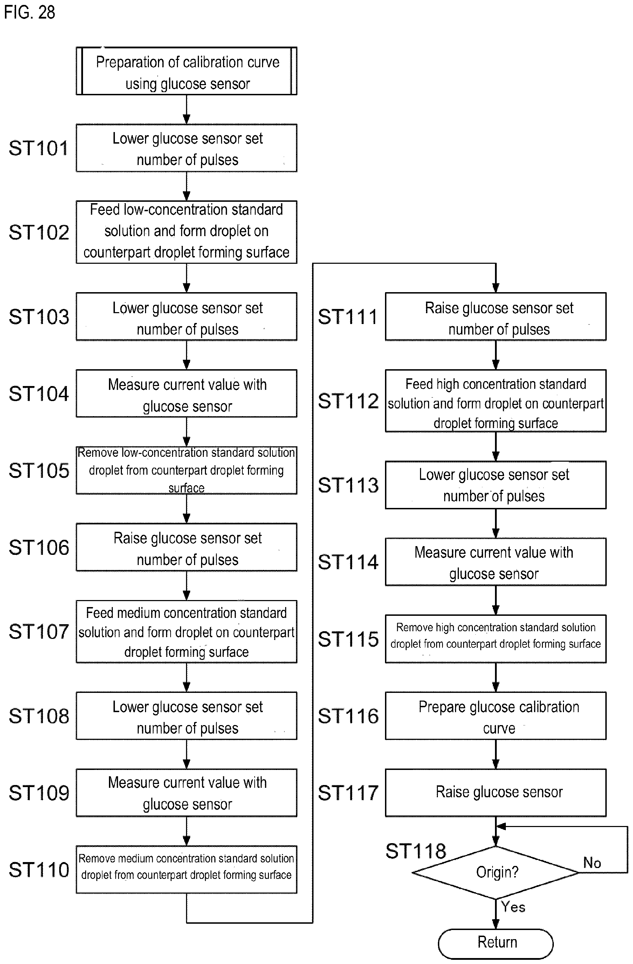

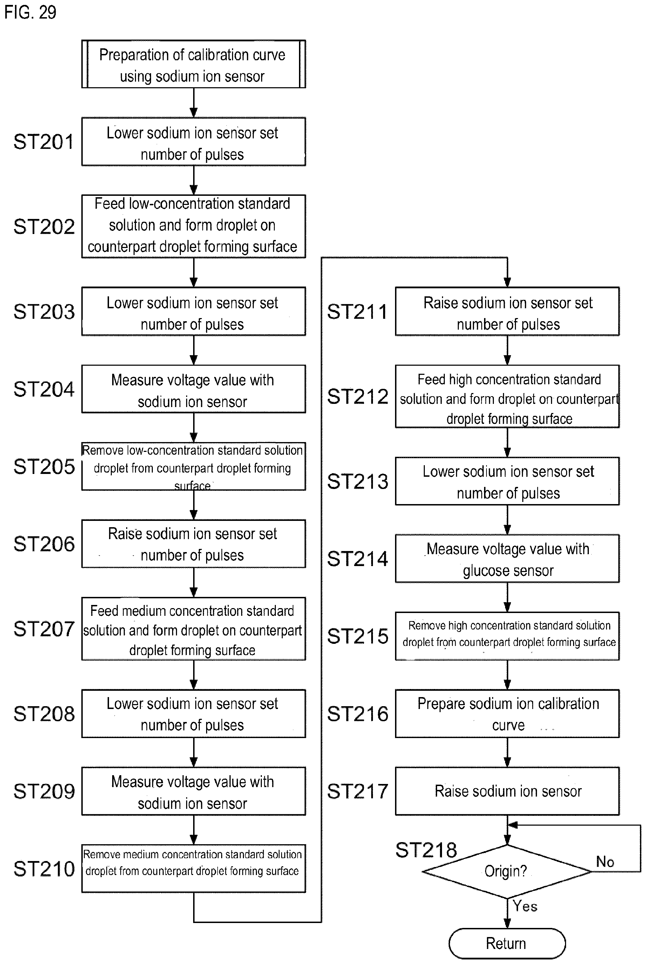

Next, as pretreatment, the control unit 5 creates a calibration curve using the glucose sensor 21A in ST13, and creates a calibration curve using the sodium ion sensor 21B in ST14. ST13 and ST14 may be performed at the same time or sequentially. FIG. 28 is a flowchart for creating a calibration curve using the glucose sensor 21A, and FIG. 29 is a flowchart for creating a calibration curve using the sodium ion sensor 21B. Note that the creation of a calibration curve means the creation of a relational expression between a component concentration and a measurement value obtained by the glucose sensor 21A and sodium ion sensor 21B based on the concentration of a component containing a material of known concentration and measured value of the component contained in a material of known concentration obtained by the glucose sensor 21A and the sodium ion sensor 21B.

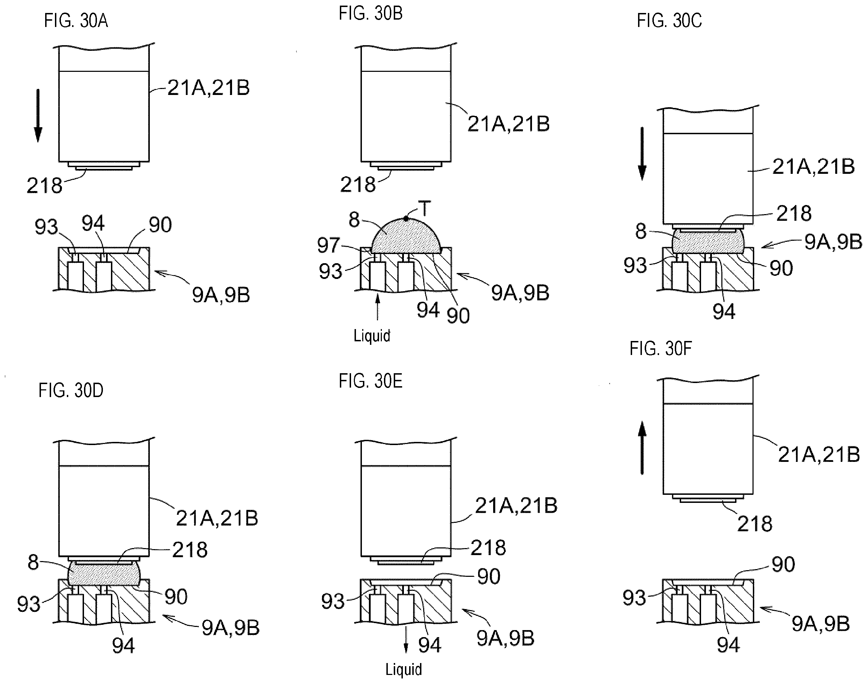

First, in ST101 and ST201, the control unit 5 controls the motor 2312 of the sensor moving unit 231 to lower the glucose sensor 21A and the sodium ion sensor 21B by a set number of pulses, respectively, such that the sensors 21A and 21B are moved from the origin position to the droplet formation standby position shown in FIG. 24C, as shown in FIG. 30A.

Next, in ST102 and ST202, the controller 5 forms a low-concentration standard droplet 8 on the droplet forming surface 90, as shown in FIG. 30B, by sending the low-concentration standard liquid in the second tank 32 to the droplet forming surface 90 of the counterpart objects 9A and 9B via the liquid supply holes 93 by driving the pumps 40A and 40B. In the present embodiment, the droplet 8 of the low-concentration standard liquid is formed so as to be convex toward the respective sensors 21A and 21B so that the top portion T is located on the center of the droplet forming surface 90. The droplet 8 of the low-concentration standard solution also is formed to a size reaching the outer peripheral edge of the droplet forming surface 90, and is formed at a height exceeding the wall surface 97 around the droplet forming surface 90. The droplet 8 of the low-concentration standard solution also may be formed by sending a fixed amount of the low-concentration standard solution onto the droplet forming surface 90 at once, or may be formed by sending it a plurality of times.

Next, in ST103 and ST203, the control unit 5 controls the motor 2312 of the sensor moving unit 231 to lower the glucose sensor 21A and the sodium ion sensor 21B by a set number of pulses, respectively, such that the sensors 21A and 21B are moved as shown in FIG. 30C from the droplet forming standby position to the droplet contact position shown in FIG. 24D. In this way the droplet 8 of the low-concentration standard solution is pressed against the surfaces of the glucose sensor 21A and the sodium ion sensor 21B, such that the shape of the droplet 8 changes to a flat shape and covers the surface of each sensor 21A and 21B as the droplet 8 of the low-concentration standard solution is pressed by the surfaces of the glucose sensor 21A and the sodium ion sensor 2. In this way the droplets 8 of the low-concentration standard solution come into wide contact with the surfaces of the glucose sensor 21A and the sodium ion sensor 21B. The glucose sensor 21A and the sodium ion sensor 21B may be moved from the droplet formation standby position to the droplet contact position at once, or the glucose sensor 21A and sodium ion sensor 21B may be moved to the droplet contact position at which the shape of the droplet 8 of the low-concentration standard solution changes to a flat shape after moving to the position at which the apex T of low-concentration solution droplet 8 is pressed against the surface of the glucose sensor 21A and sodium ion sensor 21B.

Next, in ST104 and ST204, the control unit 5 brings the droplets 8 of the low-concentration standard solution into contact with the surfaces of the glucose sensor 21A and the sodium ion sensor 21B for a fixed of time, and in this state, the glucose sensor 21A and the sodium ion sensor 21B measure the low-concentration standard solution, as shown in FIG. 30D. Specifically, the control unit 5 applies a constant voltage (for example, 0.45 V) using the electrode 218 of the glucose sensor 21A to acquire the current value $I_L$ of the low-concentration standard solution. This current value $I_L$ is designated the current value when the glucose concentration is low. The control unit 5 also uses the electrode 218 of the sodium ion sensor 21B to acquire the voltage value $V_L$ of the low-concentration standard solution. This voltage value $V_L$ is designated the voltage value when the sodium ion concentration is low. Obtained current value $I_L$ and voltage value $V_L$ are stored in the memory storage unit.

Next, in ST105 and ST205, the control unit 5 removes the droplet 8 of the low-concentration standard solution from the droplet forming surface 90 by driving the pump 40C to discharge the low-concentration standard liquid from the droplet forming surfaces 90 of the counterpart objects 9A and 9B through the liquid discharge holes 94, and sending the discharged liquid to the waste liquid tank 30, as shown in FIG. 30E.

Next, in ST106 and ST206, the control unit 5 controls the motor 2312 of the sensor moving unit 231 to raise the glucose sensor 21A and the sodium ion sensor 21B by a set number of pulses, respectively, to move the sensors 21A and 21B from the droplet contact position to the droplet formation standby position.

Next, in ST107 to ST111 and ST207 to ST211, the control unit 5 follows the same procedure as ST102 to ST106 and ST202 to ST206 described above, to send the droplet 8 of the medium concentration solution of the third tank 33 to form the droplet 8 of the medium concentration solution on the surface 90 of the counterpart objects 9A and 9B (FIG. 30B), move the glucose sensor 21A and the sodium ion sensor 21B from the droplet formation standby position to the droplet contact position, and press the droplet 8 of the medium-concentration standard solution against the surfaces of the glucose sensor 21A and the sodium ion sensor 21B (FIG. 30C). Then, with the droplets 8 of the medium-concentration standard solution kept in contact with the surfaces of the glucose sensor 21A and the sodium ion sensor 21B for a certain period of time (FIG. 30D), the medium concentration standard solution is measured by the glucose sensor 21A and the sodium ion sensor 21B to obtain the current value $I_M$, voltage value $V_M$. This current value $I_M$ and voltage value $V_M$ is the current value when the glucose concentration is medium, and the voltage value when the sodium ion concentration is medium. The obtained current value $I_M$ and voltage value $V_M$ are stored in the memory storage unit. Then, the droplet 8 of the medium-concentration standard solution is removed from the droplet forming surface 90 of the counterpart objects 9A and 9B (FIG. 30E), and the glucose sensor 21A and the sodium ion sensor 21B are respectively removed from the droplet contact position to the drop formation standby position (FIG. 30F).

Next, in ST112 to ST115 and ST212 to ST215, the control unit 5 follows the same procedure as ST102 to ST105 and ST202 to ST205 described above, to send the droplet 8 of the high concentration solution of the fourth tank 34 to form the droplet 8 of the high concentration solution on the surface 90 of the counterpart objects 9A and 9B (FIG. 30B), move the glucose sensor 21A and the sodium ion sensor 21B from the droplet formation standby position to the droplet contact position, and press the droplet 8 of the high-concentration standard solution against the surfaces of the glucose sensor 21A and the sodium ion sensor 21B (FIG. 30C). <t0/> Then, with the droplets 8 of the high-concentration standard solution kept in contact with the surfaces of the glucose sensor 21A and the sodium ion sensor 21B for a fixed period of time (FIG. 30D), the high concentration standard solution is measured by the glucose sensor 21A and the sodium ion sensor 21B to obtain the current value $I_H$ and voltage value $V_H$. This current value $I_H$ and voltage value $V_H$ are the current value when the glucose concentration is high and the voltage value when the sodium ion concentration is high, respectively. Obtained current value $I_H$ and voltage value $V_H$ are stored in the memory storage unit. Then, the droplet 8 of the high-concentration standard liquid is removed from the droplet forming surface 90 of the counterpart objects 9A and 9B (FIG. 30E).

Next, in ST116 and ST216, the control unit 5 prepares a glucose calibration curve based on the glucose concentrations of the low-concentration standard solution, the medium-concentration standard solution, and the high-concentration standard solution stored in the memory storage unit, and the acquired current value $I_L$, current value $I_M$ and current value $I_H$, and stores the data in the memory storage unit. The control unit 5 also prepares a sodium ion calibration curve based on the sodium ion concentrations of the low-concentration standard liquid, the medium-concentration standard liquid, and the high-concentration standard liquid stored in the memory storage unit, and the acquired voltage value $V_L$, voltage value $V_M$, and voltage value $V_H$, and stores the data in the memory storage unit.

Finally, in ST117 and ST217, the control unit 5 controls the motor 2312 of the sensor moving unit 231 to raise the glucose sensor 21A and the sodium ion sensor 21B to the origin (YES in ST118 and ST218) so as to move from the droplet contact position to the origin position. In this way the creation of the calibration curve using the glucose sensor 21A and the creation of the calibration curve using the sodium ion sensor 21B ends.

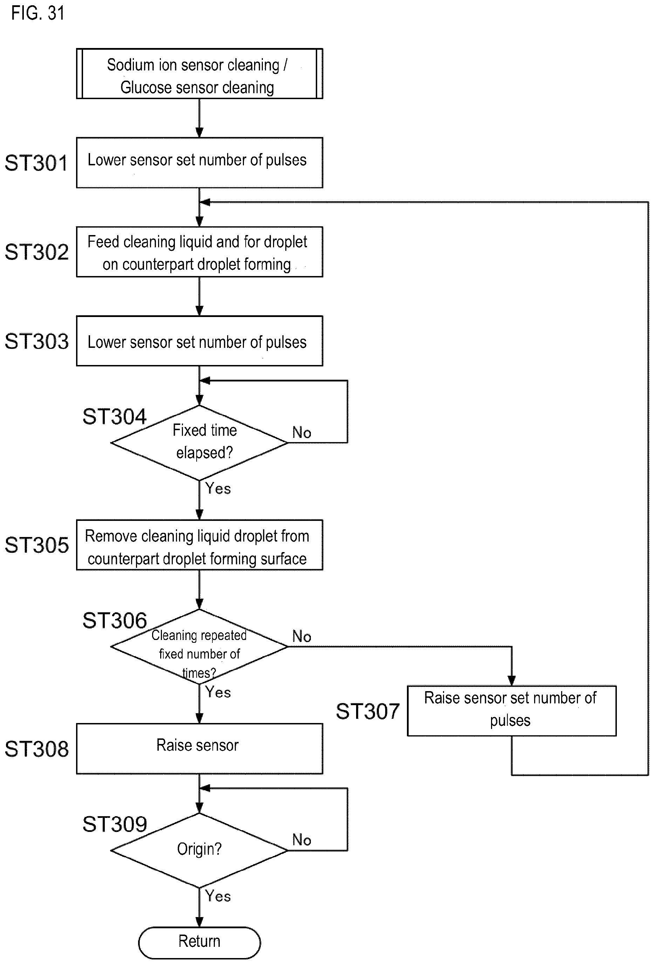

Returning to FIG. 27, when the preparation of the calibration curve using the glucose sensor 21A of ST13 and the preparation of the calibration curve using the sodium ion sensor 21B of ST14 are completed, the control unit 5 performs washing of the glucose sensor 21A in ST15 and washing of the sodium ion sensor 21B in ST16 as a pretreatment process. ST15 and ST16 may be performed at the same time or sequentially. FIG. 31 is a flowchart of cleaning the glucose sensor 21A and the sodium ion sensor 21B.

First, in ST301, the control unit 5 controls the motor 2312 of the sensor moving unit 231 to lower the glucose sensor 21A and the sodium ion sensor 21B by a set number of pulses, respectively, such that the sensors 21A and 21B are moved from the origin position to the droplet formation standby position, as shown in FIG. 30A.

Next, in S302, the control unit 5 forms the droplet 8 of the cleaning liquid on the droplet forming surface 90, as shown in FIG. 30B, by sending the cleaning liquid in the first tank 31 to the droplet forming surface 90 of the counter parts 9A and 9B via the liquid supply holes 93 by driving the pumps 40A and 40B. In the present embodiment, the droplet 8 of the cleaning liquid is formed so as to be convex toward the respective sensors 21A and 21B so that the top portion T is located on the center of the droplet forming surface 90. The cleaning droplet 8 also is formed to a size reaching the outer peripheral edge of the droplet forming surface 90, and is formed at a height exceeding the wall surface 97 around the droplet forming surface 90. The cleaning droplet 8 also may be formed by sending a fixed amount of liquid onto the droplet forming surface 90 at one time, or may be formed step-wise by sending the liquid multiple times.

Next, in ST303, the control unit 5 controls the motor 2312 of the sensor moving unit 231 to lower the glucose sensor 21A and the sodium ion sensor 21B by a set number of pulses, respectively, such that the sensors 21A and 21B are moved as shown in FIG. 30C from the droplet forming standby position to the droplet contact position. In this way the droplet 8 of the cleaning liquid is pressed against the surfaces of the glucose sensor 21A and the sodium ion sensor 21B, and is pressed by the surfaces of the glucose sensor 21A and the sodium ion sensor 21B such that the droplet shape changes into a flat shape and covers the surface of each sensor 21A and 21B. In this way the droplet 8 of the cleaning liquid come into wide contact with the surfaces of the glucose sensor 21A and the sodium ion sensor 21B. The glucose sensor 21A and the sodium ion sensor 21B also may be moved from the droplet formation standby position to the droplet contact position at once, or the glucose sensor 21A and sodium ion sensor 21B may be moved to the droplet contact position at which the shape of the droplet 8 of the cleaning liquid changes to a flat shape after moving to the position at which the apex T of cleaning droplet 8 is pressed against the surface of the glucose sensor 21A and sodium ion sensor 21B.

Next, in ST304, the control unit 5 causes the droplet 8 of the cleaning liquid to contact the surfaces of the glucose sensor 21A and the sodium ion sensor 21B for a fixed period of time, as shown in FIG. 30D. In this way the droplets 8 of the cleaning liquid can be sufficiently brought into contact with the surfaces of the glucose sensor 21A and the sodium ion sensor 21B so as to clean the electrode 218.

Next, in ST305, the controller 5 removes the cleaning droplet 8 from the droplet formation surface 90, as shown in FIG. 30E, by driving the pump 40C to discharge the cleaning liquid from the droplet forming surfaces 90 of the counterpart objects 9A and 9B through the liquid discharge holes 94 and send the cleaning liquid to the waste liquid tank 30.

The control unit 5 performs the cleaning of the glucose sensor 21A and the sodium ion sensor 21B a plurality of times using the cleaning liquid described above. The electrode 218 can be effectively cleaned by repeating the cleaning a plurality of times. ST306 is NO when the number of times of washing has not reached a predetermined number, and the control unit 5 controls the motor 2312 of the sensor moving unit 231 in step ST307 to raise the glucose sensor 21A and the sodium ion sensor 21B by a set number of pulses to move the sensors 21A and 21B from the droplet contact position to the droplet formation standby position shown in FIG. 30F. After that, the control unit 5 again cleans the glucose sensor 21A and the sodium ion sensor 21B in ST302 to ST305.

ST306 is YES when the number of times of cleaning reaches a fixed number, and in ST308, the control unit 5 controls the motor 2312 of the sensor moving unit 231 to raise the glucose sensor 21A and the sodium ion sensor 21B to the origin to move the sensors 21A and 21B from the droplet contact position to the origin position (YES in ST309). In this way the cleaning of the glucose sensor 21A and the sodium ion sensor 21B is completed.

Returning to FIG. 27, when the cleaning of the glucose sensor 21A in ST15 and the cleaning of the sodium ion sensor 21B in ST16 are completed, the control unit 5 waits in ST17 until there is an instruction to start measurement; when an instruction to start measurement is given by ST18 using the operation display unit 6, the control unit 5 confirms in ST18 whether each of the collectors 110 and 111 is installed in the installation unit 20. When the control unit 5 receives the electrical signal from the collector detection sensor 15, then in ST18 the control unit 5 determines that the collectors 110 and 111 are installed in the installation section 20, and the process proceeds to ST19 in which the glucose and sodium ions contained in the interstitial fluid collector 110 and the sweat collector 111 are measured. On the other hand, in ST18, when the electrical signal is not received from the collector detection sensor 15, the control unit 5 determines that the collectors 110 and 111 are not installed in the installation unit 20, and proceeds to ST20 in which an error message is displayed on the operation display unit 6.

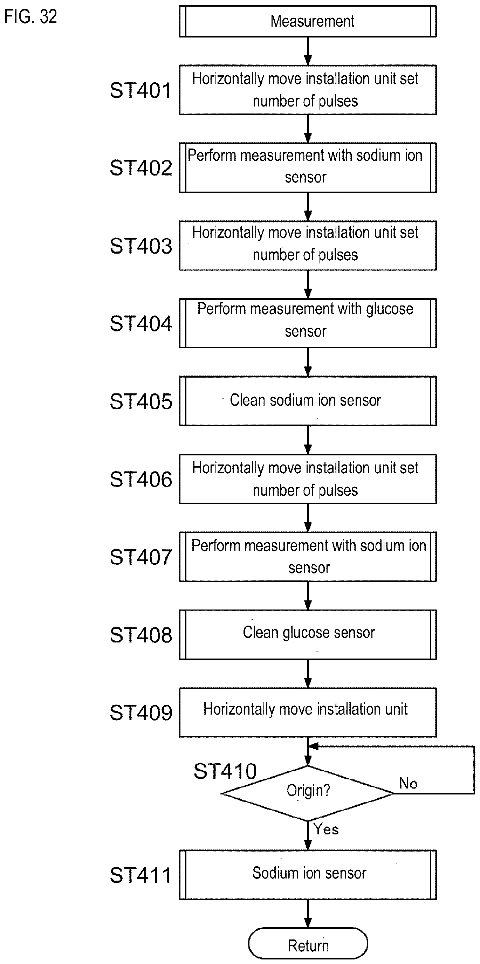

FIG. 32 is a flowchart showing details of ST19 of FIG. 27. First, in ST401, the control unit 5 controls the motor 2300 of the installation unit moving unit 230 to horizontally move the installation unit 20 by a set number of pulses so as to move the interstitial fluid collector 110 from the origin position (refer to FIG. 21A) to the first measurement position (shown in FIG. 21B) located below the sodium ion sensor 21B. Then, in ST402, the control unit 5 measures the interstitial fluid collector 110 with the sodium ion sensor 21B.

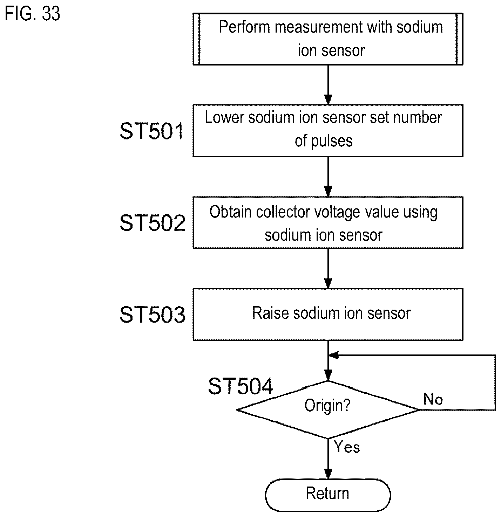

FIG. 33 is a flowchart of the measurement by the sodium ion sensor 21B. First, in ST501, the control unit 5 controls the motor 2312 of the sensor moving unit 231 to lower the sodium ion sensor 21B by a set number of pulses to move the sensor 21B from the origin position (shown in FIG. 24A) to a measurement position (shown in FIG. 24B) so as to bring the electrode 218 into contact with the interstitial fluid collector 110. Then, in ST502, the control unit 5 acquires a voltage $V_{Na1}$ of the interstitial fluid collector 110 via the electrode 218 of the sodium ion sensor 21B. This voltage value $V_{Na1}$ is dependent on the sodium ion concentration $C_{Na1}$ of the interstitial fluid collected in the interstitial fluid collector 110. The acquired voltage value $V_{Na1}$ is stored in the memory storage unit. Then, in ST503, the control unit 5 controls the motor 2312 of the sensor moving unit 231 to raise the sodium ion sensor 21B to the origin (YES in ST504) so as to move the sensor 21B from the measurement position to the origin position.

Returning to FIG. 32, when the measurement by the sodium ion sensor 21B in ST402 is completed, next, in ST403, the control unit 5 controls the motor 2300 of the installation unit moving unit 230 to horizontally move the installation unit 20 a fixed number of pulses, from the first measurement position to the second measurement position (shown in FIG. 21C) located below the glucose sensor 21A. Then, the control unit 5 measures the interstitial fluid collector 110 with the glucose sensor 21A in ST404, and cleans the sodium ion sensor 21B in ST405.

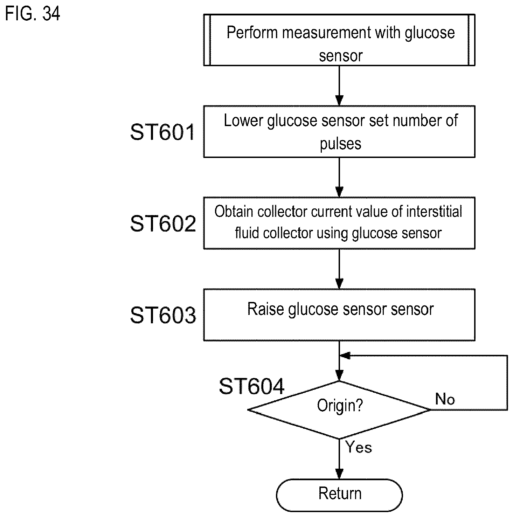

FIG. 34 is a flowchart of measurement by the glucose sensor 21A. First, in ST601, the control unit 5 controls the motor 2312 of the sensor moving unit 231 to lower the glucose sensor 21A by a set number of pulses to move the sensor 21A from the origin position to the measurement position, and bring the electrode 218 into contact with the interstitial fluid collector 110. Then, in ST602, the control unit 5 applies a constant voltage to the interstitial fluid collector 110 by the electrode 218 of the glucose sensor 21A to acquire a current value $I_{Glu}$. This current value $I_{Glu}$ is dependent on the glucose concentration $C_{Glu}$ in the interstitial fluid collected in the interstitial fluid collector 110. The acquired current value is stored in the memory storage unit. Then, in ST603, the control unit 5 controls the motor 2312 of the sensor moving unit 231 to raise the glucose sensor 21A to the origin (YES in ST604) so as to move the sensor 21A from the measurement position to the origin position.

The procedure for cleaning the sodium ion sensor 21B in ST405 is identical to the procedure for cleaning the sodium ion sensor 21B in ST16 of FIG. 27 (flow chart of FIG. 31).

Returning to FIG. 32, when the measurement by the glucose sensor 21A in ST404 and the cleaning of the sodium ion sensor 21B in ST405 are completed, the control unit 5 then controls the motor 2300 of the installation unit moving unit 230 in ST406 to horizontally move the installation unit 20 by the set number of pulses so as to move the second interstitial fluid collector 110 from the second measurement position to the third measurement position (FIG. 21D) below the sodium ion sensor 21B. Then, the control unit 5 measures the sweat collector 111 by the sodium ion sensor 21B in ST407, and cleans the glucose sensor 21A in ST408.

The flowchart of measurement by the sodium ion sensor 21B in ST407 is the same as the flowchart of FIG. 33. Specifically, in ST501, the control unit 5 controls the motor 2312 of the sensor moving unit 231 to lower the sodium ion sensor 21B by a set number of pulses so as to move it from the origin position to the measurement position and bring the electrode 218 into contact with the sweat collector 111. Then, in ST502, the control unit 5 acquires a voltage $V_{Na2}$ of the sweat collector 111 via the electrode 218 of the sodium ion sensor 21B. This voltage value $V_{Na2}$ is dependent on the sodium ion concentration $C_{Na2}$ in the sweat collected in the sweat collector 111. The acquired voltage value $V_{Na2}$ is stored in the memory storage unit. Then, in ST503, the control unit 5 controls the motor 2312 of the sensor moving unit 231 to raise the sodium ion sensor 21B to the origin (YES in ST504) so as to move the sensor 21B from the measurement position to the origin position.

The procedure for cleaning the glucose sensor 21A in ST408 is the same as the procedure for cleaning the glucose sensor 21A in ST15 of FIG. 27 (flowchart in FIG. 31) described above, and thus detailed description thereof is omitted here.

Returning to FIG. 32, when the measurement with the sodium ion sensor 21B in ST407 and the cleaning of the glucose sensor 21A in ST408 are completed, the control unit 5 then controls the motor 2300 of the installation unit moving unit 230 in ST409 to horizontally move the installation unit 20to the origin (YES in ST410) so as to move from the third measurement position to the origin position. Then, the control part 5 cleans the sodium ion sensor 21B in ST411. The procedure for cleaning the sodium ion sensor 21B in ST411 is identical to the procedure for cleaning the sodium ion sensor 21B in ST16 of FIG. 27 (flow chart of FIG. 31), and thus detailed description thereof is omitted here.

Returning to FIG. 27, when the measurement in ST19 is completed, the control unit 5 analyzes each component collected in the interstitial fluid collector 110 and the sweat collector 111 in ST21.

FIG. 32 is a flowchart showing details of ST19 of FIG. 27. First, in ST701, the control unit 5 analyzes the glucose concentration $C_{Glu}$ and sodium ion concentration $C_{Na1}$ contained in the interstitial fluid collected in the interstitial fluid collector 110, and sodium ion concentration $C_{Na2}$ in the sweat collected in the sweat collector 111. Specifically, the control unit 5 first reads a glucose calibration curve (a relational expression between a current value and a glucose concentration) from the memory storage unit, and applies the current value Iciu based on the detection signal output from the glucose sensor 21A to the calibration curve to calculate the glucose concentration $C_{Glu}$. A sodium ion calibration curve (a relational expression between the voltage value and the sodium ion concentration) is read out from the memory storage unit, and the voltage value $V_{Na1}$ and the voltage value $V_{Na2}$ based on the detection signal output from the sodium ion sensor 21B are applied to each calibration curve, and to calculate the sodium concentration $C_{Na1}$ and sodium ion concentration $C_{Na2}$.

Next, in ST702, control unit 5 determines whether the perspiration rate R is equal to or greater than a threshold value. The perspiration rate R is represented by $R=C_{Na1}/C_{Na2}$. When the control unit 5 determines that the perspiration rate R is lower than the threshold value, the blood glucose AUC is not calculated, and in ST703 the analysis result is displayed with a message containing "The analysis result of the blood glucose AUC cannot be displayed due to the high perspiration amount and the reliability of the analysis result cannot be guaranteed. Please remeasure." In this way it possible to avoid unreliable analysis of blood glucose AUC. Note that the threshold value can be obtained from the blood glucose AUC calculation value, which will be described later, blood glucose AUC from to blood collection, and experimental data on the amount of sweat.

On the other hand, when the control unit 5 determines in S702 that the perspiration rate R is equal to or more than the threshold value, the control unit 5 calculates the blood glucose AUC in S704. Specifically, the blood glucose AUC is calculated based on the glucose concentration $C_{Glu}$ obtained in ST701, the sodium concentration $C_{Na1}$, and the sodium concentration $C_{Na2}$ and the following equation (1). Note that in the formula (1) below, T is the time for collecting the interstitial fluid. Further, $\alpha$ is the ratio of the transmittance of glucose and sodium ions, which is a constant obtained by experiments. Also, $C'_{Na}$ is the sodium ion concentration in blood and is a constant obtained by actual measurement.

$$AUC=C'_{Na}\times T\times\{C_{Glu}/A(C_{Na1}-C_{Na2})\}\ ...(1)$$

Then the control unit 5 generates the analysis result containing the calculated blood glucose AUC in ST705. The analysis result can include glucose concentration, sodium concentration, perspiration rate, and the like.

Returning to FIG. 27, the control unit 5 then displays the analysis result generated in ST21 on the operation display unit 6 in ST22.. When the measurement of one interstitial fluid collector 110 is completed, the control unit 5 waits until the device is shut down (YES in ST23) or an instruction to start measurement is issued in ST17.

According to the in-vivo component measuring device 1 of the present embodiment described above, a droplet 8 of a liquid solution such as a standard solution or a cleaning solution used for pretreatment is formed on the droplet forming surface 90 of the counterpart objects 9A and 9B facing the sensors 21A and 21B, and the droplet 8 is brought into contact with the surface of each sensor 21A and 21B by pressing the droplet 8 against the surface of the sensor 21A and 21B. [fuzzy]In this way, even if bubbles <t0/>are mixed in when the droplet 8 is formed, the bubbles float on the surface of the droplet during the formation of the droplet 8, and the bubbles escape or break from the surface of the droplet 8 when the droplet 8 is pressed against the surface of the electrode type sensor 21A and 21B. Hence, since the presence of bubbles between the surface of the sensor 21A and 21B and the electrode 218 can be suppressed when the droplet 8 used for the pretreatment is brought into contact with the surface of the sensor 21A and 21B, the possibility of causing various problems in pretreatment is reduced, and pretreatment performance such as cleaning efficiency and calibration accuracy can be improved. Since the amount of bubbles mixed in the droplet 8 is small, it also is possible to prevent the amount of the liquid used for the pretreatment from increasing, so that the pretreatment can be performed with a lesser amount of the liquid.

In addition, according to the in-vivo component measuring device 1 of the present embodiment, since the fresh droplets 8 are usually in contact with the surfaces of the sensors 21A and 21B, the pretreatment performance can be improved.

In addition, according to the in-vivo component measuring device 1 of the present embodiment, the droplets 8 to be used for the pretreatment are formed on the droplet forming surfaces 90 of the counterpart objects 9A and 9B, and pressed against the surfaces of the sensors 21A and 21B with a simple configuration to accomplish pretreatment. Hence, the in-vivo component measuring device 1 can be made compact.

In addition, according to the in-vivo component measuring device 1 of the present embodiment, the surface of each sensor 21A, 21B hits the droplet 8 and the shape of the droplet 8 changes to a flat shape, whereby each sensor 21A and 21B are coated with the droplets 8 of liquid. Hence, the liquid used for the pretreatment can be brought into contact with the surfaces of the respective sensors 21A and 21B over a wide range.

In addition, according to the in-vivo component measuring apparatus 1 of the present embodiment, the droplet 8 is applied to each sensor after the portion T closest to the surface of each sensor 21A, 21B is pressed against the surface of each sensor 21A and 21B, the droplet 8 is spread in a plane over the surfaces of the sensors 21A and 21B so as to come into contact with the surfaces of the sensors 21A and 21B by being pressed against the surface of each sensor 21A and 21B. Hence, it is possible to favorably prevent bubbles from remaining between the surface of each of the sensors 21A and 21B and the droplet 8.

In addition, according to the in-vivo component analyzer 1 of the present embodiment, the droplet 8 is formed in such a size that the surface of the sensor 21A and 21B fits inside the outer peripheral edge thereof in plan view. Therefore, when the surface of each sensor 21A, 21B hits the droplet 8 and the shape of the droplet 8 is deformed into a flat shape, the surface of each sensor 21A, 21B is suitably covered with the droplet 8. Since the droplet 8 is formed at a height exceeding the wall surface 97 of the counterpart objects 9A and 9B, the surfaces of the sensors 21A and 21B also can be pressed against the droplet 8 satisfactorily.

Modification of In-vivo Component Measuring Device

Although one embodiment of the in-vivo component measuring device has been described above, the in-vivo component measuring device of the present invention is not limited to the above-described embodiment, inasmuch as various modifications can be made without departing from the spirit of the present invention. For example, the following changes are possible.

In pretreatment such as cleaning of each sensor 21A and 21B and preparation of a calibration curve using each sensor 21A and 21B, the sensor moving unit 231 moves each sensor 21A and 21B in a direction facing the corresponding counterpart object 9A and 9B, such that the droplet 8 is pressed against the surface of each sensor 21A and 21B. However, the present invention is not limited to this configuration inasmuch as the in-vivo component measuring device 1 also may include a counterpart object moving unit that moves the counterpart objects 9A and 9B in a direction facing the corresponding sensors 21A and 21B by the control unit 5 controlling the counterpart object moving unit, whereby the counterpart objects 9A and 9B move in a direction facing the corresponding sensors 21A and 21B and the droplets 8 are pressed against the surfaces of the sensors 21A and 21B. Alternatively, the in-vivo component measuring device 1 may include the sensor moving unit 231 and the counterpart object moving unit, and the control unit 5 controls the sensor moving unit 231 and the counterpart object moving unit so that both the sensors 21A and 21B and the counterpart objects 9A and 9B move in the facing directions and the droplet 8 is pressed against the surface of each sensor 21A and 21B.

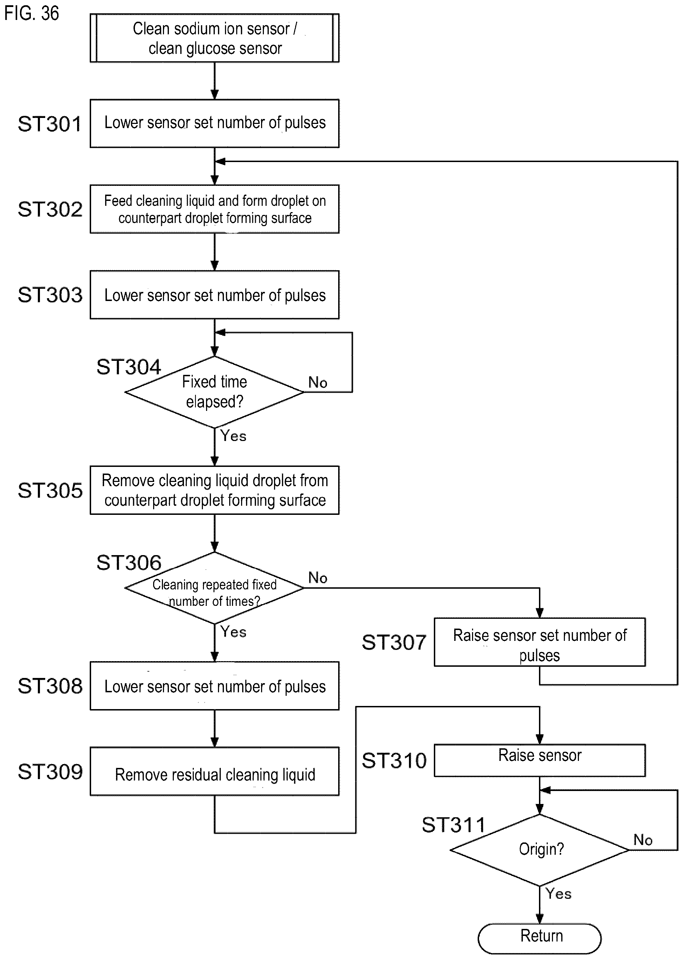

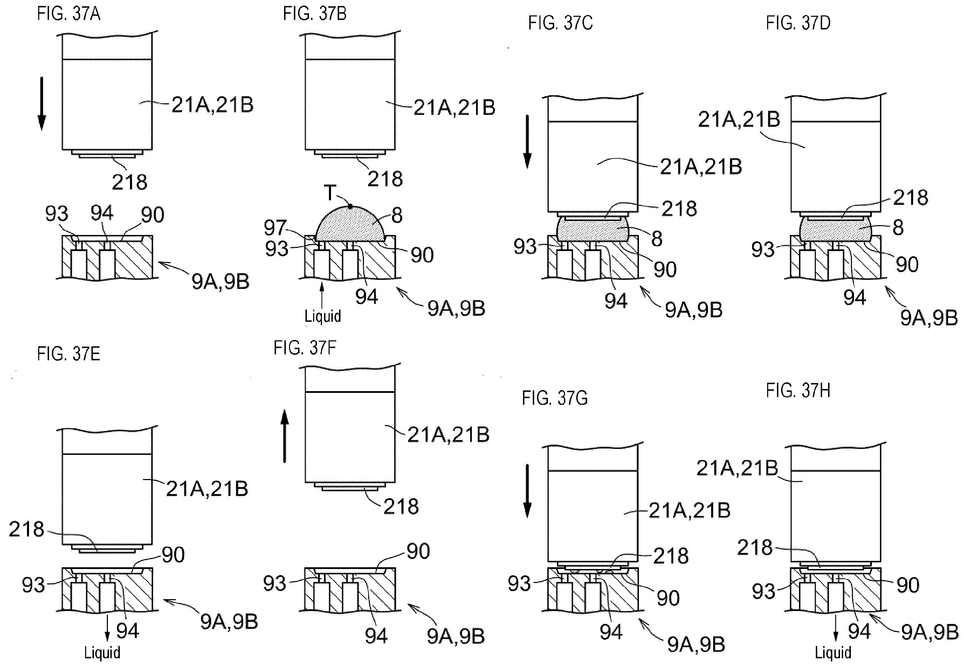

In pretreatment such as cleaning of each sensor 21A and 21B and preparation of a calibration curve using each sensor 21A and 21B, a step of removing the remaining liquid also may be performed when there is concern of residual liquid remaining on the surface of the sensors 21A and 21B and the droplet forming surface 90 after removing the droplet 8 from the droplet forming surface 90 and each sensor 21A and 21B. FIG. 36 and FIG. 37 show a processing procedure for removing the remaining cleaning liquid in cleaning the sensors 21A and 21B.

In ST301 to 305 of FIG. 36, the control unit 5 moves the glucose sensor 21A and the sodium ion sensor 21B from the origin position to the droplet formation standby position in the same procedure as ST301 to 305 of FIG. 31 described above (see FIG. 37A), forms the droplet 8 of the cleaning liquid on the droplet forming surface 90 of the counterpart objects 9A and 9B (FIG. 37B), moves the glucose sensor 21A and the sodium ion sensor 21B from the droplet formation standby position to the droplet contact position, and presses the droplet 8 of the cleaning liquid against the surfaces of the glucose sensor 21A and the sodium ion sensor 21B (FIG. 37C). Then, the droplets 8 of the cleaning liquid are brought into contact with the surfaces of the glucose sensor 21A and the sodium ion sensor 21B for a fixed period of time to clean the electrode 218 (FIG. 37D). Then, the cleaning droplets 8 are removed from the droplet forming surfaces 90 of the counterpart objects 9A and 9B (FIG. 37E).

The control unit 5 monitors whether the cleaning of the glucose sensor 21A and the sodium ion sensor 21B using the cleaning liquid described above has been performed a plurality of times. When the number of repetitions of cleaning has not reached a fixed number (NO in ST306 of FIG. 36), the control unit 5 controls the motor 2312 of the sensor moving unit 231 in ST307 of FIG. 36 to move the glucose sensor 21A and the sodium ion sensor 21B from the droplet contact position to the droplet formation standby position (FIG. 37F), and the glucose sensor 21A and the sodium ion sensor 21B are washed again in ST302 to ST305 of FIG. 36.

When the number of repetitions of cleaning reaches a fixed number (YES in ST306 of FIG. 36), the control unit 5 controls the motor 2312 of the sensor moving unit 231 in ST308 of FIG. 36 to move the glucose sensor 21A and the sodium ion sensor 21B from the droplet contact position to the residual liquid removal position shown in FIG. 24E to bring the surfaces of the glucose sensor 21A and the sodium ion sensor 21B and the droplet formation surface 90 close to each other.

Next, in ST309 of FIG. 36, the control unit 5 drives the pump 40C to discharge the cleaning liquid adhering to the droplet forming surface 90 of the counterpart objects 9A and 9B and the surfaces of the glucose sensor 21A and the sodium ion sensor 21B into liquid discharge hole 94 and sending the discharged liquid to the waste liquid tank 30, thereby removing the remaining cleaning liquid as shown in FIG. 37H.

When the residual cleaning liquid is removed, the control unit 5 raises the glucose sensor 21A and the sodium ion sensor 21B to the origin in ST310 to ST311 of FIG. 36 in the same procedure as ST308 to ST309 of FIG. 31, to move from the residual liquid removal position to the origin position. In this way the cleaning of the glucose sensor 21A and the sodium ion sensor 21B is completed.

According to the embodiment of FIG. 36 and FIG. 37, in the measurement after the pretreatment, it is possible to suppress the liquid used for the pretreatment from being mixed in the measurement sample since it is possible to reliably remove the liquid after using it for pretreatment.

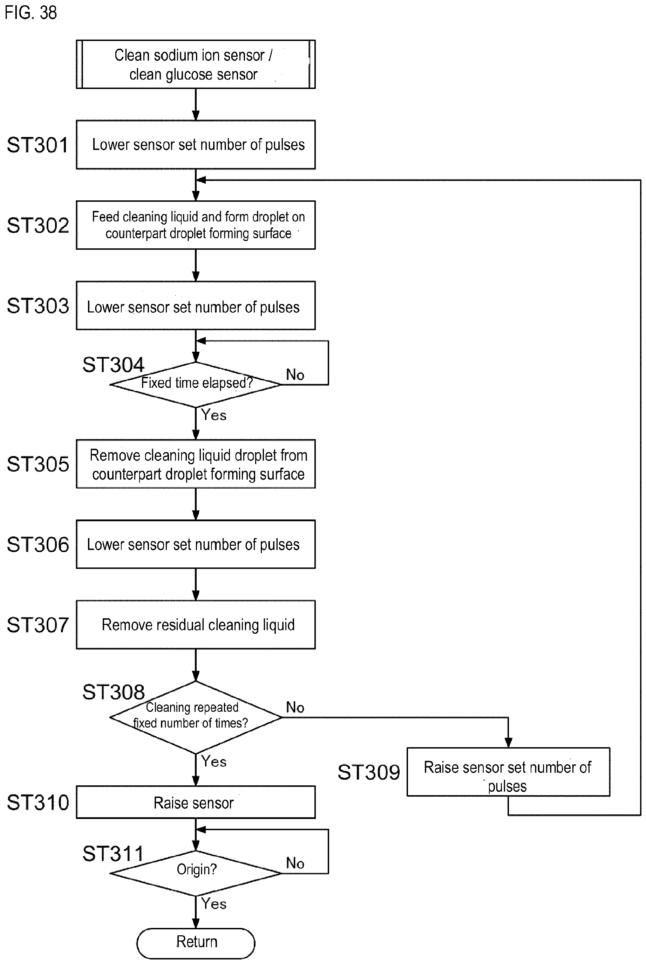

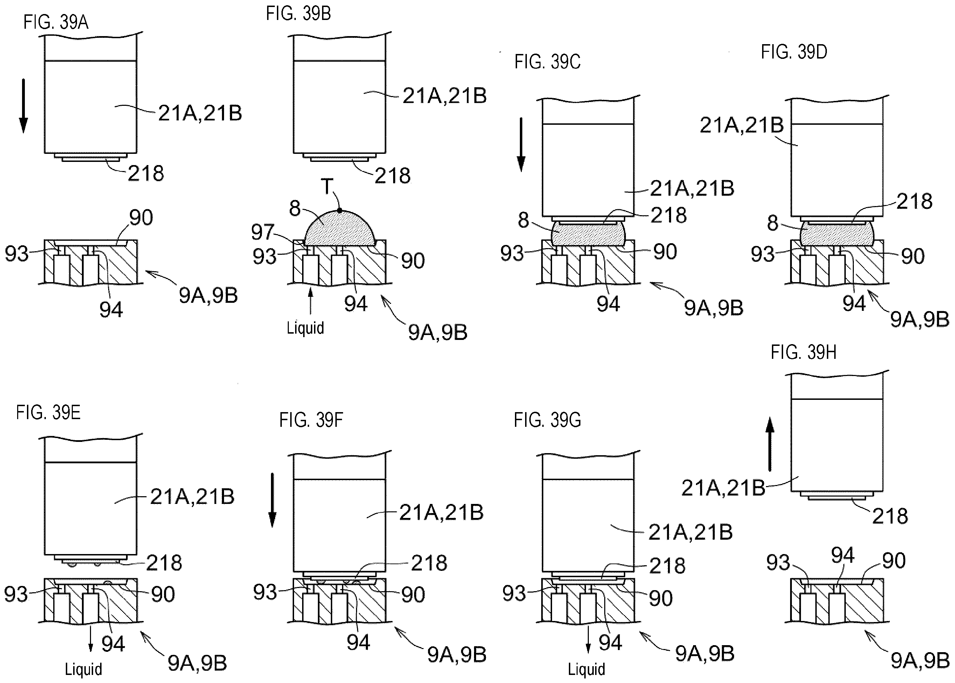

The residual liquid removing step may be performed after cleaning the glucose sensor 21A and the sodium ion sensor 21B a plurality of times as shown in FIGS. 36 and 37, or the cleaning may be performed each time after cleaning the glucose sensor 21A and the sodium ion sensor 21B, as shown in FIGS. 38 and 39.

Specifically, in ST301 to ST305 of FIG. 38, the control unit 5 moves the glucose sensor 21A and the sodium ion sensor 21B from the origin position to the droplet formation standby position in the same procedure as ST301 to ST305 of FIG. 31 described above (see FIG. 39A), forms the droplet 8 of the cleaning liquid on the droplet forming surface 90 of the counterpart objects 9A and 9B (FIG. 39B), moves the glucose sensor 21A and the sodium ion sensor 21B from the droplet formation standby position to the droplet contact position, and presses the droplet 8 of the cleaning liquid against the surfaces of the glucose sensor 21A and the sodium ion sensor 21B (FIG. 39C). Then, the droplets 8 of the cleaning liquid are brought into contact with the surfaces of the glucose sensor 21A and the sodium ion sensor 21B for a fixed period of time to clean the electrode 218 (FIG. 39D). Then, the cleaning droplets 8 are removed from the droplet forming surfaces 90 of the counterpart objects 9A and 9B (FIG. 39E).

Next, in ST306 of FIG. 38, the control unit 5 controls the motor 2312 of the sensor moving unit 231 to move the glucose sensor 21A and the sodium ion sensor 21B from the droplet contact position to the residual liquid removal position as shown in FIG. 39F and bring the surfaces of the glucose sensor 21A and the sodium ion sensor 21B and the droplet forming surface 90 close to each other.

Next, in ST307 of FIG. 38, the control unit 5 drives the pump 40C to discharge the cleaning liquid adhering to the droplet forming surface 90 of the counterpart objects 9A and 9B and the surfaces of the glucose sensor 21A and the sodium ion sensor 21B from liquid discharge hole 94 to the discharged liquid to the waste liquid tank 30, thereby removing the remaining cleaning liquid as shown in FIG. 39G.

The control unit 5 monitors whether the cleaning of the glucose sensor 21A and the sodium ion sensor 21B using the cleaning liquid described above has been performed a plurality of times. When the number of repeats of washing has not reached a fixed number (NO in ST308 of FIG. 38), the control unit 5 controls the motor 2312 of the sensor moving unit 231 in ST309 of FIG. 38 to move the glucose sensor 21A and the sodium ion sensor 21B from the residual liquid removal position to the droplet formation standby position (FIG. 39H), and cleans and removes the residual liquid of the glucose sensor 21A and the sodium ion sensor 21B again in ST302 to ST307 of FIG.38.

When the number of cleanings reaches a fixed number (YES in ST308 of FIG. 38), the control unit 5 performs the same procedure as ST308 to ST309 of FIG. 31 in ST310 to ST311 of FIG. 38 to raise the glucose sensor 21A and the sodium ion sensor 21B to the origin to move from the residual liquid removal position to the origin position. In this way the cleaning of the glucose sensor 21A and the sodium ion sensor 21B is completed.

According to the embodiment of FIG. 36 and FIG. 37, in the measurement after the pretreatment, it is possible to suppress the liquid used for the pretreatment from being mixed in the measurement sample since it is possible to reliably remove the liquid after using it for pretreatment.

Note that although the procedure shown in FIGS. 36 to 39 has been described in terms of removing the cleaning liquid remaining when the pretreatment is cleaning of the sensors 21A and 21B, the procedure similarly applies to pretreatment involving removal of residual standard solution when preparing a calibration curve using the sensors 21A and 21B, the description of which is omitted. The removal of the remaining standard solution also may be performed each time the standard solution of each concentration is measured by the glucose sensor 21A and the sodium ion sensor 21B, and the standard solution of all the concentrations is measured by the glucose sensor 21A and the sodium ion sensor 21B.

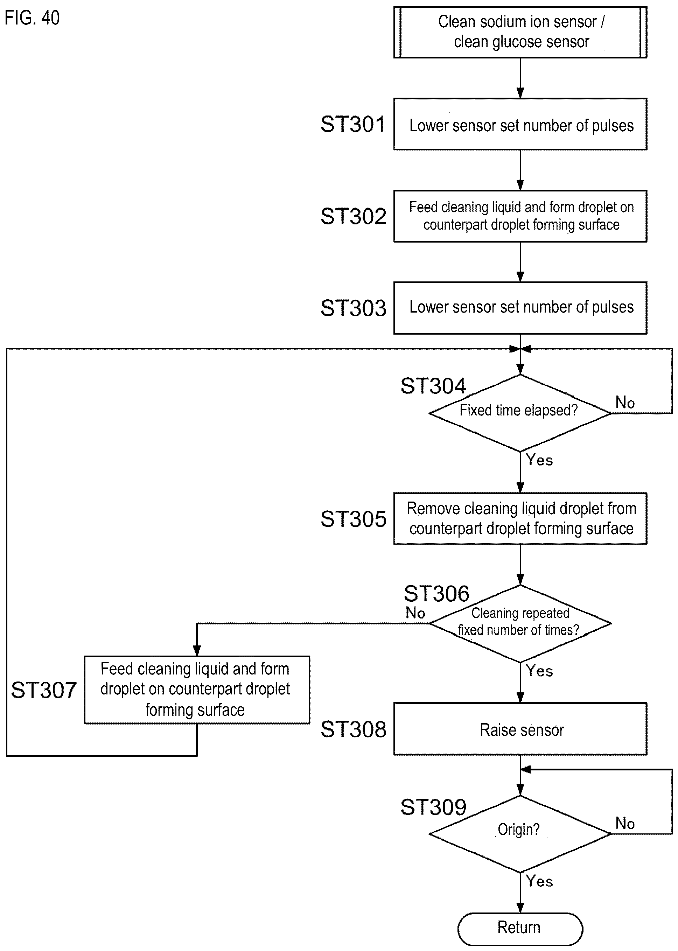

The examples of the above embodiments describe moving each sensor 21A and 21B and/or moving the droplet 8 in conjunction with moving the sensors 21A and 21B with regard to the relative movement of the droplet 8 and the sensors 21A and 21B when the droplet 8 is pressed against the sensors 21A and 21B in pretreatment such as cleaning of each sensor 21A, 21B and preparation of a calibration curve using each sensor 21A and 21B. However, the movement of the droplet 8 is not limited to movement in conjunction with movement of the counterpart objects 9A and 9B, and includes adding the amount of liquid of the droplet 8 formed on the droplet forming surface 90 of the counterpart objects 9A and 9B, and displacement of the droplet surface in the direction facing the respective sensors 21A, 21B by the droplets 8 swelling gradually toward the respective sensors 21A, 21B. FIGS. 40 to 41 show, in cleaning the sensors 21A and 21B, a processing procedure for pressing the droplet 8 against the surfaces of the sensors 21A and 21B by increasing the liquid amount of the droplets 8 on the droplet forming surfaces 90 of the counterpart objects 9A and 9B.

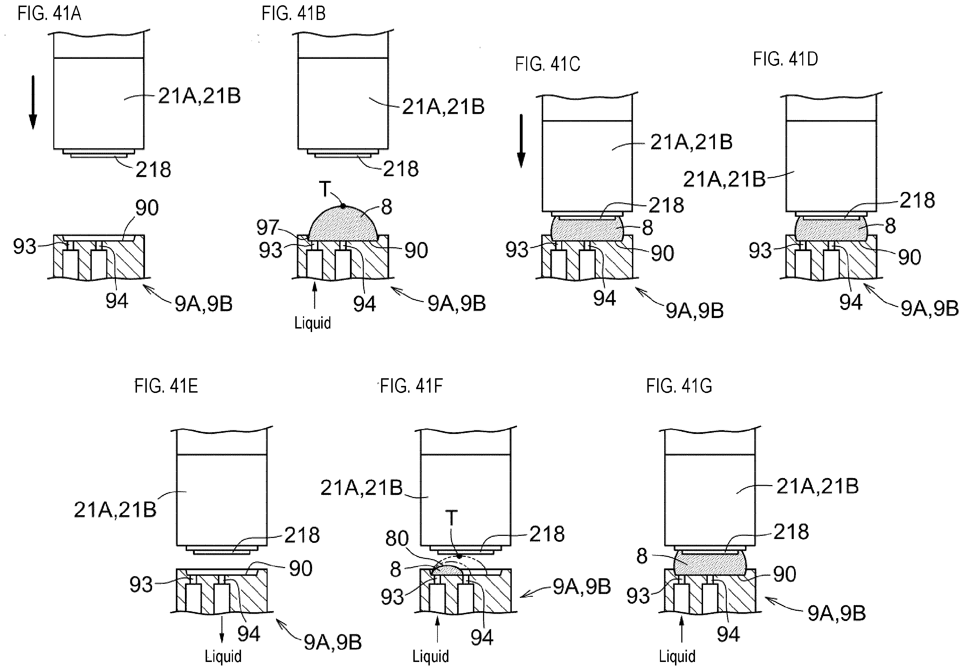

In ST301 to ST305 of FIG. 36, the control unit 5 moves the glucose sensor 21A and the sodium ion sensor 21B from the origin position to the droplet formation standby position (see FIG. 37A) in the same procedure as ST301 to 305 of FIG. 31 described above, forms the droplet 8 of the cleaning liquid on the droplet forming surface 90 of the counterpart objects 9A and 9B (FIG. 37B), moves the glucose sensor 21A and the sodium ion sensor 21B from the droplet formation standby position to the droplet contact position, and presses the droplet 8 of the cleaning liquid against the surfaces of the glucose sensor 21A and the sodium ion sensor 21B (FIG. 37C). Then, the droplets 8 of the cleaning liquid are brought into contact with the surfaces of the glucose sensor 21A and the sodium ion sensor 21B for a fixed period of time to clean the electrode 218 (FIG. 41D). Then, the cleaning droplets 8 are removed from the droplet forming surfaces 90 of the counterpart objects 9A and 9B (FIG. 41E).

The control unit 5 monitors whether the cleaning of the glucose sensor 21A and the sodium ion sensor 21B using the cleaning liquid described above has been performed a plurality of times. When the number of times of cleaning has not reached a fixed number (NO in ST306 of FIG. 40), the controller 5 drives the pump 40A, 40B to feed the cleaning liquid through the liquid supply hole 93 to the droplet forming surface 90 of the counterpart objects 9A and 9B to form the droplet 8 of the cleaning liquid on the droplet forming surface 90 as shown in FIG. 41F. The droplet 8 of the cleaning liquid gradually swells and grows larger as the amount of the liquid increases. In this way the droplet 8 of the cleaning liquid is displaced in the direction in which the droplet surface 80 faces the glucose sensor 21A and the sodium ion sensor 21B (upward in this embodiment). When the amount of the cleaning droplet 8 reaches a fixed amount, the portion of the cleaning droplet 8 that is closest to the surface of the electrode-type sensor 21 on the surfaces of the glucose sensor 21A and the sodium ion sensor 21B, the top portion T in the present embodiment hits the surface and the amount of liquid further increases to a certain amount, as shown in FIG. 41G, such that shape of the droplet 8 changes and is flattened by pressing against surface of the glucose sensor 21A and the sodium ion sensor 21B, and the droplet 8 of cleaning liquid covers the surface of each sensor 21A and 21B. In this way the droplet 8 of the cleaning liquid comes into contact with the surfaces of the glucose sensor 21A and the sodium ion sensor 21B over a wide range. The droplet 8 also may be formed by sending a predetermined amount of liquid onto the droplet forming surface 90 at once, or may be formed stepwise by sending it a plurality of times.

Then, in ST304 to ST305 of FIG. 40, the control unit 5 again brings the droplet 8 into contact with the surface of the glucose sensor 21A and the sodium ion sensor 21B for a fixed time to clean the electrode 218 (FIG. 41D), and thereafter removes the cleaning droplet 8 from the droplet forming surface 90 of the counterpart objects 9A and 9B (FIG. 41E).

When the number of cleanings reaches a fixed number (YES in ST306 of FIG. 40), control unit 5 performs the same procedure as ST308 to ST309 of FIG. 31 in ST308 to ST309 of FIG. 31, to raise the glucose sensor 21A and the sodium ion sensor 21B to the origin such that the sensors 21A and 21B move from the liquid contact position to the origin position. In this way the cleaning of the glucose sensor 21A and the sodium ion sensor 21B is completed.

In the embodiment of FIGS. 40 and 41, a step of removing residual liquid may be performed when there is concern liquid may be left on the surface of each of the sensors 21A and 21B or the liquid drop forming surface 90 after the droplet 8 has been removed from the droplet forming surface 90.

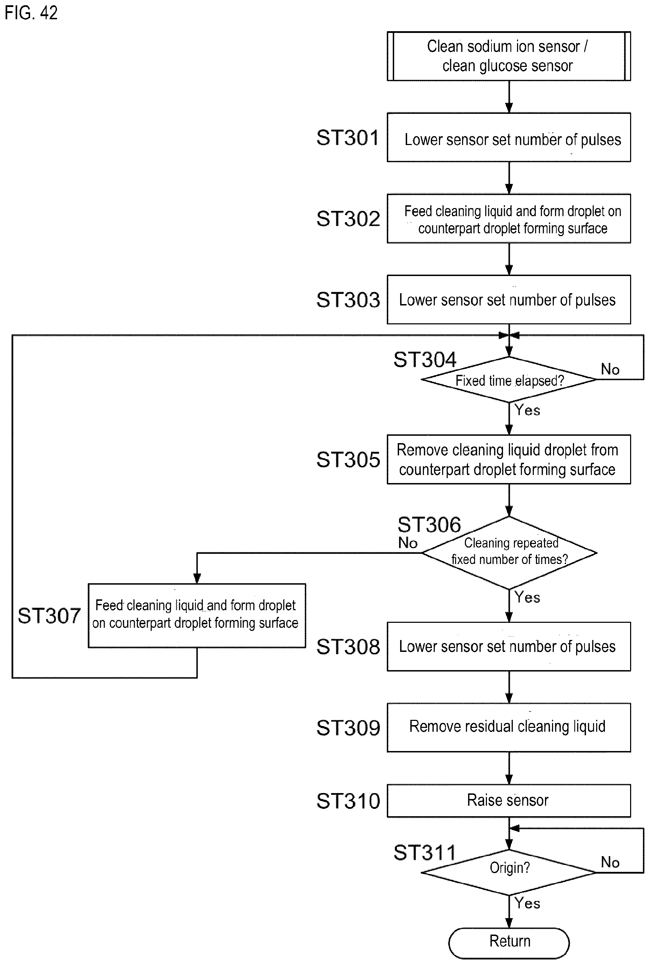

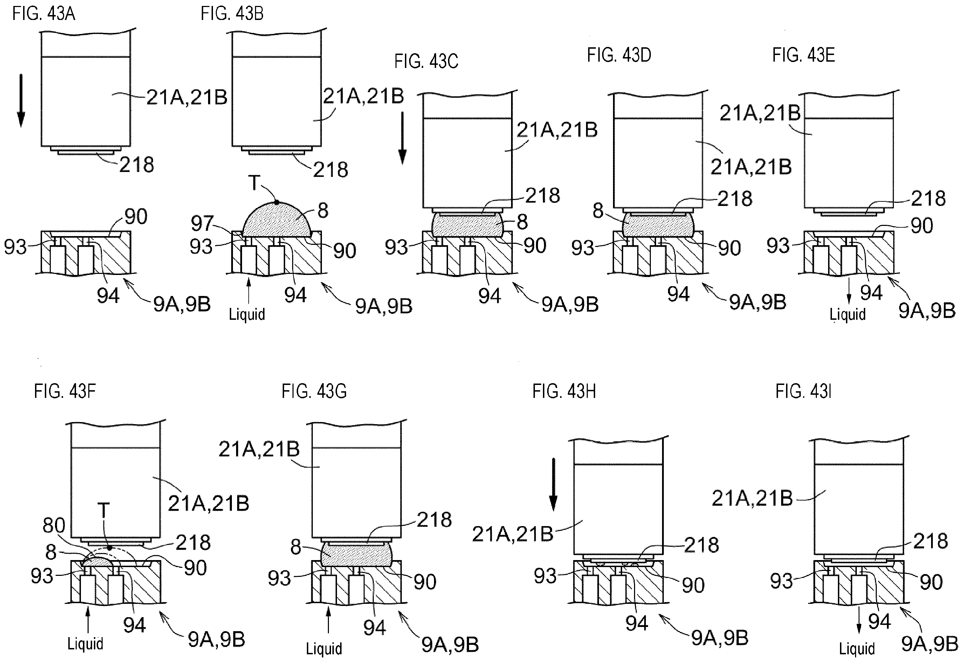

FIG. 42 and 43 show a treatment procedure when a step of removing the residual liquid is performed after the glucose sensor 21A and the sodium ion sensor 21B have been cleaned a plurality of times.

Specifically, in ST301 to ST305 of FIG. 42, the control unit 5 moves the glucose sensor 21A and the sodium ion sensor 21B from the origin position to the droplet formation standby position in the same procedure as ST301 to ST305 of FIG. 31 described above (see FIG. 43A), forms the droplet 8 of the cleaning liquid on the droplet forming surface 90 of the counterpart objects 9A and 9B (FIG. 43B), moves the glucose sensor 21A and the sodium ion sensor 21B from the droplet formation standby position to the droplet contact position, and presses the droplet 8 of the cleaning liquid against the surfaces of the glucose sensor 21A and the sodium ion sensor 21B (FIG. 43C). Then, the droplets 8 of the cleaning liquid are brought into contact with the surfaces of the glucose sensor 21A and the sodium ion sensor 21B for a fixed period of time to clean the electrode 218 (FIG. 43D). Then, the cleaning droplets 8 are removed from the droplet forming surfaces 90 of the counterpart objects 9A and 9B (FIG. 43E).

The control unit 5 monitors whether the cleaning of the glucose sensor 21A and the sodium ion sensor 21B using the cleaning liquid described above has been performed a plurality of times. When the number of times of cleaning has not reached a fixed number (NO in ST306 of FIG. 42), the controller 5 drives the pumps 40A and 40B to feed the cleaning liquid through the liquid supply hole 93 to the droplet forming surface 90 of the counterpart objects 9A and 9B to form the droplet 8 of the cleaning liquid on the droplet forming surface 90 as shown in FIG. 43F. The droplet 8 of the cleaning liquid gradually swells and grows larger as the amount of the liquid increases. In this way the droplet 8 of the cleaning liquid is displaced in the direction in which the droplet surface 80 faces the glucose sensor 21A and the sodium ion sensor 21B (upward in this embodiment). When the amount of the cleaning droplet 8 reaches a fixed amount, the portion of the cleaning droplet 8 that is closest to the surface of the electrode-type sensor 21 on the surfaces of the glucose sensor 21A and the sodium ion sensor 21B, the top portion T in the present embodiment, hits the surface and the amount of liquid further increases to a certain amount, such that the shape of the droplet 8 changes and is flattened by pressing against surface of the glucose sensor 21A and the sodium ion sensor 21B, and the droplet 8 of cleaning liquid covers the surface of each sensor 21A and 21B, as shown in FIG. 43G. In this way the droplet 8 of the cleaning liquid comes into contact with the surfaces of the glucose sensor 21A and the sodium ion sensor 21B over a wide range.

Then, in ST304 to ST305 of FIG. 42, the control unit 5 again brings the droplet 8 into contact with the surface of the glucose sensor 21A and the sodium ion sensor 21B for a fixed time to clean the electrode 218 (FIG. 43D), and thereafter removes the cleaning droplet 8 from the droplet forming surface 90 of the counterpart objects 9A and 9B (FIG. 43E).

When the number of repetitions of cleaning reaches a fixed number (YES in ST306 of FIG. 42), the control unit 5 controls the motor 2312 of the sensor moving unit 231 in ST308 of FIG. 42 to move the glucose sensor 21A and the sodium ion sensor 21B from the droplet contact position to the residual liquid removal position shown in FIG. 43H to bring the surfaces of the glucose sensor 21A and the sodium ion sensor 21B and the droplet formation surface 90 close to each other.

Next, in ST309 of FIG. 42, the control unit 5 drives the pump 40C to discharge the cleaning liquid adhering to the droplet forming surface 90 of the counterpart objects 9A and 9B and the surfaces of the glucose sensor 21A and the sodium ion sensor 21B into liquid discharge hole 94 and send the discharged liquid to the waste liquid tank 30, thereby removing the remaining cleaning liquid as shown in FIG. 43I.

When the residual cleaning liquid is removed, the control unit 5 raises the glucose sensor 21A and the sodium ion sensor 21B to the origin in ST310 to ST311 of FIG. 42 in the same procedure as ST308 to ST309 of FIG. 31, to move from the residual liquid removal position to the origin position. In this way the cleaning of the glucose sensor 21A and the sodium ion sensor 21B is completed.

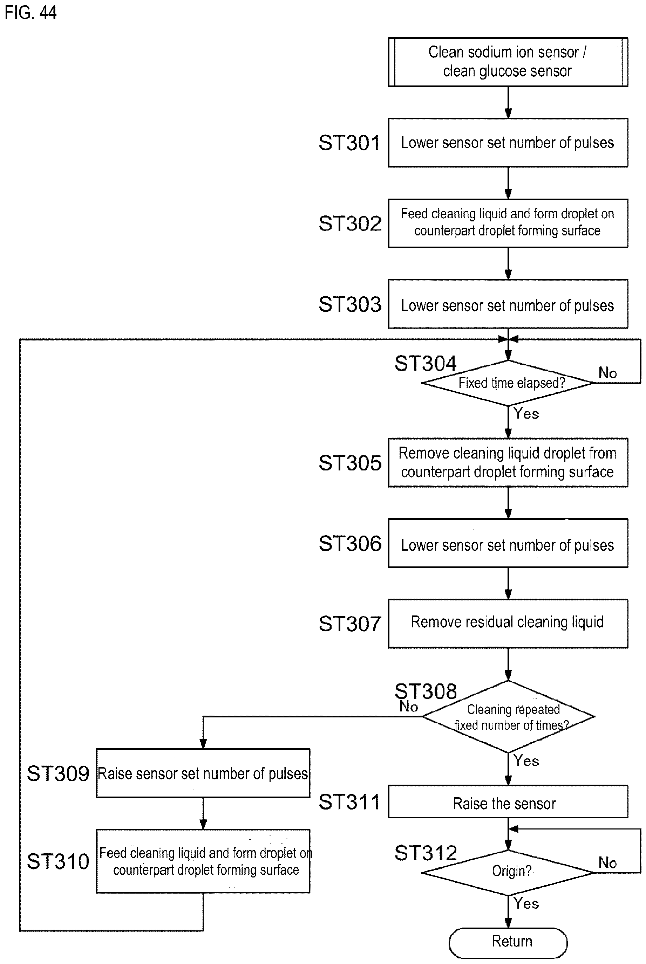

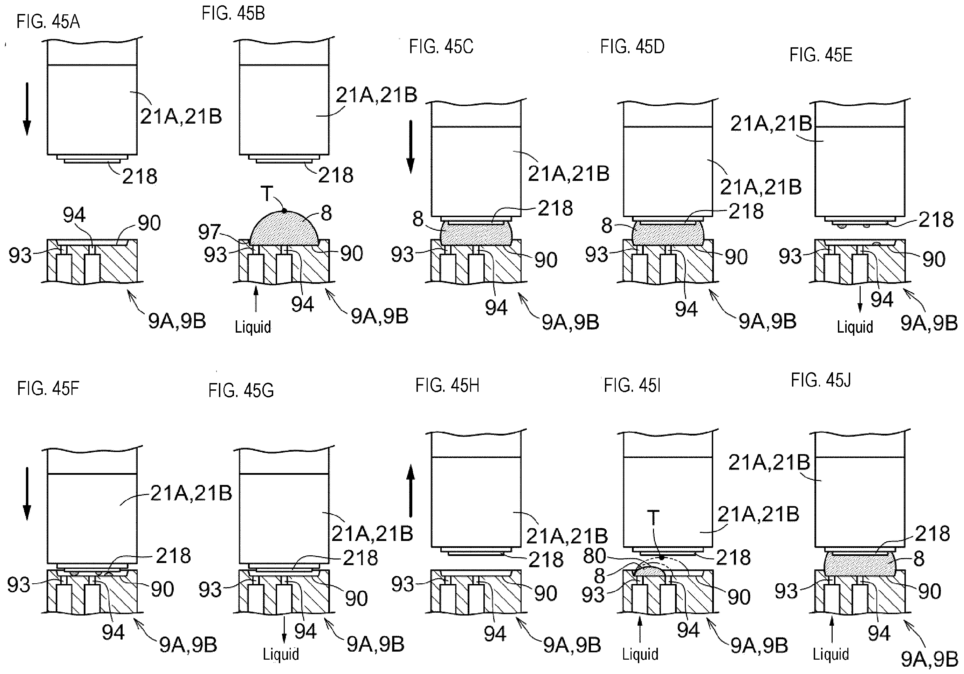

FIG. 44 and 45 show a treatment procedure when a step of removing the residual liquid is performed after the glucose sensor 21A and the sodium ion sensor 21B have been cleaned a plurality of times.

Specifically, in ST301 to ST305 of FIG. 44, the control unit 5 moves the glucose sensor 21A and the sodium ion sensor 21B from the origin position to the droplet formation standby position in the same procedure as ST301 to ST305 of FIG. 31 described above (see FIG. 45A), forms the droplet 8 of the cleaning liquid on the droplet forming surface 90 of the counterpart objects 9A and 9B (FIG. 45B), moves the glucose sensor 21A and the sodium ion sensor 21B from the droplet formation standby position to the droplet contact position, and presses the droplet 8 of the cleaning liquid against the surfaces of the glucose sensor 21A and the sodium ion sensor 21B (FIG. 45C). Then, the droplets 8 of the cleaning liquid are brought into contact with the surfaces of the glucose sensor 21A and the sodium ion sensor 21B for a fixed period of time to clean the electrode 218 (FIG. 45D). Then, the cleaning droplets 8 are removed from the droplet forming surfaces 90 of the counterpart objects 9A and 9B (FIG. 45E).

Next, in ST306 of FIG. 44, the control unit 5 controls the motor 2312 of the sensor moving unit 231 to move the glucose sensor 21A and the sodium ion sensor 21B from the droplet contact position to the residual liquid removal position as shown in FIG. 45F and bring the surfaces of the glucose sensor 21A and the sodium ion sensor 21B and the droplet forming surface 90 close to each other.

Next, in ST307 of FIG. 44, the control unit 5 drives the pump 40C to discharge the cleaning liquid adhering to the droplet forming surface 90 of the counterpart objects 9A and 9B and the surfaces of the glucose sensor 21A and the sodium ion sensor 21B from liquid discharge hole 94 to the discharged liquid to the waste liquid tank 30, thereby removing the remaining cleaning liquid as shown in FIG. 45G.

The control unit 5 monitors whether the cleaning of the glucose sensor 21A and the sodium ion sensor 21B using the cleaning liquid described above has been performed a plurality of times. When the number of times of cleaning has not reached a fixed number (NO in ST308 of FIG. 44), the control unit 5 controls the motor 2312 of the sensor moving unit 231 in ST309 of FIG. 44 to move the glucose sensor 21A and the sodium ion sensor 21B from the residual liquid removal position to the droplet contact position (FIG. 45H).

Then, in ST310 of FIG. 44, the control unit 5 sends the cleaning liquid to the droplet forming surface 90 of the counterpart objects 9A and 9B via the liquid supply holes 93 via the drive of the pumps 40A and 40B to form the droplet 8 of the cleaning liquid on the droplet forming surface 90, as shown in FIG. 45I. The droplet 8 of the cleaning liquid gradually swells and grows larger as the amount of the liquid increases. In this way the droplet 8 of the cleaning liquid is displaced in the direction in which the droplet surface 80 faces the glucose sensor 21A and the sodium ion sensor 21B (upward in this embodiment). When the amount of the cleaning droplet 8 reaches a fixed amount, the portion of the cleaning droplet 8 that is closest to the surface of the electrode-type sensor 21 on the surfaces of the glucose sensor 21A and the sodium ion sensor 21B, the top portion T in the present embodiment, hits the surface and the amount of liquid further increases to a certain amount, such that the shape of the droplet 8 changes and is flattened by pressing against surface of the glucose sensor 21A and the sodium ion sensor 21B, and the droplet 8 of cleaning liquid covers the surface of each sensor 21A and 21B, as shown in FIG. 45J. In this way the droplet 8 of the cleaning liquid comes into contact with the surfaces of the glucose sensor 21A and the sodium ion sensor 21B over a wide range.

Then, in ST304 to ST305 of FIG. 44, the control unit 5 again brings the droplet 8 into contact with the surface of the glucose sensor 21A and the sodium ion sensor 21B for a fixed time to clean the electrode 218 (FIG. 45D), and thereafter removes the cleaning droplet 8 from the droplet forming surface 90 of the counterpart objects 9A and 9B (FIG. 45E). Then, after moving the glucose sensor 21A and the sodium ion sensor 21B from the droplet contacting position to the residual liquid removing position (FIG. 45F), the droplet forming surfaces 90 of the counterpart objects 9A and 9B, the glucose sensor 21A and the sodium. The residual cleaning liquid is removed by discharging the cleaning liquid adhering to the surface of the ion sensor 21B from the liquid discharge hole 94 and sending it to the waste liquid tank 30 (FIG. 45G).

When the number of cleanings reaches a fixed number (YES in ST308 of FIG. 44), the control unit 5 performs the same procedure as ST308 to ST309 of FIG. 31 in ST311 to ST312 of FIG. 44 to raise the glucose sensor 21A and the sodium ion sensor 21B to the origin to move from the residual liquid removal position to the origin position. In this way the cleaning of the glucose sensor 21A and the sodium ion sensor 21B is completed.

Note that although the processing procedure shown in FIGS. 40 to 45 has been described in terms of a pretreatment of cleaning the sensors 21A and 21B, the procedure similarly applies to when the pretreatment is the preparation of the calibration curve using the sensors 21A and 21B, although the detailed description thereof is omitted, to wit, the droplet 8 of the standard liquid can be pressed against the surface of each sensor 21A, 21B by increasing the liquid amount of the standard droplet 8 on the droplet forming surface 90 of the counterpart objects 9A and 9B.

Also in the embodiments of FIGS. 40 to 45, even if bubbles are mixed in when the droplet 8 is formed, the bubbles float on the droplet surface 80 during the formation of the droplet 8, and the bubble escapes or breaks from the droplet surface 80 to the outside of the droplet when the droplet 8 is pressed against the surface of the sensors 21A and 21B. Hence, since the presence of bubbles on the surface of the sensor 21A and 21B can be suppressed when the liquid used for the pretreatment is brought into contact with the surface of the sensor 21A and 21B, the possibility of causing various problems in pretreatment is reduced, and pretreatment performance such as cleaning efficiency and calibration accuracy can be improved. Since the amount of bubbles mixed in the droplet 8 is small, it also is possible to avoid increasing the amount of the liquid used for the pretreatment, so that the pretreatment can be performed with a lesser amount of the pretreatment liquid.

In the embodiment of FIGS. 40 to 45, the first pressing of the droplet 8 onto the surfaces of the sensors 21A and 21B forms the droplet 8 on the droplet forming surface 90 of the counterpart objects 9A and 9B, and thereafter each sensor 21A, 21B is moved to the droplet contact position. However, the present invention is not limited to this configuration inasmuch as the droplet 8 may be pressed against the surface of each sensor 21A and 21B by increasing the amount of liquid of the droplet 8 on the droplet forming surface 90 of the counterpart objects 9A and 9B after the sensors 21A and 21B have been moved to the droplet contact position.

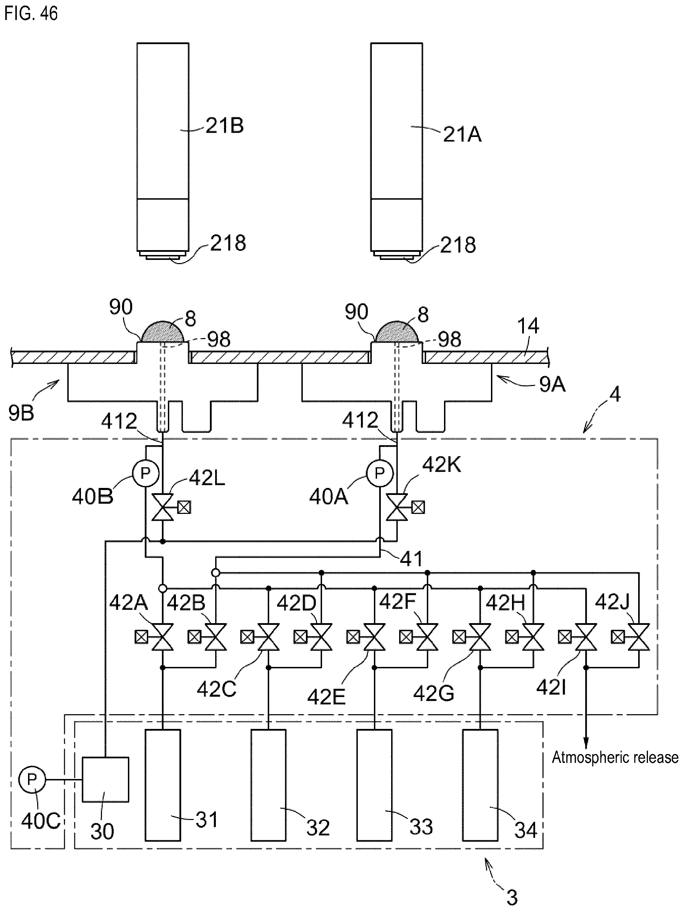

The counterpart objects 9A and 9B in the above-described embodiment also may be provided with two holes, a liquid supply hole 93 and a liquid discharge hole 94 on the droplet forming surface 90, as shown in FIG. 25. The counterpart objects 9A and 9B are not limited to this configuration, however, inasmuch as only a single liquid supply/discharge hole 98 also may be provided on the droplet forming surface 90, such that the liquid may be supplied to and discharged from the droplet forming surface 90 via the liquid supply/discharge hole 98, as shown in FIG. 46. Since the counterpart objects 9A and 9B shown in FIG. 46 have the same configuration as the counterpart object 9 shown in FIG. 8 described above in the pretreatment method, detailed description thereof will be omitted here.

FIG. 46 shows a summary of the structure of a sample storage unit and a fluid circuit unit connected to counterpart objects 9A and 9B of a modification. Although the sample storage unit 3 and the fluid circuit unit 4 shown in FIG. 46 have basically the same configuration as the sample storage unit 3 and the fluid circuit unit 4 shown in FIG. 25, the fluid circuit unit 4 shown in FIG. 46 has a pipe 41 including a liquid supply/drain passage 412 corresponding to each of the counterpart objects 9A and 9B, and the liquid supply/drain passage 412 is connected to the liquid supply/drain holes 98 of the counterpart objects 9A and 9B, whereas in the fluid circuit unit 4 shown in FIG. 25, the pipe 41 includes a liquid supply passage 410 and a liquid discharge passage 411 corresponding to the counterpart objects 9A and 9B, respectively. The liquid supply/drain passage 412 branches into two parts on the side opposite to the side connected to the liquid supply/drain hole 98 of each counterpart object 9A, 9B, one of which is connected to the waste liquid tank 30, and the other is connected to each of the tanks 31 to 34.

Although the in-vivo component measuring device 1 includes the two electrode type sensors 21 of the glucose sensor 21A and the sodium ion sensor 21B in the above-described embodiment, one or three or more electrode type sensors 21 also may be provided.

Although a calibration curve is created when the in-vivo component measuring device 1 is started in the above-described embodiment, the invention is not limited to this configuration inasmuch as a calibration curve be created every time a measurement by the sensors 21A and 21B is completed, or alternatively, may be performed every time a predetermined number of measurements are completed.

Although the in-vivo component measuring device 1 is described by way of example in which sodium ions are measured as the electrolyte in the interstitial fluid in the above embodiment, the invention is not limited to this configuration inasmuch as potassium ions, calcium ions, magnesium ions, zinc ions, chloride ions and like inorganic ions also may be measured.

Although the in-vivo component measuring device 1 installs the set of the interstitial fluid collector 110 and the sweat collector 111 in the installation unit 20 for measurement in the above embodiments, the invention is not limited to this configuration inasmuch as the measurement may be performed by installing only the interstitial fluid collector 110 in the installation unit 20.

Although the in-vivo component measuring device 1 is described by way of example of calculating the blood glucose AUC in the above embodiments, the calculation is not limited to blood AUC and may be another value insofar as the value corresponds to the in-vivo glucose concentration.

Although the in-vivo component measuring device 1 is described by way of example in which glucose in the interstitial fluid is measured in the above-described embodiment, the present invention is not limited to this configuration inasmuch as a component other than glucose contained in the interstitial fluid also may be measured. Examples of the components measured by the in-vivo component measuring device 1 include biochemical components and drugs administered to a subject. Examples of biochemical components include albumin, globulin, and enzymes, which are proteins that are one type of biochemical component. Examples of biochemical components other than proteins include creatinine, creatine, uric acid, amino acids, fructose, galactose, pentose, glycogen, lactic acid, caffeine, pyruvic acid and ketone bodies. Examples of the drugs include digitalis preparations, theophylline, antiarrhythmic agents, antiepileptic agents, amino acid glycoside antibiotics, glycopeptide antibiotics, antithrombotic agents and immunosuppressants.

EXPLANATION OF REFERENCE NUMBERS

[0216]

1 In-vivo component measuring device
4 Fluid circuit unit
5 Control unit
8 liquid droplet
9 Counterpart
9A First counterpart
9B Second counterpart
21 Electrode-type sensor
21A Glucose sensor
21B Sodium ion sensor
90 Droplet forming surface
93 Liquid supply hole
97 Wall surface
231 Sensor moving unit

**Claims**

1. A pretreatment method using an electrode-type sensor for measuring a measurement target component contained in a measurement sample collected from a subject, and a liquid used for the pretreatment of the measurement, the method comprising:

   (A) forming a droplet of the liquid;
   (B) pressing the droplet against a surface of the electrode-type sensor by relative movement of the droplet and the electrode-type sensor; and
   (C) removing the droplet.

2. The pretreatment method according to claim 1, wherein

   the forming comprises forming the droplet on a droplet forming surface provided on a counterpart object, the droplet forming surface facing the surface of the electrode-type sensor in the (A); and
   the removing comprises removing the droplet from the droplet forming surface in the (C).

3. The pretreatment method according to claim 1 or 2, wherein
   the (A), the (B), and the (C) are performed, and thereafter the (A), the (B), and the (C) are performed again.

4. The pretreatment method according to any one of claims 1 to 3, wherein
the pressing comprises covering at least a part of the surface of the electrode-type sensor by deforming a shape of the droplet to a flat shape in the (B).

5. The pretreatment method according to claim 2, wherein
the forming comprises forming the droplet by sending the liquid onto the droplet forming surface from a liquid supply hole provided on the droplet forming surface of the counterpart object in the (A).

6. The pretreatment method according to any one of claims 1 to 5, wherein

the pressing comprises (B-1) pressing the portion of the droplet closest to the surface of the electrode-type sensor against the surface of the electrode-type sensor by moving at least one of the electrode-type sensor and the counterpart object in a facing direction; and
the pressing comprises (B-2) narrowing a distance between the electrode-type sensor and the counterpart object to a certain distance by further moving at least one of the electrode-type sensor and the counterpart object in the facing direction.

7. The pretreatment method according to any one of claims 1 to 5, wherein
the pressing comprises (B-3) pressing the portion of the droplet closest to the surface of the electrode-type sensor against the surface of the electrode-type sensor by increasing an amount of the droplet; and
(B-4) further increasing the amount of the droplet.

8. The pretreatment method according to claim 7, wherein
the pressing comprises increasing the liquid amount of the droplet by sending the liquid to the droplet forming surface from a liquid supply hole provided on the droplet forming surface in the (B-3) and the (B-4).

9. The pretreatment method according to any one of 1 to 8, wherein
the electrode-type sensor is configured to acquire a signal that reflects an amount of glucose contained in the measurement sample, or a signal that reflects an amount of electrolyte contained in the measurement sample.

10. The pretreatment method according to claim 2, wherein

the droplet is formed in a size such that the surface of the electrode-type sensor fits inside an outer peripheral edge of the droplet in a plan view.

11. The pretreatment method according to claim 2, wherein

a wall surface of the counterpart object rises from the outer peripheral edge of the droplet forming surface of the counterpart object; and
the droplet is formed at a height exceeding the wall surface of the counterpart object.

12. The pretreatment method according to claim 2, wherein

the droplet forming surface of the counterpart object is inclined as the droplet forming surface of the counterpart object goes from a center of the droplet forming surface of the counterpart object toward the outer peripheral edge of the droplet forming surface of the counterpart object; and
the droplet is formed at a height exceeding the outer peripheral edge of the droplet forming surface of the counterpart object.

13. The pretreatment method according to any one of claims 1 to 12, wherein

the liquid is a cleaning liquid,
the (A), the (B) and the (C) are performed before the measurement target component contained in the measurement sample is measured by the electrode- type sensor.

14. The pretreatment method according to any one of claims 1 to 12, wherein

the liquid is a standard solution,
the (A), the (B) and the (C) are performed before the measurement target component contained in the measurement sample is measured by the electrode type sensor.

15. An in-vivo component measuring device for measuring a component contained in a measurement sample collected from a subject, comprising:

an electrode-type sensor configured to measure a measurement target component contained in the measurement sample by contacting a collecting body that collects the measurement sample;
a counterpart object facing the surface of the electrode-type sensor;
a fluid circuit unit configured to send a liquid onto a liquid drop forming surface of the counterpart object, and to discharge the liquid from the liquid drop forming surface;

a moving unit configured to move at least one of the electrode-type sensor and the counterpart object; and

a control unit configured to control the fluid circuit unit and the moving unit,

wherein the control unit is configured to

control the fluid circuit unit to form the droplet of the liquid on the droplet forming surface;

control at least one of the fluid circuit unit and the moving unit so that the droplet is pressed against the surface of the electrode-type sensor by a relative movement of the droplet and the electrode-type sensor; and

control the fluid circuit unit so as to remove the droplet from the droplet forming surface.

```
                    ┌─────────────┐
                    │    START    │
                    └─────────────┘
                           │
                           ▼
        ┌─────────────────────────────────────────┐
ST1     │            Form liquid droplet           │
        └─────────────────────────────────────────┘
                           │
   ┌───────────────────────┼────────────────────────────┐
   │                       ▼                             │
   │    ┌──────────────────────────────────────────┐    │
   │    │ Press portion of liquid droplet nearest the│   │
   │ ST2-1│ surface of electrode sensor against the  │   │
   │    │      surface of electrode sensor          │    │
   │    └──────────────────────────────────────────┘    │
ST2│                       │                             │
   │                       ▼                             │
   │    ┌──────────────────────────────────────────┐    │
   │    │ Constrict space between the surface of the │   │
   │ ST2-2│ electrode sensor and the droplet forming │   │
   │    │      surface to a fixed distance G        │    │
   │    └──────────────────────────────────────────┘    │
   └───────────────────────┼────────────────────────────┘
                           ▼
        ┌─────────────────────────────────────────┐
ST3     │ Maintain pressing of liquid droplet against│
        │   the surface of the electrode sensor    │
        └─────────────────────────────────────────┘
                           │
                           ▼
        ┌─────────────────────────────────────────┐
ST4     │            Remove liquid droplet          │
        └─────────────────────────────────────────┘
                           │
                           ▼
                    ┌─────────────┐
                    │     END     │
                    └─────────────┘
```

FIG. 1

FIG. 2A

FIG. 2B

FIG. 2C

FIG. 2D

FIG. 2E

FIG. 3A

FIG. 3B

FIG. 3C

FIG. 4

FIG. 5A

FIG. 5B

FIG. 6A

FIG. 6B

FIG. 6C

FIG. 6D

FIG. 6E

FIG. 7F

218

93

21

94   90

9

FIG. 7G

218

93

21

94   90

9

Liquid

FIG. 8A

FIG. 8B

FIG. 8C

FIG. 9A

111 ─ 112   113

111 ─ 111   110

C   C

FIG. 9B

111   110   113
112

FIG. 10A

FIG. 10B

FIG. 10C

FIG. 11A

FIG. 11B

FIG. 12A

FIG. 12B

FIG. 13

FIG. 14A

FIG. 14B

FIG. 14C

FIG. 15A

FIG. 15B

FIG. 16A

F

2013

201

G                                                G

2010     2014(61)     2011

2013

F

FIG. 16B

2013                                                2013

2012                                                2012

2010        201

FIG. 16C

2014     2012

2010     2011     201

FIG. 17A

21A,21B
210
217
213
213
215
214
216
211
214
212
218

FIG. 17B    (B)

21A,21B
210
215
211
214
216
212
218

FIG. 18A

FIG. 18B

FIG. 19

FIG. 20

Sodium ion sensor        Glucose sensor

21B                      21A

218                      218

FIG. 21A   Origin position

111        110

2000

200  } 20
201

18

2010    15

FIG. 21B   1st Measurement position

111        110

20

FIG. 21C   2nd Measurement position

111        110

20

FIG. 21D   3rd Measurement position

111        110

20

EP 3 785 632 A1

FIG. 22A

FIG. 22B

FIG. 23A

24

2310

2311

21A,21B

218

FIG. 23B

2310

2311

21A,21B

218

FIG. 24A  Origin position

FIG. 24B  Measurement position

FIG. 24C  Droplet forming standby position

FIG. 24D  Droplet contact position

FIG. 24E  Residual liquid removal position

FIG. 25

FIG. 26

FIG. 27

FIG. 28

```
      ┌─────────────────────────────────┐
      │  Preparation of calibration curve│
      │      using glucose sensor        │
      └─────────────────────────────────┘
                      │
                      ▼
ST101  ┌─────────────────────────────────┐
       │     Lower glucose sensor set     │
       │        number of pulses          │
       └─────────────────────────────────┘
                      │
                      ▼
ST102  ┌─────────────────────────────────┐
       │   Feed low-concentration standard│
       │    solution and form droplet on  │
       │ counterpart droplet forming surface│
       └─────────────────────────────────┘
                      │
                      ▼
ST103  ┌─────────────────────────────────┐            ST111  ┌─────────────────────────────────┐
       │     Lower glucose sensor set     │                   │     Raise glucose sensor set     │
       │        number of pulses          │                   │        number of pulses          │
       └─────────────────────────────────┘                   └─────────────────────────────────┘
                      │                                                       │
                      ▼                                                       ▼
ST104  ┌─────────────────────────────────┐            ST112  ┌─────────────────────────────────┐
       │     Measure current value with   │                   │  Feed high concentration standard│
       │         glucose sensor           │                   │solution and form droplet on counterpart│
       └─────────────────────────────────┘                   │      droplet forming surface     │
                      │                                        └─────────────────────────────────┘
                      ▼                                                       │
ST105  ┌─────────────────────────────────┐                                   ▼
       │Remove low-concentration standard solution│        ST113  ┌─────────────────────────────────┐
       │ droplet from counterpart droplet forming │               │     Lower glucose sensor set     │
       │             surface              │                       │        number of pulses          │
       └─────────────────────────────────┘                       └─────────────────────────────────┘
                      │                                                       │
                      ▼                                                       ▼
ST106  ┌─────────────────────────────────┐            ST114  ┌─────────────────────────────────┐
       │     Raise glucose sensor set     │                   │     Measure current value with   │
       │        number of pulses          │                   │         glucose sensor           │
       └─────────────────────────────────┘                   └─────────────────────────────────┘
                      │                                                       │
                      ▼                                                       ▼
ST107  ┌─────────────────────────────────┐            ST115  ┌─────────────────────────────────┐
       │  Feed medium concentration standard│                 │ Remove high concentration standard solution│
       │ solution and form droplet on counterpart│             │ droplet from counterpart droplet forming surface│
       │      droplet forming surface     │                   └─────────────────────────────────┘
       └─────────────────────────────────┘                                   │
                      │                                                       ▼
                      ▼                                        ST116  ┌─────────────────────────────────┐
ST108  ┌─────────────────────────────────┐                           │    Prepare glucose calibration   │
       │     Lower glucose sensor set     │                           │             curve                │
       │        number of pulses          │                           └─────────────────────────────────┘
       └─────────────────────────────────┘                                   │
                      │                                                       ▼
                      ▼                                        ST117  ┌─────────────────────────────────┐
ST109  ┌─────────────────────────────────┐                           │       Raise glucose sensor       │
       │     Measure current value with   │                           └─────────────────────────────────┘
       │         glucose sensor           │                                   │
       └─────────────────────────────────┘                                   ▼
                      │                                        ST118   ◇───────────────────────── No
                      ▼                                                ◇  Origin?  ◇────────────────┐
ST110  ┌─────────────────────────────────┐                                ◇─────────◇              │
       │Remove medium concentration standard solution│                         │ Yes               │
       │ droplet from counterpart droplet forming surface│                      ▼                   │
       └─────────────────────────────────┘                              ┌──────────────┐           │
                      │                                                   │    Return    │           │
                      └───────────────────────────────────────────────── └──────────────┘
```

FIG. 29

Preparation of calibration curve
using sodium ion sensor

ST201 | Lower sodium ion sensor set
number of pulses

ST202 | Feed low-concentration standard
solution and form droplet on
counterpart droplet forming surface

ST203 | Lower sodium ion sensor set
number of pulses

ST204 | Measure voltage value with
sodium ion sensor

ST205 | Remove low-concentration standard solution
droplet from counterpart droplet forming
surface

ST206 | Raise sodium ion sensor set
number of pulses

ST207 | Feed medium concentration standard
solution and form droplet on counterpart
droplet forming surface

ST208 | Lower sodium ion sensor set
number of pulses

ST209 | Measure voltage value with
sodium ion sensor

ST210 | Remove medium concentration standard solution
droplet from counterpart droplet forming surface

ST211 | Raise sodium ion sensor set
number of pulses

ST212 | Feed high concentration standard
solution and form droplet on counterpart
droplet forming surface

ST213 | Lower sodium ion sensor set
number of pulses

ST214 | Measure voltage value with
glucose sensor

ST215 | Remove high concentration standard solution
droplet from counterpart droplet forming surface

ST216 | Prepare sodium ion calibration
curve

ST217 | Raise sodium ion sensor

ST218 | Origin?   No

Yes

Return

FIG. 30A

FIG. 30B

FIG. 30C

FIG. 30D

FIG. 30E

FIG. 30F

FIG. 31

ST301 — Lower sensor set number of pulses

ST302 — Feed cleaning liquid and for droplet on counterpart droplet forming

ST303 — Lower sensor set number of pulses

ST304 — Fixed time elapsed? — No / Yes

ST305 — Remove cleaning liquid droplet from counterpart droplet forming surface

ST306 — Cleaning repeated fixed number of times? — No / Yes

ST307 — Raise sensor set number of pulses

ST308 — Raise sensor

ST309 — Origin? — No / Yes

Return

FIG. 32

```
                    ┌──────────────────────────────┐
                    │         Measurement          │
                    └──────────────┬───────────────┘
                                   ▼
            ┌──────────────────────────────────────┐
   ST401    │ Horizontally move installation unit  │
            │        set number of pulses          │
            └──────────────────┬───────────────────┘
                               ▼
            ┌──────────────────────────────────────┐
   ST402    │  Perform measurement with sodium ion │
            │               sensor                 │
            └──────────────────┬───────────────────┘
                               ▼
            ┌──────────────────────────────────────┐
   ST403    │ Horizontally move installation unit  │
            │        set number of pulses          │
            └──────────────────┬───────────────────┘
                               ▼
            ┌──────────────────────────────────────┐
   ST404    │    Perform measurement with glucose  │
            │               sensor                 │
            └──────────────────┬───────────────────┘
                               ▼
            ┌──────────────────────────────────────┐
   ST405    │        Clean sodium ion sensor       │
            └──────────────────┬───────────────────┘
                               ▼
            ┌──────────────────────────────────────┐
   ST406    │ Horizontally move installation unit  │
            │        set number of pulses          │
            └──────────────────┬───────────────────┘
                               ▼
            ┌──────────────────────────────────────┐
   ST407    │  Perform measurement with sodium ion │
            │               sensor                 │
            └──────────────────┬───────────────────┘
                               ▼
            ┌──────────────────────────────────────┐
   ST408    │         Clean glucose sensor         │
            └──────────────────┬───────────────────┘
                               ▼
            ┌──────────────────────────────────────┐
   ST409    │  Horizontally move installation unit │
            └──────────────────┬───────────────────┘
                               ▼
   ST410          ◇ Origin? ◇ ──No──┐
                       │ Yes        │
                       ▼            │ (back up to ST409)
            ┌──────────────────────────────────────┐
   ST411    │          Sodium ion sensor           │
            └──────────────────┬───────────────────┘
                               ▼
                       (   Return   )
```

FIG. 33

```
        ┌─────────────────────────────┐
        │ Perform measurement with sodium │
        │         ion sensor          │
        └─────────────────────────────┘
                      │
                      ▼
        ┌─────────────────────────────┐
ST501   │ Lower sodium ion sensor set number │
        │          of pulses          │
        └─────────────────────────────┘
                      │
                      ▼
        ┌─────────────────────────────┐
ST502   │ Obtain collector voltage value using │
        │        sodium ion sensor        │
        └─────────────────────────────┘
                      │
                      ▼
        ┌─────────────────────────────┐
ST503   │      Raise sodium ion sensor     │
        └─────────────────────────────┘
                      │
                      ▼
ST504         ◇ Origin? ◇ ──No──┐
                 │              │
                Yes             │
                 │              │
                 ▼              │
           ( Return )           │
```

FIG. 34

```
          ┌─────────────────────────────────┐
          │ Perform measurement with glucose │
          │             sensor               │
          └─────────────────────────────────┘
                          │
                          ▼
ST601     ┌─────────────────────────────────┐
          │ Lower glucose sensor set number  │
          │           of pulses              │
          └─────────────────────────────────┘
                          │
                          ▼
ST602     ┌─────────────────────────────────┐
          │ Obtain collector current value   │
          │ of interstitial fluid collector  │
          │     using glucose sensor         │
          └─────────────────────────────────┘
                          │
                          ▼
ST603     ┌─────────────────────────────────┐
          │   Raise glucose sensor sensor    │
          └─────────────────────────────────┘
                          │
                          ▼
ST604           ◇ Origin? ◇ ──── No ────┐
                     │                  │
                    Yes                 │
                     │                  │
                     ▼                  │
               (  Return  )             │
```

FIG. 35

```
                    ┌──────────────────────┐
                    │       Analysis       │
                    └──────────────────────┘
                               │
                               ▼
        ST701      ┌──────────────────────┐
                   │ Measure concentration │
                   └──────────────────────┘
                               │
                               ▼
        ST702         ◇ Perspiration rate R        No
                      = threshold value or  ──────────────────┐
                         higher?                              │
                           │ Yes                              │
                           ▼                                  ▼
        ST704  ┌──────────────────────┐          ┌────────────────────────────────┐
               │ Calculate glucose AUC │   ST703  │ Prepare analysis result with message │
               └──────────────────────┘          │ indicating glucose AUC not prepared │
                           │                      └────────────────────────────────┘
                           ▼                                  │
        ST705  ┌──────────────────────┐                      │
               │  Calculate analysis result │                 │
               │  including glucose AUC │                      │
               └──────────────────────┘                      │
                           │◄─────────────────────────────────┘
                           ▼
                    ╭──────────────╮
                    │    Return    │
                    ╰──────────────╯
```

FIG. 36

```
        ┌─────────────────────────────────┐
        │ Clean sodium ion sensor /       │
        │ clean glucose sensor            │
        └─────────────────────────────────┘
                       │
                       ▼
ST301   ┌─────────────────────────────────┐
        │ Lower sensor set number of pulses│
        └─────────────────────────────────┘
                       │
                       ▼
ST302   ┌─────────────────────────────────┐
        │ Feed cleaning liquid and form droplet on│
        │ counterpart droplet forming surface │
        └─────────────────────────────────┘
                       │
                       ▼
ST303   ┌─────────────────────────────────┐
        │ Lower sensor set number of pulses│
        └─────────────────────────────────┘
                       │
                       ▼
ST304      ◇ Fixed time elapsed? ◇ ──No──┐
                       │ Yes            ↑
                       ▼                │
ST305   ┌─────────────────────────────────┐
        │ Remove cleaning liquid droplet from│
        │ counterpart droplet forming surface │
        └─────────────────────────────────┘
                       │
                       ▼
ST306   ◇ Cleaning repeated fixed number of times? ◇ ──No──┐
                       │ Yes                               ▼
                       ▼                          ST307 ┌──────────────────────┐
ST308   ┌─────────────────────────────┐                │ Raise sensor set number of│
        │ Lower sensor set number of pulses│            │ pulses               │
        └─────────────────────────────┘                └──────────────────────┘
                       │
                       ▼
ST309   ┌─────────────────────────────┐    ST310 ┌──────────────────────┐
        │ Remove residual cleaning liquid│         │ Raise sensor         │
        └─────────────────────────────┘          └──────────────────────┘
                                                          │
                                                          ▼
                                               ST311  ◇ Origin? ◇ ──No──┐
                                                          │ Yes        ↑
                                                          ▼            │
                                                     ( Return )
```

EP 3 785 632 A1

FIG. 37A

21A,21B

218

93  94  90

9A,9B

FIG. 37B

21A,21B

218

T

97  8

93  90

94  9A,9B

Liquid

FIG. 37C

21A,21B

218

8

93

94  90

9A,9B

FIG. 37D

21A,21B

218

8

93  90

94

9A,9B

FIG. 37E

21A,21B

218

90

93

94  9A,9B

Liquid

FIG. 37F

21A,21B

218

90

93

94  9A,9B

FIG. 37G

21A,21B

218

90

93

94  9A,9B

FIG. 37H

21A,21B

218

90

93

94  9A,9B

Liquid

FIG. 38

Clean sodium ion sensor /
clean glucose sensor

ST301    Lower sensor set number of pulses

ST302    Feed cleaning liquid and form droplet on counterpart droplet forming surface

ST303    Lower sensor set number of pulses

ST304    Fixed time elapsed?    No

Yes

ST305    Remove cleaning liquid droplet from counterpart droplet forming surface

ST306    Lower sensor set number of pulses

ST307    Remove residual cleaning liquid

ST308    Cleaning repeated fixed number of times?    No

Yes

ST309    Raise sensor set number of pulses

ST310    Raise sensor

ST311    Origin?    No

Yes

Return

FIG. 39A
FIG. 39B
FIG. 39C
FIG. 39D
FIG. 39E
FIG. 39F
FIG. 39G
FIG. 39H

EP 3 785 632 A1

FIG. 40

Clean sodium ion sensor /
clean glucose sensor

ST301 — Lower sensor set number of pulses

ST302 — Feed cleaning liquid and form droplet on counterpart droplet forming surface

ST303 — Lower sensor set number of pulses

ST304 — Fixed time elapsed? — No

Yes

ST305 — Remove cleaning liquid droplet from counterpart droplet forming surface

ST306 — No — Cleaning repeated fixed number of times? — Yes

ST307 — Feed cleaning liquid and form droplet on counterpart droplet forming surface

ST308 — Raise sensor

ST309 — Origin? — No

Yes

Return

FIG. 41A

FIG. 41B

FIG. 41C

FIG. 41D

FIG. 41E

FIG. 41F

FIG. 41G

21A, 21B
218
90
94
93
9A, 9B
8
T
97
Liquid
80

FIG. 42

```
          ┌──────────────────────────────┐
          │   Clean sodium ion sensor /   │
          │     clean glucose sensor      │
          └──────────────────────────────┘
                          │
          ┌──────────────────────────────┐
ST301     │  Lower sensor set number of pulses │
          └──────────────────────────────┘
                          │
          ┌──────────────────────────────┐
ST302     │ Feed cleaning liquid and form droplet on │
          │   counterpart droplet forming surface    │
          └──────────────────────────────┘
                          │
          ┌──────────────────────────────┐
ST303     │  Lower sensor set number of pulses │
          └──────────────────────────────┘
                          │
ST304          ◇ Fixed time elapsed? ◇ ──No──┐
                          │ Yes               │
          ┌──────────────────────────────┐   │
ST305     │ Remove cleaning liquid droplet from │ │
          │  counterpart droplet forming surface │ │
          └──────────────────────────────┘   │
                          │                   │
ST306   No ◇ Cleaning repeated fixed number of times? ◇
                          │ Yes
ST307  ┌──────────────────────────────┐
       │ Feed cleaning liquid and form droplet │
       │ on counterpart droplet forming surface │
       └──────────────────────────────┘
                          │
          ┌──────────────────────────────┐
ST308     │  Lower sensor set number of pulses │
          └──────────────────────────────┘
                          │
          ┌──────────────────────────────┐
ST309     │   Remove residual cleaning liquid  │
          └──────────────────────────────┘
                          │
          ┌──────────────────────────────┐
ST310     │          Raise sensor          │
          └──────────────────────────────┘
                          │
ST311          ◇ Origin? ◇ ──No──┐
                          │ Yes
                      ( Return )
```

FIG. 43A FIG. 43B FIG. 43C FIG. 43D FIG. 43E

FIG. 43F FIG. 43G FIG. 43H FIG. 43I

FIG. 44

Clean sodium ion sensor /
clean glucose sensor

ST301 | Lower sensor set number of pulses

ST302 | Feed cleaning liquid and form droplet on counterpart droplet forming surface

ST303 | Lower sensor set number of pulses

ST304 | Fixed time elapsed? — No

Yes

ST305 | Remove cleaning liquid droplet from counterpart droplet forming surface

ST306 | Lower sensor set number of pulses

ST307 | Remove residual cleaning liquid

ST308 | Cleaning repeated fixed number of times? — No

Yes

ST309 | Raise sensor set number of pulses

ST310 | Feed cleaning liquid and form droplet on counterpart droplet forming surface

ST311 | Raise the sensor

ST312 | Origin? — No

Yes

Return

FIG. 45A
FIG. 45B
FIG. 45C
FIG. 45D
FIG. 45E

FIG. 45F
FIG. 45G
FIG. 45H
FIG. 45I
FIG. 45J

EP 3 785 632 A1

FIG. 46

Atmospheric release

FIG. 47A

100

101

FIG. 47B

102

103

104

FIG. 48

Europäisches
Patentamt

European
Patent Office

Office européen
des brevets

# EUROPEAN SEARCH REPORT

Application Number

EP 20 19 2627

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/231234 A1 (KOJIMA JUNKO [JP] ET AL) 1 August 2019 (2019-08-01) <br> * figures 6A-C, 17, 20A-C, 25A, 29 * <br> * paragraphs [0132] - [0134], [0142] * <br> ----- | 1-15 | INV. <br> A61B5/145 <br> A61B5/1495 <br> B08B3/04 |
| X | US 2009/114538 A1 (TAKAYAMA HIROKO [JP] ET AL) 7 May 2009 (2009-05-07) <br> * figures 1, 2 * <br> * paragraphs [0047], [0051], [0052] * <br> ----- | 1-15 | |

TECHNICAL FIELDS
SEARCHED (IPC)

A61B
B08B

The present search report has been drawn up for all claims

| Place of search | Date of completion of the search | Examiner |
|---|---|---|
| The Hague | 26 November 2020 | Almeida, Mariana |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 20 19 2627

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2020

| Patent document cited in search report | Publication date | Patent family member(s) | Publication date |
|---|---|---|---|
| US 2019231234 A1 | 01-08-2019 | CN 110095609 A<br>EP 3533520 A2<br>JP 2019132733 A<br>US 2019231234 A1 | 06-08-2019<br>04-09-2019<br>08-08-2019<br>01-08-2019 |
| US 2009114538 A1 | 07-05-2009 | CN 101430338 A<br>JP 2009133851 A<br>US 2009114538 A1 | 13-05-2009<br>18-06-2009<br>07-05-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019158640 A
- JP 1207756 A
- JP 2006126046 A